# FASCICULE DE BREVET EUROPEEN

(11) **EP 3 233 838 B1**
(45) Date de publication et mention de la délivrance du brevet: **30.01.2019**
(21) Numéro de dépôt: 15820128.5
(22) Date de dépôt: 18.12.2015
(51) Int. Cl.: C07D 405/06, C07D 413/06, C07D 401/06, C07D 405/04, C07D 401/04, C07D 409/06, C07D 405/14, C07D 403/06, A61K 31/4184, A61P 17/00

(54) **DÉRIVÉS BENZIMIDAZOLES SULFONAMIDES EN TANT QU'AGONISTES INVERSES DU RÉCEPTEUR GAMMA ORPHELIN ASSOCIÉ AUX RÉTINOÏDES ROR GAMMA (T)**
BENZIMIDAZOLSULFONAMIDDERIVATE ALS INVERSE AGONISTEN VON RETINOID-RELATED ORPHAN RECEPTOR GAMMA (ROR GAMMA (T))
BENZIMIDAZOLE SULFONAMIDE DERIVATIVES AS INVERSE AGONISTS OF RETINOID-RELATED ORPHAN RECEPTOR GAMMA (ROR GAMMA (T))

(30) Priorité: 19.12.2014 FR 1463035; 03.07.2015 FR 1556341
(43) Date de publication de la demande: 25.10.2017
(73) Titulaire: Galderma Research & Development, 06410 Biot (FR)
(72) Inventeur: MUSICKI, Branislav, 06000 Nice (FR); THOREAU, Etienne, 06460 Saint-Vallier-de-Thiey (FR); BOUIX-PETER, Claire, 06220 Vallauris (FR); OUVRY, Gilles, 06410 Biot (FR)
(74) Mandataire: Casalonga
(86) Numéro de dépôt international: PCT/EP2015/080691
(87) Numéro de publication internationale: WO 2016/097393

(56) Documents cités:
- WO-A1-2013/160418
- WO-A1-2014/090712
- YAN ZHANG ET AL: "Discovery of 2-oxo-1,2-dihydrobenzo[cd]indole-6-sulfona mide derivatives as new ROR[gamma] inhibitors using virtual screening, synthesis and biological evaluation", EUROPEAN JOURNAL OF MEDICINAL CHEMISTRY, vol. 78, 22 mars 2014 (2014-03-22), pages 431-441, XP028847891, ISSN: 0223-5234, DOI: 10.1016/J.EJMECH.2014.03.065

## Description

La présente invention concerne des dérivés sulfonamides bicycles particuliers, leurs sels d'addition pharmaceutiquement acceptables leurs hydrates et/ou leurs solvates ainsi que leur utilisation en tant qu'agoniste inverse du récepteur gamma orphelin associé aux rétinoïdes RORyt.

L'invention est également relative à une composition pharmaceutique comprenant de tels composés ainsi que son utilisation pour le traitement par voie topique et/ou orale des maladies inflammatoires médiées par les récepteurs RORγt, notamment l'acné, la dermatite atopique et/ou le psoriasis.

Les récepteurs nucléaires forment une grande famille (appelée superfamille) de facteurs de transcription qui correspondent à des protéines capables d'être activées par un ligand, de se fixer sur des séquences d'ADN spécifiques et de réguler la transcription de gènes cibles. Ainsi ces récepteurs se retrouvent impliqués dans la régulation d'une grande variété de fonctions biologiques, dont la croissance, le développement, la reproduction, la différenciation et le métabolisme dans une multitude d'organismes vivants.

Les premiers membres de cette superfamille à avoir été identifiés et décrits dans la littérature scientifique sont les récepteurs nucléaires des hormones stéroïdes tels que les récepteurs aux glucocorticoïdes et les récepteurs oestrogènes. Cette superfamille comprend également parmi ses membres de nombreux récepteurs pour lesquels aucun ligand n'a été identifié. Ces récepteurs nucléaires sont appelés « récepteurs orphelins ».

Les récepteurs orphelins associés aux rétinoïdes (*Retinoid-related orphan receptors* en langue anglaise) constituent donc une sous-famille des récepteurs nucléaires. Cette sous-famille est composée de trois membres ayant chacun leur propre profil d'expression : ROR alpha (dénommé RORα), ROR beta (dénommé RORβ) et ROR gamma (dénommé RORγ). Deux isoformes des récepteurs orphelins RORγ ont déjà été identifiés, à savoir RORγ1, qui s'exprime dans une variété de tissus tels que le thymus, les reins, les muscles et le foie, et RORγ2 (aussi nommé RORγt) qui s'exprime exclusivement dans les cellules du système immunitaire.

En particulier, le récepteur RORγt joue un rôle important de régulateur dans la différenciation cellulaire des lymphocytes Th17 qui correspondent à des lymphocytes T auxiliaires ayant pour fonction d'assurer la défense de l'organisme par rapport à un grand nombre d'agents pathogènes extracellulaires tels que les bactéries et les infections fongiques.

Toutefois, il a été démontré que les lymphocytes Th17 sont également impliqués dans une large variété de désordres inflammatoires, tels que l'acné, et de maladies auto-immunes telles que le psoriasis, l'arthrite rhumatoïde ou encore la sclérose en plaques (Peck A, Mellins ED. Precarious balance; Th17 cells in host defense. Infect Immun. 2010 Jan ; 78(1) :32-8 ; Suarez-Farinas: J. Allergy Clin. Immunol. 2014; J. Invest. Dermatol. 2008, 128(11), 2625).

En effet, les lymphocytes Th17 produisent de nombreuses cytokines ayant des profils distincts telles que l'interleukine-17A (IL-17A), l'interleukine-17F (IL-17F), l'interleukine-26 (IL-26), l'interleukine-21 (IL-21), l'interleukine-22 (IL-22) et le TNFα dont leur développement, leur survie et leur prolifération dépendent de l'interleukine-23 (IL-23). Ces cytokines sont capables d'activer différents types de cellules effectrices, telles que les kératinocytes, conduisant ainsi à leur hyperprolifération et à la production supplémentaire de cytokines pro-inflammatoires, de chimiokines et de peptides antimicrobiens, qui à leur tour recrutent et activent d'autres cellules du système immunitaire dans la peau enflammée, ce qui peut conduire à une amplification de la réponse inflammatoire.

Ainsi l'activation des lymphocytes Th17 est responsable du recrutement de cytokines, notamment d'interleukine-17 (IL17), et d'autres types de cellules pro-inflammatoires qui vont conduire à la médiation de désordres inflammatoires tels que l'acné et/ou de maladies auto-immunes telles que le psoriasis.

Des expériences menées sur des souris montrent qu'une diminution au niveau de l'expression du récepteur RORγt conduit à une baisse de l'activité des lymphocytes Th17 ce qui permet, par conséquent, de fortement réduire l'expression d'interleukine-17 (IL-17) (Ivanov II, McKenzie BS, Zhou L, Tadokoro CE, Lepelley A, Lafaille JJ, Cua DJ, Littman DR : Cell 2006, 126, 1121-1133) et de traiter efficacement les désordres inflammatoires et les maladies auto-immunes médiées par ces cytokines, notamment celles pour lesquelles des taux importants en interleukine-17 (IL-17) sont détectés.

A cet effet, la demande de brevet WO 2013/160418 décrit des composés sulfonamides utilisés comme agonistes inverses du récepteur RORγt afin de pouvoir traiter les désordres inflammatoires et les maladies auto-immunes. De la même façon, d'autres composés ont également été développés en tant qu'agonistes inverses du récepteur RORγt comme ceux décrits dans les demandes de brevet WO 2014/090712, WO 2014/008214, WO2013/169588, WO2013/160419, WO2013/1002027, WO2013/092939, WO2013/092941, WO2013/085890 et WO2012/ 100732.

Il existe donc un réel besoin de développer de nouveaux composés en tant qu'agonistes inverses du récepteur RORγt afin de pouvoir traiter efficacement les maladies médiées par un tel récepteur, notamment les désordres inflammatoires, tels que l'acné et/ou les maladies auto-immunes tels que le psoriasis ou la dermatite atopique.

Ce but est atteint grâce à la mise en oeuvre de dérivés sulfonamides bicycles particuliers tels que décrits ci-après qui permettent de moduler l'activité du récepteur RORyt et de traiter par conséquent efficacement les désordres inflammatoires et les maladies auto-immunes de certaines pathologies.

La présente invention a donc notamment pour objet un ou des composés de formule (I), leurs sels d'addition pharmaceutiquement acceptables, leurs hydrates et/ou leurs solvates : 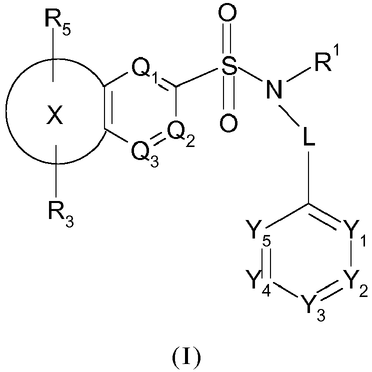 Formule (I) dans laquelle :
- L représente une liaison simple ou un groupe méthylène CH₂,
- X représente le radical cyclique suivant : 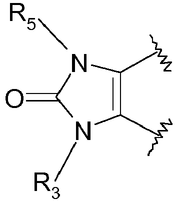
- un ou deux des éléments Y¹, Y², Y³, Y⁴ et Y⁵ représente(nt) un atome d'azote et les autres éléments correspondent à un groupement -CR², ou chacun des éléments Y¹, Y², Y³, Y⁴ et Y⁵ correspond à un groupement -CR²,
- un ou deux des éléments Q¹, Q² et Q³ représente(nt) un atome d'azote et le ou les autres éléments corresponde(nt) à un groupement - CR^{2a}, ou chacun des éléments Q¹, Q² et Q³ correspond à un groupement -CR^{2a},
- R¹ représente un radical alkyle, linéaire ou ramifié, en C₃-C₅, un radical cycloalkyle en C₃-C₅, un radical alkényle, linéaire ou ramifié, en C₂-C₅, un radical -CH₂-cycloalkyle en C₃-C₅, un radical hétérocycloalkyle en C₄-C₅, un radical -CH₂-hétérocycloalkyle en C₄-C₆,
- R² représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement cyano -CN, un radical -C(=O)R'² avec R'² désignant un radical alcoxy en C₁-C₃, un radical -CF₃ ; lesdits radicaux alkyle, alkényle et alcoxy pouvant être substitués par un ou plusieurs atomes d'halogène ;
- R^{2a} représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement -CN, un groupement hydroxy -OH, un groupement - CH(R^{3a})OH, un groupement carboxylique -COOH, un groupement carbamoyle -CONR^{2c}R^{2d}, un groupement amido -NR^{2c}COR^{2d}, un groupement -SO₂R^{2c}, un groupement -SOR^{2c}, un groupement - S(=O)(=NH-R^{2c}), lesdits radicaux alkyle, alkényle et alcoxy pouvant être substitués par un ou plusieurs atomes d'halogène,
- R^{2c} et R^{2d}, identiques ou différents, représentent un atome d'hydrogène ou un radical alkyle, linéaire ou ramifié, en C₁-C₅ ;
- R^{3a} représente un atome d'hydrogène ou un radical alkyle, linéaire ou ramifié, en C₁-C₅ ;
- R³ représente un atome d'hydrogène ou un groupement (CHR⁶)ₙ-(Z)ₒ-(CHR'⁶)ₚ-R⁷,
- n, o et p, identiques ou différents, représentent zéro ou un entier naturel allant de 1 à 3,
- Z représente un groupement divalent choisi parmi un groupement méthylène -CH₂-, un groupement amino -NH- et un atome d'oxygène - O-,
- R⁶ et R'⁶, identiques ou différents, représentent un atome d'hydrogène, un groupement méthyle -CH₃, un groupement -OH, un groupement hydroxyméthyle, une fonction carboxylique -COOH,
- R⁷ représente :
   - un atome d'hydrogène ou un atome d'halogène,
   - un groupement COOR'⁷ avec R'⁷ désignant alkyl(C₁)hétérocycle(C₆),
   - un radical hétérocycloalkyle non cationique éventuellement substitué par un ou plusieurs atomes d'halogène, un ou plusieurs groupements alkyle en C₁-C₃, linéaire ou ramifié, un ou plusieurs groupements -OH, une ou plusieurs fonctions carbonyles, un ou plusieurs groupements hydroxyalkyle en C₁-C₄, linéaire ou ramifié, un ou plusieurs groupements amino, un ou plusieurs groupements -C(=O)R^{7a}, un ou plusieurs groupements S(=O)₂R^{7a} ; R^{7a} représentant un radical alkyle, linéaire ou ramifié, en C₁-C₃, un radical alcoxy en C₁-C₃, linéaire ou ramifié, ou un radical amino N(R^{8a})(R^{8b}),
   - un radical cycloalkyle non cationique en C₃-C₆ éventuellement substitué par un ou plusieurs radicaux méthyle, un ou plusieurs atomes d'halogène, un groupement cyano -CN ou un ou plusieurs groupements -COR¹³; R¹³ désignant un radical alcoxy en C₁-C₃, linéaire ou ramifié, ou un groupement hydroxy,
   - un radical aromatique ou hétéroaromatique, non cationique, éventuellement substitué par un ou plusieurs atomes d'halogène, un ou plusieurs groupements alkyle en C₁-C₃, linéaire ou ramifié, éventuellement substitué par un ou plusieurs atomes d'halogène, un ou plusieurs groupements alcoxy en C₁-C₃, un ou plusieurs groupements amino -NR¹¹R¹², un ou plusieurs groupements -COR¹¹, un ou plusieurs groupements -COOR¹¹, un ou plusieurs groupements amido - CONR¹¹R¹², un ou plusieurs groupements -SOR¹¹, un ou plusieurs groupements -SO₂R¹¹, un ou plusieurs groupements - NHCOR¹¹, un ou plusieurs groupements -NHCOOR¹¹, un ou plusieurs groupements -SO₂NR¹¹R¹² ou un ou plusieurs groupements -CN ; R¹¹ et R¹², identiques ou différents, représentant un atome d'hydrogène, un radical hydroxy -OH, un radical alkyle, linéaire ou ramifié, en C₁-C₃, éventuellement substitué par un ou plusieurs atomes d'halogène,
- R⁵ représente un atome d'hydrogène ou un atome d'halogène, un radical alkyle en C₁-C₃, linéaire ou ramifié éventuellement substitué par un ou plusieurs atomes d'halogène; un radical amino -NH₂, un radical CH₂R'^{7a} avec R'^{7a} désignant un radical méthoxy, un groupement hydroxy -OH, un groupement -CH₂COOH, un groupement -CH(R^{5b})OH, un groupement carboxylique -COOH, un groupement -CN, une fonction thioxo,
- R^{5b} représente un atome d'hydrogène ; un radical alkyle, linéaire ou ramifié, en C₁-C₃ éventuellement substitué par une ou plusieurs fonctions carboxyliques ; un radical cyclopropyle,
- R^{8a} et R^{8b}, identiques ou différents, désignent un atome d'hydrogène, un radical alkyle, linéaire ou ramifié, en C₁-C₃ ou un radical cyclopropyle.

Le ou les composés selon l'invention correspondent ainsi à des dérivés sulfonamides bicycles, soit donc à un ou des composés sulfonamides comportant dans leur structure au moins deux cycles qui sont condensés l'un sur l'autre.

Conformément à la définition de la formule (I), la liaison intracyclique entre le radical cyclique X, tel que représenté ci-avant, et le noyau aromatique comportant les éléments Q₁ à Q³ est une liaison double. Ainsi la double liaison est commune entre le radical cyclique X et le noyau aromatique comportant les éléments Q₁ à Q₃. La double liaison intracyclique peut se délocaliser à l'intérieur du noyau aromatique comportant les éléments Q₁ à Q₃.

Les composés selon l'invention permettent de moduler, c'est-à-dire d'inhiber, l'activité du récepteur RORyt.

La présente invention a également pour objet le ou les composés tels que définis précédemment en tant que médicament et cosmétique.

Un autre objet de l'invention porte sur le ou les composés tels que définis précédemment pour leur utilisation dans le traitement des maladies médiées par le récepteur RORγt, notamment les désordres inflammatoires et/ou les maladies auto-immunes médiées par le récepteur RORγt.

Par ailleurs, l'invention concerne également une composition pharmaceutique comprenant, dans un milieu pharmaceutiquement acceptable, un ou plusieurs composés de formule (Ia) telle que définie précédemment, leurs sels d'addition pharmaceutiquement acceptables, leurs hydrates et/ou leurs solvates.

La présente invention a aussi trait à la composition pharmaceutique telle que décrite précédemment pour son utilisation dans le traitement des maladies médiées par le récepteur RORγt, notamment les désordres inflammatoires et/ou les maladies auto-immunes.

Enfin, l'invention est relative à une méthode de traitement des maladies médiées par le récepteur RORγt comprenant l'administration, notamment par voie topique ou orale, d'une quantité thérapeutiquement efficace d'un ou plusieurs composés tels que définis ci-avant à un patient.

D'autres objets et caractéristiques, aspects et avantages de l'invention apparaîtront encore plus clairement à la lecture de la description et des exemples qui suivent.

Selon un mode de réalisation, dans la formule (I), L représente une liaison simple.

Selon un autre mode de réalisation, dans la formule (I), L représente un groupe méthylène -CH₂.

Préférentiellement, dans la formule (I), L représente une liaison simple.

Préférentiellement, R³ est différent d'un atome d'halogène et R⁵ représente un atome d'hydrogène ou un radical alkyle en C₁-C₃, linéaire ou ramifié, éventuellement substitué par un ou plusieurs atomes d'halogène.

Selon un mode de réalisation, R³ représente un atome d'hydrogène, un groupement (CHR⁶)ₙ-(Z)ₒ-(CHR'⁶)ₚ-R⁷ et R⁵ représente un atome d'hydrogène ou un radical alkyle en C₁-C₃, linéaire ou ramifié, éventuellement substitué par un ou plusieurs atomes d'halogène.

Selon un mode de réalisation, dans la formule (I), R³ représente un atome d'hydrogène.

Selon un mode de réalisation, dans la formule (I), R³ représente un groupement (CHR⁶)ₙ-(Z)ₒ-(CHR'⁶)ₚ-R⁷.

Selon un mode de réalisation, dans la formule (I), les indices n, o, et p, identiques ou différents, désignent zéro.

Selon un mode de réalisation, dans la formule (I), les indices n, o, et p, identiques ou différents, désignent un entier naturel variant de 1 à 3.

Selon un mode de réalisation, dans la formule (I), les indices n et p désignent zéro et l'indice o vaut 1.

Selon un mode de réalisation, dans la formule (I), Z représente un groupe méthylène -CH₂.

Selon un mode de réalisation, dans la formule (I), Z représente un groupe divalent -O-.

Selon un mode de réalisation, dans la formule (I), Z représente un groupe divalent -NH-.

Selon un mode de réalisation, dans la formule (I), R³ représente un groupement Z-R⁷, avec Z ayant la signification précédemment décrite.

Selon un mode de réalisation particulier, dans la formule (I), R³ représente un groupement -CH₂-R⁷.

Selon un mode de réalisation particulier, dans la formule (I), R³ représente un groupement -O-R⁷.

Selon un mode de réalisation particulier, dans la formule (I), R³ représente un groupement -NH-R⁷.

Selon un mode de réalisation, dans la formule (I), R⁷ représente un radical hétérocyclique choisi parmi les hétérocycles suivants : 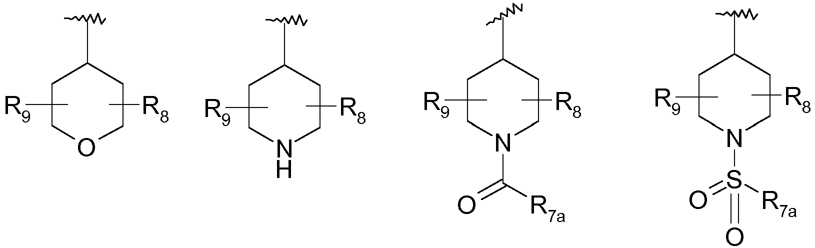 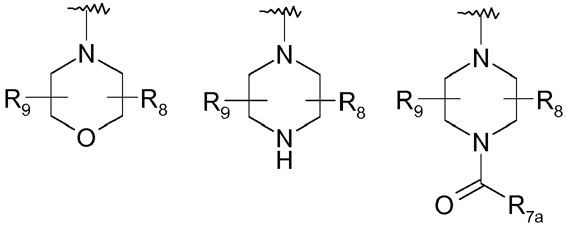 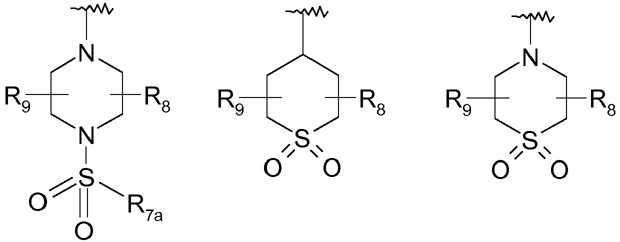 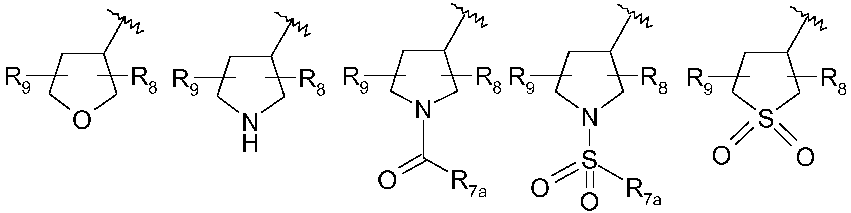 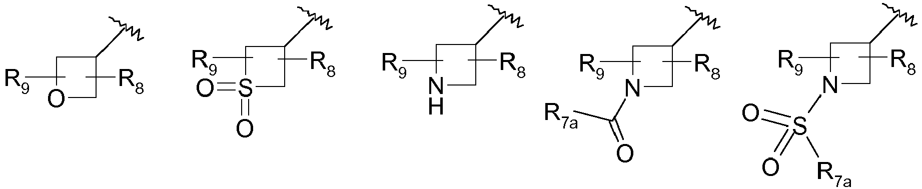 dans lesquels :
- R₇ₐ représente un radical alkyle, linéaire ou ramifié, en C₁-C₃, un radical alcoxy, linéaire ou ramifié, en C₁-C₃ ou un radical amino en N(R^{8a})(R^{8b}),
- R^{8a} et R^{8b}, identiques ou différents, désignent un atome d'hydrogène, un radical alkyle, linéaire ou ramifié, en C₁-C₃ ou un radical cyclopropyle,
- R₈ et R₉, identiques ou différents, représentent un atome d'hydrogène, un radical alkyle, linéaire ou ramifié, en C₁-C₃, un groupement hydroxy -OH, une fonction carbonyle =O, un radical hydroxyalkyle en C₁(-CH₂OH), un groupe amino NH₂,
- R₈ et R₉ peuvent former ensemble avec les atomes de carbone auxquels ils sont attachés un cycle carbocyclique comportant de 5 à 7 chaînons.

Selon un mode de réalisation, dans la formule (I), R⁷ représente un radical aromatique ou hétéroaromatique choisi parmi : 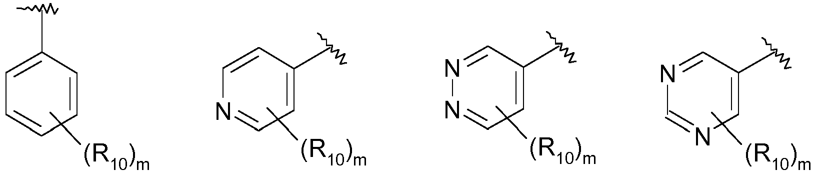 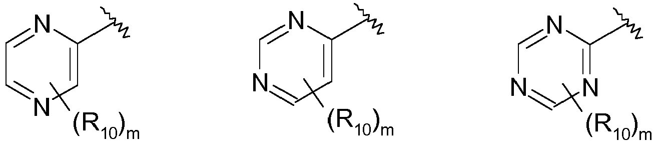 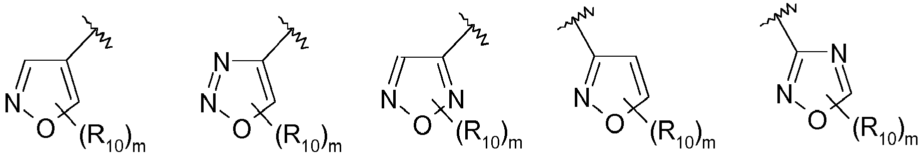 dans lesquels :
- R₁₀ représente un atome d'hydrogène ou un atome d'halogène, un groupement alkyle en C₁-C₃, linéaire ou ramifié, éventuellement substitué par un ou plusieurs atomes d'halogène ; un groupement alcoxy en C₁-C₃, un groupement amino -NR¹¹R¹², un groupement -COR¹¹, un groupement -COOR¹¹, un groupement amido - CONR¹¹R¹², un groupement -SOR¹¹, un groupement -SO₂R¹¹, un groupement -NHCOR¹¹, un groupement -NHCOOR¹¹, un groupement - SO₂NR¹¹R¹² ou un groupement -CN ; R¹¹ et R¹², identiques ou différents, représentant un atome d'hydrogène ou un radical alkyle, linéaire ou ramifié, en C₁-C₃ éventuellement substitué par un ou plusieurs atomes d'halogène,
- m désigne zéro ou un entier naturel allant de 1 à 3.

Préférentiellement, R⁷ représente un radical aromatique ou hétéroaromatique tel que défini précédemment éventuellement substitué par un ou plusieurs groupements méthyle -CH₃, un ou plusieurs groupements méthoxy -OCH₃, un ou plusieurs groupements hydroxy - OH, un ou plusieurs groupements amino -NH₂, un ou plusieurs groupements -CH₂OH, un ou plusieurs groupements cyano -CN, un ou plusieurs atomes d'halogène ou une ou plusieurs fonctions carbonyles.

Selon un mode de réalisation, l'indice m vaut zéro.

Selon un mode de réalisation, l'indice m désigne un entier naturel allant de 1 à 3.

Préférentiellement, l'indice m vaut 1.

Selon un mode de réalisation, dans la formule (I), chacun des éléments Y¹, Y², Y³, Y⁴ et Y⁵ correspond à un groupement -CR² avec R² ayant la même signification que celle décrite précédemment.

Selon un mode de réalisation, dans la formule (I), chacun des éléments Y¹, Y², Y³, Y⁴ et Y⁵ correspond à un groupement -CR² avec R² représentant un atome d'hydrogène.

Selon un mode de réalisation, dans la formule (I), chacun des éléments Y¹, Y², Y³, Y⁴ et Y⁵ correspond à un groupement -CR² avec R² représentant un radical alkyle, linéaire ou ramifié en C₁-C₅.

Selon un mode de réalisation, dans la formule (I), chacun des éléments Q¹, Q² et Q³ représente un groupement -CR^{2a} avec R^{2a} ayant la même signification que celle décrite précédemment.

Selon un mode de réalisation, dans la formule (I), chacun des éléments Q¹, Q² et Q³ représente un groupement -CR^{2a} avec R^{2a} représentant un atome d'hydrogène.

Selon un mode de réalisation, dans la formule (I), Q¹ et Q² représentent un groupement -CR^{2a} avec R^{2a} représentant un atome d'hydrogène et Q³ représente un groupement -CR^{2a} avec R^{2a} représentant un radical alkyle, linéaire ou ramifié, en C₁-C₅.

Selon un mode de réalisation, dans la formule (I), R¹ représente un radical alkyle, linéaire ou ramifié, en C₃-C₅, de préférence un radical alkyle ramifié en C₃-C₅, plus préférentiellement ramifié en C₄.

Selon un mode de réalisation, dans la formule (I), R¹ représente un radical cycloalkyle en C₃-C₅, de préférence cyclopropyle.

Selon un mode de réalisation, dans la formule (I), R¹ représente un radical alkényle, linéaire ou ramifié, en C₂-C₅.

Selon un mode de réalisation, dans la formule (I), R¹ représente un radical CH₂-cycloalkyle en C₃-C₅.

Selon un mode de réalisation, dans la formule (I), R¹ représente un radical hétérocycloalkyle en C₄-C₅.

Selon un mode de réalisation, dans la formule (I), R¹ représente un radical CH₂-hétérocycloalkyle en C₄-C₆, en particulier un radical CH₂-hétérocycloalkyle en C₄-C₅.

Préférentiellement, R¹ représente un radical alkyle, linéaire ou ramifié, en C₃-C₅ ou un radical CH₂-hétérocycloalkyle en C₄-C₅.

Selon un mode de réalisation, R⁵ représente un atome d'hydrogène.

De préférence, le ou les composés de formule (I) est ou sont choisis parmi le ou les composés de formule (IV), leurs sels d'addition pharmaceutiquement acceptables, leurs hydrates et/ou leurs solvates :

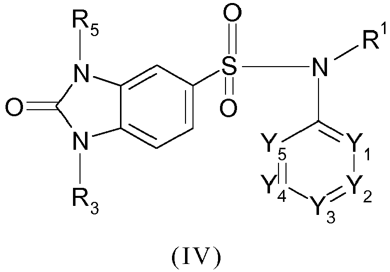

Formule (IV) dans laquelle R¹, R³, R⁵ et Y¹ à Y⁵ ont les mêmes significations que dans la formule (I) précédemment décrite

Préférentiellement, R¹ représente un radical alkyle, linéaire ou ramifié, en C₃-C₅ ou un radical cycloalkyle en C₃-C₅. Plus préférentiellement, R¹ représente un radical alkyle, linéaire ou ramifié, en C₃-C₅, notamment ramifié.

De préférence, Y¹-Y² et Y⁴-Y⁵ correspondent à un groupement - CR² avec R² désignant un atome d'hydrogène et Y³ correspond à un groupement -CR² avec R² désignant un radical alkyle, linéaire ou ramifié, en C₁-C₅, de préférence en C₂.

De préférence, Y¹ et Y³, identiques ou différents, correspondent à un groupement -CR² avec R² désignant un atome d'hydrogène ou un radical alkyle, linéaire ou ramifié, en C₁-C₅, de préférence en C₂.

Préférentiellement, Y¹ et Y³ sont identiques et correspondent à un groupement -CR² avec R² désignant un atome d'hydrogène ou un radical alkyle, linéaire ou ramifié, en C₁-C₅, de préférence en C₂.

De préférence, R⁵ représente un atome d'hydrogène.

De préférence, R³ représente un groupement (CHR⁶)ₙ-(Z)ₒ-(CHR'⁶)ₚ-R⁷ avec les indices n, p, o, R⁶, R'⁶ et R⁷ ayant les significations précédemment indiquées, et de préférence R⁷ est choisi parmi les radicaux hétérocycles et les radicaux aromatiques ou hétéroaromatiques précédemment décrits.

Plus préférentiellement, R³ représente un groupement CH₂-R⁷.

Selon un mode de réalisation, R¹ représente un radical alkyle, linéaire ou ramifié, en C₃-C₅, R⁵ représente un atome d'hydrogène et R³ représente un groupement (CHR⁶)ₙ-(Z)ₒ-(CHR'⁶)ₚ-R⁷ avec les indices n, p, o, R⁶, R'⁶ et R⁷ ayant les significations précédemment indiquées.

Les composés de formule (I) peuvent se présenter sous la forme de sels pharmaceutiquement acceptables. Des exemples de sels pharmaceutiquement acceptables sont décrits dans Berge et al., 1977, «sels pharmaceutiquement acceptables», J. Pharm. Sci., Vol. 66, pp 1 - 19.

En particulier, lorsque que les composés de formule selon l'invention se présentent sous la forme de sels alors l'électroneutralité desdits composés est assurée par un contre ion cationique Y externe pouvant être organique ou minérale.

Y peut être choisi parmi les cations inorganiques appropriés tels que les ions de métaux alcalins, notamment Na⁺, K⁺, les ions métaux alcalino-terreux, notamment Ca²⁺, Mg²⁺, ou encore d'autres cations tels que l'ion aluminium Al³⁺.

Y peut être choisi parmi les cations organiques appropriés tels que l'ion ammonium NH₄⁺, les ions ammonium substitués tels que NH₃R⁺, NHR₂⁺, NR₄⁺ avec R représentant un radical alkyle en C₁-C₄.

En particulier, les ions ammonium substitués sont ceux choisis parmi les dérivés de l'éthylamine, la diéthylamine, la dicyclohexylamine, la triéthylamine, la butylamine, l'étylènediamine, l'éthanolamine, la diéthanolamine, la pipérazine, la benzylamine, la phénylbenzylamine, la choline, la meglumine, et trométhamine, les acides aminés tels que la lysine et l'arginine.

Un exemple d'un ion ammonium quaternaire peut être l'ion N⁺ (CH₃)₄.

Le ou les composés selon l'invention peuvent se présenter sous la forme de leurs solvates.

Au sens de la présente invention, le terme « solvate » signifie un complexe de soluté (c'est-à-dire le composé selon l'invention ou le sel dudit composé) et de solvant.

Si le solvant est l'eau alors le solvate peut être commodément considéré comme un hydrate, par exemple, un semi-hydrate, un monohydrate, un dihydrate, un trihydrate, etc.

Par exemple, les solvates et/ou hydrates peuvent être obtenus directement à la fin du processus de synthèse, le composé cible étant isolé sous la forme d'un hydrate, par exemple un monohydrate ou hémi-hydrate, ou sous la forme d'un solvate du solvant de réaction et/ou du solvant de purification.

Sauf indication contraire, toute référence à un composé selon l'invention inclut également le solvate ou l'hydrate du composé correspondant.

Des procédures typiques pour la préparation et l'identification des hydrates et des solvates sont bien connus de l'homme du métier, voir par exemple, pages 202-209 de KJ Guillory, « Génération of Polymorphs, Hydrates, Solvates, and Amorphous Solids » dans Polymorphism in Pharmaceutical Solids, edition. Harry G. Britain, Vol. 95, Marcel Dekker, Inc., New York, 1999.

Les hydrates et les solvates peuvent être isolés et caractérisés par des méthodes connues dans l'art telles que l'analyse thermogravimétrique (TGA), la spectroscopie TGA-masse, la spectroscopie TGA-infrarouge, la diffraction de poudres aux rayons X, le titrage de Karl Fisher, la diffraction haute résolution aux rayons X et analogue.

De préférence, le ou les composés de formule (I) sont choisis parmi les composés tels que décrits dans les tableaux ci-après, ainsi que leurs sels d'addition pharmaceutiquement acceptable, leurs hydrates et/ou leurs solvates :

**Tableau 1 :**

| | | IC50 hRORg | IC50 hCD4/IL17 |
|---|---|---|---|
| 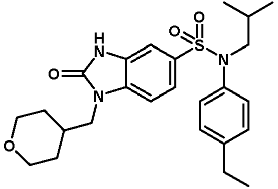 | Acide 2-oxo-1-(tétrahydro-pyran-4-ylméthyl)-2,3-dihydro-1H-benzoimidazole-5-sulfonique (4-éthyl-phényl)-isobutyl-amide | A | A |
| | Composé 2 | | |

**Tableau 2 :**

| | | IC50 hRORg | IC50 hCD4/IL17 |
|---|---|---|---|
| 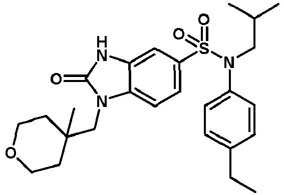 | Acide 1-(4-méthyl-tétrahydro-pyran-4-ylméthyl)-2-oxo-2,3-dihydro-1H-benzoimidazole-5-sulfonique (4-éthyl-phényl)-isobutyl-amide | A | A |
| | Composé 4 | | |
| 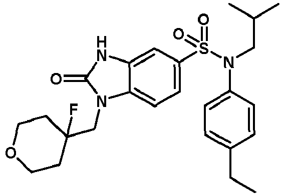 | Acide 1-(4-fluoro-tétrahydro-pyran-4-ylméthyl)-2-oxo-2,3-dihydro-1H-benzoimidazole-5-sulfonique(4-éthyl-phényl)-isobutyl-amide | A | A |
| | Composé 5 | | |
| 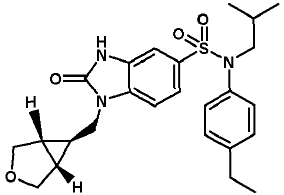 | Acide 1-[(1R,5S,6S)-1-(3-oxa-bicyclo[3.1.0]hex-6-yl)methyl]-2-oxo-2,3-dihydro-1H-benzoimidazole-5-sulfonique(4-éthyl-phényl)-isobutyl-amide | A | ND |
| | Composé 6 | | |
| 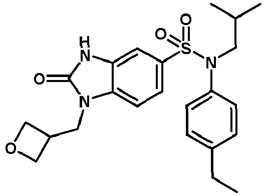 | Acide 1-oxetan-3-ylméthyl-2-oxo-2,3-dihydro-1H-benzoimidazole-5-sulfonique(4-éthyl-phényl)-isobutyl-amide | A | A |
| | Composé 7 | | |
| 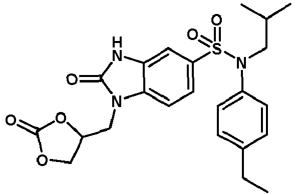 | Acide 2-oxo-1-(2-oxo-[1,3]-dioxolan-4-ylmethyl)-2,3-dihydro-1H-benzoimidazole-5-sulfonique(4-éthyl-phényl)-isobutyl-amide | A | ND |
| | Composé 8 | | |
| 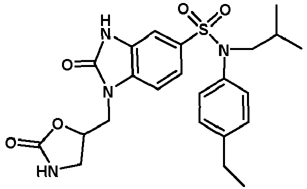 | Acide 2-oxo-1-(2-oxo-oxazolidin-5-ylmethyl)-2,3-dihydro-1H-benzoimidazole-5-sulfonique(4-éthyl-phényl)-isobutyl-amide | C | ND |
| | Composé 9 | | |
| 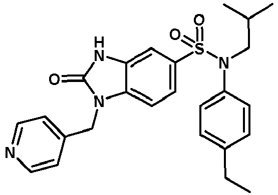 | Acide 2-oxo-1-pyridin-4-ylméthyl-2,3-dihydro-1H-benzoimidazole-5-sulfonique(4-éthyl-phényl)-isobutyl-amide | B | A |
| | Composé 10 | | |
| 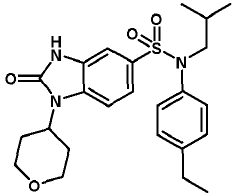 | Acide 2-oxo-1-(tetrahydro-pyran-4-yl)-2,3-dihydro-1H-benzoimidazole-5-sulfonique(4-éthyl-phényl)-isobutyl-amide | C | ND |
| | Composé 11 | | |
| 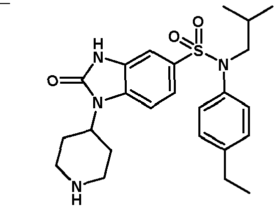 | Acide 2-oxo-1-piperidin-4-yl-2,3-dihydro-1H-benzoimidazole-5-sulfonique(4-éthyl-phényl)-isobutyl-amide | C | ND |
| | Composé 13 | | |
| 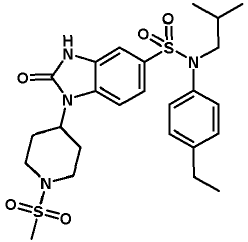 | Acide 1-(1-méthanesulfonyl-pipéridin-4-yl)-2-oxo-2,3-dihydro-1H-benzoimidazole-5-sulfonique(4-éthyl-phényl)-isobutyl-amide | C | ND |
| | Composé 14 | | |
| 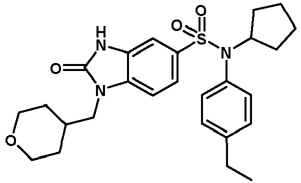 | Acide 2-oxo-1-(tétrahydro-pyran-4-ylmethyl)-2,3-dihydro-1H-benzoimidazole-5-sulfonique (4-éthyl-phényl)-cyclopentyl-amide | A | A |
| | Composé 15 | | |
| 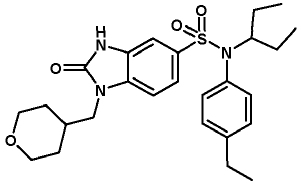 | Acide 2-oxo-1-(tétrahydro-pyran-4-ylméthyl)-2,3-dihydro-1H-benzoimidazole-5-sulfonique(4-éthyl-phényl)-(1-éthyl-propyl)-amide | A | A |
| | Composé 16 | | |
| 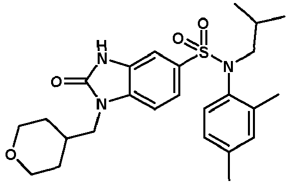 | Acide 2-oxo-1-(tetrahydro-pyran-4-ylmethyl)-2,3-dihydro-1H-benzoimidazole-5-sulfonique(2,4-diméthyl-phényl)-isobutyl-amide | A | A |
| | Composé 17 | | |
| 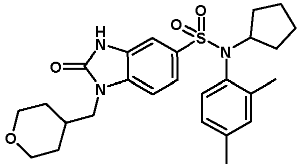 | Acide 2-oxo-1-(tétrahydro-pyran-4-ylméthyl)-2,3-dihydro-1H-benzoimidazole-5-sulfonique cyclopentyl-(2,4-diméthyl-phényl)-amide | A | A |
| | Composé 18 | | |
| 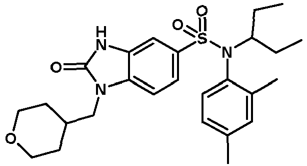 | Acide 2-oxo-1-(tétrahydro-pyran-4-ylméthyl)-2,3-dihydro-1H-benzoimidazole-5-sulfonique(2,4-diméthyl-phényl)-(1-éthyl-propyl)-amide | A | A |
| | Composé 19 | | |
| 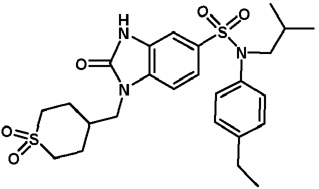 | Acide 1-(1,1-dioxo-hexahydro-1λ⁶-thiopyran-4-ylmethyl)-2-oxo-2,3-dihydro-1H-benzoimidazole-5-sulfonique (4-éthyl-phényl)-isobutyl-amide | A | A |
| | Composé 20 | | |
| 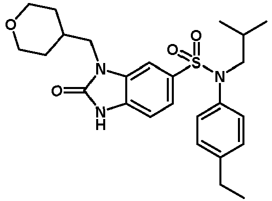 | Acide 2-oxo-3-(tétrahydro-pyran-4-ylmethyl)-2,3-dihydro-1H-benzoimidazole-5-sulfonique(4-éthyl-phényl)-isobutyl-amide | B | B |
| | Composé 21 | | |
| 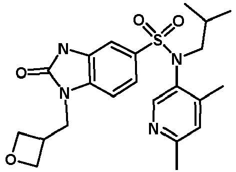 | Acide 1-oxetan-3-ylmethyl-2-oxo-2,3-dihydro-1H-benzoimidazole-5-sulfonique(4,6-dimethyl-pyridin-3-yl)-isobutyl-amide | ND | ND |
| | Composé 22 | | |
| 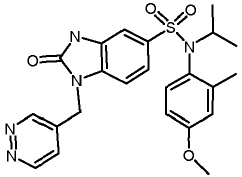 | Acide2-oxo-1-pyridazin-4-ylmethyl-2,3-dihydro-1H-benzoimidazole-5-sulfonique isopropyl-(4-methoxy-2-methyl-phényl)-amide | ND | ND |
| | Composé 23 | | |

| | | | |
|---|---|---|---|
| ND : non déterminé ; A : IC50 <100 nM.; B : IC50 = 100nM-1µM; C : IC50 > 1µM | | | |

Dans les tableaux décrits ci-avant, les concentrations inhibitrices médianes IC₅₀ pour les composés appartenant à la formule (I) selon l'invention ont été données selon les modèles suivants :

### Transactivation GAL4-RORγ

Le modèle de transactivation RORγ a été développé à partir de la lignée HG5LN qui est une lignée HeLa exprimant stablement un gène reporter luciférase contrôlé par un pentamère du domaine de reconnaissance GAL4 de levure et d'un promoteur β-globine. La lignée HG5LN a été transfectée stablement par le DNA-binding domain (DBD) (ou domaine de liaison à l'ADN) de GAL4 fusionné au ligand-binding domain (LBD) ROR gamma. Les molécules inhibant l'activité constitutive ROR gamma réduisent l'expression de la luciférase induisant ainsi une baisse de la luminescence émise.

Les cellules sont ensemencées en plaques 384 puits (5 000 cellules dans 45µL/puits de milieu de culture contenant 10% de sérum de veau foetal) et incubées pendant 4 heures à 37°C, 5% CO₂. 5µL des molécules à tester (composés décrits dans les tableaux décrits ci-avant) sont ensuite ajoutés à chaque puits et les plaques sont incubées pendant 18 heures à une température de 37°C sous 5% de CO₂. 20µL du substrat de la luciférase (Promega) sont ajoutés à chaque puits et la luminescence émise est lue par un lecteur de microplaques.

Les unités de luminescence ('RLU') sont normalisées par des contrôles positifs ('POS' contenant une concentration saturante de benzenesulfonamide, N-(2,2,2-trifluoroéthyl)-*N*-[4-[2,2,2-trifluoro-1-hydroxy-1-(trifluorométhyl)éthyl]phényl]) et des contrôles négatifs ('NEG' contenant du DMSO) : % inhibition=((RLU-NEG)*100)/(POS-NEG). Les IC50 sont calculées à partir d'un modèle logistique à 4 paramètres à l'aide du logiciel XLFit (IDBS).

### Sécrétion IL-17A

Ce modèle permet de mesurer l'effet d'inhibiteurs sur la sécrétion d'IL-17A par des cellules CD4+. Les cellules sont des CD4+ congelées (STEMCELL, # 70026), isolées de sang humain périphérique et activées par les anticorps anti-CD3 et anti-CD28. La quantité d'IL-17A sécrété est mesurée par la technologie TR-FRET (kit HTRF® Human Interleukin 17A (Cisbio, #64H17PEC)).

Les cellules sont décongelées rapidement, resuspendues dans leur milieu de culture (RPMI 10% SVF inactivé) supplémenté avec des anticorps anti-CD28 solubles et ensemencées (100 000 cellules/puits) dans des plaques 96 puits préalablement coatées avec des anticorps anti-CD3. Les cellules sont ensuite traitées par les gammes des inhibiteurs à tester (de 1000nM à 0.05nM, 0.1% DMSO). Après 4 jours d'incubation, le signal HTRF est mesuré à l'aide d'un lecteur de microplaque (λexcitation=337nm, λemission=620/665nm). Les ratios obtenus (665/620) sont normalisés par rapport au contrôle positif (cellules activées par anti-CD3 et anti-CD28, 0.1% DMSO). Les IC₅₀ sont calculées à partir d'un modèle logistique à 4 paramètres à l'aide du logiciel XLFit (IDBS).

Préférentiellement, les composés de formule (I) selon l'invention sont choisis parmi les composés suivants :

**Tableau 3 :**

| | |
|---|---|
| 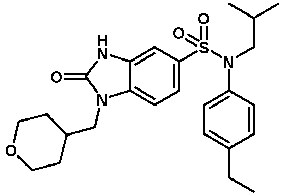 | Acide 2-oxo-1-(tétrahydro-pyran-4-ylméthyl)-2,3-dihydro-1H-benzoimidazole-5-sulfonique (4-éthyl-phényl)-isobutyl-amide |
| | Composé 2 |
| 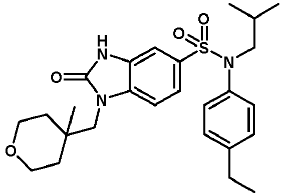 | Acide 1-(4-méthyl-tétrahydro-pyran-4-ylméthyl)-2-oxo-2,3-dihydro-1H-benzoimidazo le-5-sulfonique(4-éthyl-phényl)-isobutyl-amide |
| | Composé 4 |
| 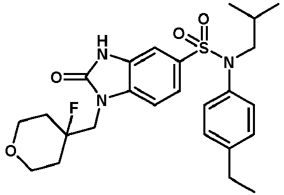 | Acide 1-(4-fluoro-tétrahydro-pyran-4-ylméthyl)-2-oxo-2,3-dihydro-1H-benzoimidazole-5-sulfonique(4-éthyl-phényl)-isobutyl-amide |
| | Composé 5 |
| 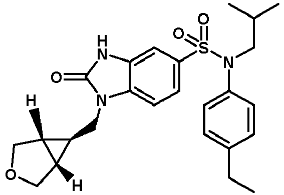 | Acide 1-[(1R,5S,6S)-1-(3-oxa-bicyclo[3.1.0]hex-6-yl)methyl]-2-oxo-2,3-dihydro-1H-benzoimidazole-5-sulfonique (4-éthyl-phényl)-isobutyl-amide |
| | Composé 6 |
| 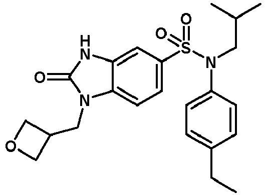 | Acide 1-oxetan-3-ylméthyl-2-oxo-2,3-dihydro-1H-benzoimidazole-5-sulfonique(4-éthyl-phényl)-isobutyl-amide |
| | Composé 7 |
| 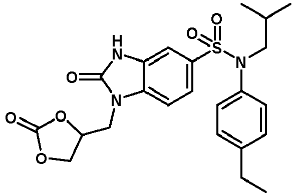 | Acide 2-oxo-1-(2-oxo-[1,3]-dioxolan-4-ylméthyl)-2,3-dihydro-1H-benzoimidazole-5-sulfonique (4-éthyl-phényl)-isobutyl-amide |
| | Composé 8 |
| 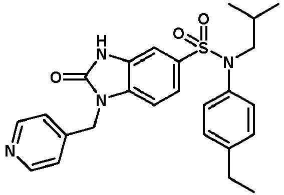 | Acide 2-oxo-1-pyridin-4-ylméthyl-2,3-dihydro-1H-benzoimidazole-5-sulfonique (4-éthyl-phényl)-isobutyl-amide |
| | Composé 10 |
| 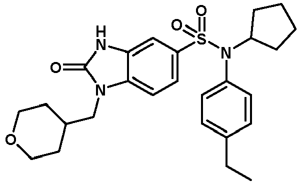 | Acide 2-oxo-1-(tétrahydro-pyran-4-ylmethyl)-2,3-dihydro-1H-benzoimidazole-5-sulfonique (4-éthyl-phényl)-cyclopentyl-amide |
| | Composé 15 |
| 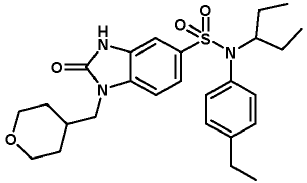 | Acide 2-oxo-1-(tétrahydro-pyran-4-ylméthyl)-2,3-dihydro-1H-benzoimidazole-5-sulfonique (4-éthyl-phényl)-(1-éthyl-propyl)-amide |
| | Composé 16 |
| 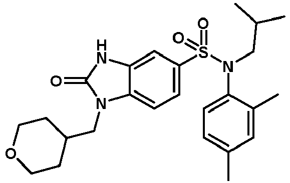 | Acide 2-oxo-1-(tetrahydro-pyran-4-ylmethyl)-2,3-dihydro-1H-benzoimidazole-5-sulfonique (2,4-diméthyl-phényl)-isobutyl-amide |
| | Composé 17 |
| 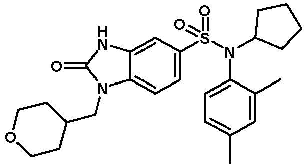 | Acide 2-oxo-1-(tétrahydro-pyran-4-ylméthyl)-2,3-dihydro-1H-benzoimidazole-5-sulfonique cyclopentyl-(2,4-diméthyl-phényl)-amide |
| | Composé 18 |
| 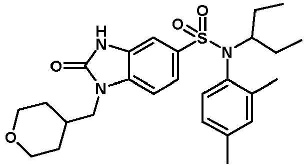 | Acide 2-oxo-1-(tétrahydro-pyran-4-ylméthyl)-2,3-dihydro-1H-benzoimidazole-5-sulfonique (2,4-diméthyl-phényl)-( 1-éthyl-propyl)-amide |
| | Composé 19 |

Ainsi les composés 2, 4 à 8, 10, et 15 à 19 sont préférés.

L'invention concerne également le ou les composés tels que décrits précédemment en tant que médicament et cosmétique.

De préférence, l'invention concerne également le ou les composés tels que décrits précédemment en tant que médicament.

En effet, les composés selon l'invention présentent des propriétés pharmacologiques intéressantes étant donné que lesdits composés modulent, c'est-à-dire inhibent, l'activité du récepteur RORγt.

Ainsi ces propriétés rendent le ou les composés de formule (I) telle que décrite précédemment utilisables en tant que médicament dans le traitement des maladies médiées par le récepteur RORγt.

De préférence, le ou les composés selon l'invention sont utilisés dans le traitement des désordres inflammatoires et/ou des maladies auto-immunes médiées par le récepteur RORγt.

Plus préférentiellement, le ou les composés selon l'invention, de préférence ceux choisis parmi les composés répondant aux formules (I), sont utilisés dans le traitement de l'acné, le psoriasis et/ou la dermatite atopique.

Selon un autre mode de réalisation, les composés selon l'invention sont utilisés pour le traitement cosmétique de la peau.

Comme indiqué ci-avant, la présente invention est aussi relative à une composition pharmaceutique comprenant, dans un milieu pharmaceutiquement acceptable, un ou plusieurs composés de formule (I) telle que définie précédemment, leurs sels d'addition pharmaceutiquement acceptables, leurs hydrates et/ou leurs solvates.

Plus préférentiellement, la composition pharmaceutique comprend un ou plusieurs composés de formule (I) choisis parmi les composés (1) à (20) définis précédemment.

Encore plus préférentiellement, la composition pharmaceutique comprend un ou plusieurs composés de formule (I) choisis parmi les composés 2, 4 à 8, 10 et 15 à 19.

L'administration de la composition pharmaceutique selon l'invention telle que décrite précédemment peut être effectuée par voie orale ou topique.

De préférence, la composition pharmaceutique est conditionnée sous une forme convenant à une application par voie topique.

Par voie orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée.

Par voie topique, la composition pharmaceutique selon l'invention est plus particulièrement destinée au traitement de la peau et des muqueuses et peut se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de suspensions, de sticks, de shampoings, ou de bases lavantes. Elle peut également se présenter sous forme de suspensions de microsphères ou nanosphères ou de vésicules lipidiques ou polymériques ou de patches polymériques ou gélifiés permettant une libération contrôlée.

La composition pharmaceutique est utilisée pour le traitement des désordres inflammatoires et/ou les maladies auto-immunes médiées par le récepteur RORγt.

Plus préférentiellement, la composition pharmaceutique est utilisée dans le traitement de l'acné et/ou le psoriasis.

L'invention concerne aussi un procédé de traitement des maladies médiées par le récepteur RORγt comprenant l'administration, notamment par voie topique ou orale, d'une quantité thérapeutiquement efficace de la composition pharmaceutique telle que définie ci-avant à un patient.

De préférence, la composition pharmaceutique est appliquée par voie topique.

Préférentiellement, l'invention a pour objet le ou les composés de formule (I) pour leur utilisation dans le traitement de l'acné.

En variante, l'invention a également pour objet le ou les composés de formule (I) pour leur utilisation dans le traitement du psoriasis.

Alternativement, le ou les composés de formule (I) selon l'invention sont utilisés pour le traitement cosmétique de la peau

L'invention concerne aussi la composition pharmaceutique telle que définie ci-avant pour son utilisation dans le traitement des désordres inflammatoires et/ou les maladies auto-immunes médiées par le récepteur RORγt, en particulier l'acné et/ou le psoriasis.

Les exemples suivants servent à illustrer l'invention sans toutefois présenter un caractère limitatif.

### EXEMPLES

La méthode standard LCMS pour l'analyse des produits est la suivante : colonne standard BEH C₁₈ (150*2.1mm, 1.8 µm) solvant eau/acétonitrile 0.1% acide formique.

Les purifications par HPLC préparative ont été réalisées sur colonne C₁₈, avec pour éluant : 85% d'acétonitrile dans eau/0.1% d'acide formique.

### Partie I : Synthèse des sulfonamides bicycliques à partir du schéma réactionnel 1

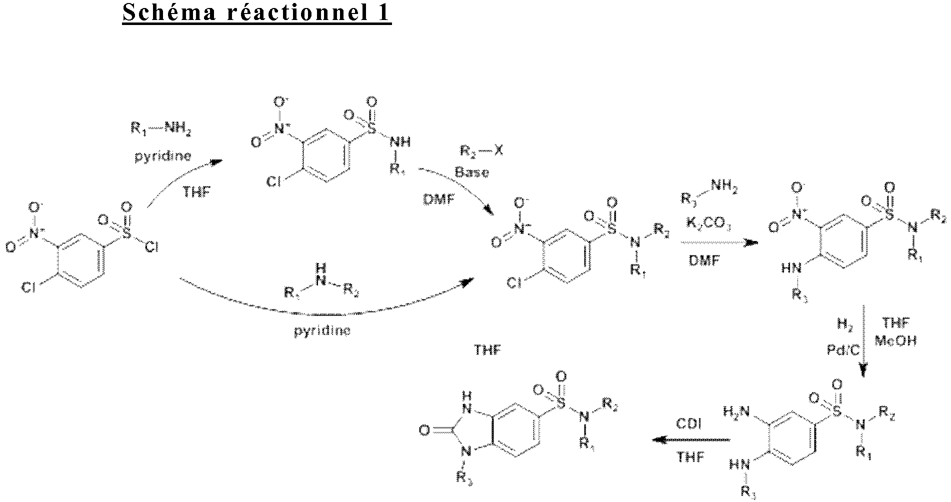

### Exemple 1 : Synthèse de l'acide 2-oxo-1-(tetrahydro-pyran-4-ylmethyl)-2,3-dihydro-1H-benzoimidazole-5-sulfonique (4-ethyl-phenyl)-isobutyl-amide

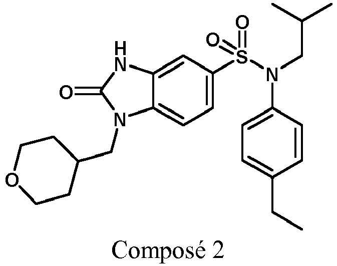

### 1. Synthèse de l'intermédiaire 1.1

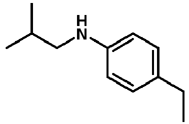

### (4-Ethyl-phenyl)-isobutyl-amine

A la 4-éthylaniline (9.48 ml; 0.08 mol) est ajouté l'isobutyraldehyde (6.33 ml; 0.07 mol.) dans le tétrahydrofurane (100ml). Le mélange est agité 2 heures à température ambiante. Puis le triacétoxy-borohydrure de sodium (22.04 g; 0.10 mol) est ajouté. Le mélange est agité pendant une nuit à température ambiante, additionné d'eau (100ml) et extrait à l'acétate d'éthyle (2 x 100ml). Les phases organiques sont rassemblées, lavées à la saumure (100ml), séchées (Na₂SO₄) et concentrées.

Le produit brut est chromatographié sur gel de silice (éluant heptane/dichlorométhane de 0 à 50% de dichlorométhane). La (4-éthyl-phényl)-isobutyl-amine est obtenue sous la forme d'une huile orange avec une RMN¹H conforme.
MS : [M+H] = 179

### 2. Synthèse de l'intermédiaire 1.1

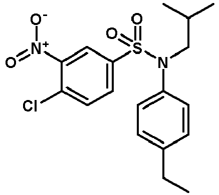

### 4-Chloro-N-(4-éthyl-phényl)-N-isobutyl-3-nitro-benzenesulfonamide

A une solution de (4-éthyl-phényl)-isobutyl-amine (0.68 g; 3.83 mmol) dans du tétrahydrofurane (15ml) sont ajoutés de la pyridine (1.85 ml; 22.96 mmol) et du chlorure de 4-chloro-3-nitrobenzene-sulfonyle (1.0 g; 3.83 mmol).

Le mélange est agité pendant 3 heures, hydrolysé et extrait à l'acétate d'éthyle. La phase organique est séchée sur Na₂SO₄ anhydre, filtrée et concentrée.

Le produit brut est chromatographié sur gel de silice (éluant heptane/acétate d'éthyle de 0 à 20% d'acétate d'éthyle). Le 4-chloro-N-(4-ethyl-phenyl)-N-isobutyl-3-nitro-benzenesulfonamide (1.42 g; 93%) est obtenu sous la forme d'un solide jaune avec une RMN¹H conforme.
MS : [M+H] = 397

### 3. Synthèse de l'intermédiaire 1.3

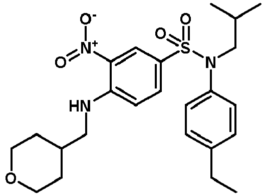

### N-(4-éthyl-phényl)-N-isobutyl-3-nitro-4-[(tétrahydro-pyran-4-ylméthyl)-amino]-benzenesulfonamide

Un mélange de 4-chloro-N-(4-ethyl-phenyl)-N-isobutyl-3-nitro-benzenesulfonamide (0.98 g; 2.47 mmol), de 4-aminomethyltétrahydropyrane (0.30 g; 2.59 mmol) et de carbonate de potassium (0.50 g; 3.62 mmol) dans du N,N-diméthylformamide (5 ml) est agité 4 heures à 60°C, hydrolysé et extrait à l'acétate d'éthyle. La phase organique est séchée (Na₂SO₄), filtrée et concentrée. Le produit brut est chromatographié sur gel de silice (éluant heptane/acétate d'éthyle de 10 à 50% d'acétate d'éthyle). Le N-(4-ethyl-phenyl)-N-isobutyl-3-nitro-4-[(tetrahydro-pyran-4-ylmethyl)-amino]-benzenesulfonamide (0.99 g; 84%) est obtenu sous la forme d'un solide jaune avec une RMN¹H conforme.
MS : [M+H] = 476

### 4. Synthèse de l'intermédiaire 1.4

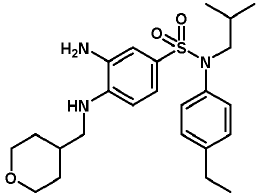

### 3-amino-N-(4-éthyl-phényl)-N-isobutyl-4-[(tétrahydro-pyran-4-ylméthyl)-amino]-benzenesulfonamide

A une suspension de N-(4-éthyl-phényl)-N-isobutyl-3-nitro-4-[(tétrahydro-pyran-4-ylméthyl)-amino]-benzenesulfonamide (19.82 g; 32.50 mmol) dans tétrahydrofurane (120ml) et méthanol (120ml) est ajouté du Pd/C 10% (50% H₂O) (3.46 g; 1.63 mmol).

Le milieu réactionnel est agité la nuit sous atmosphère d'hydrogène, filtré sur célite qui est rincée plusieurs fois avec un mélange DCM/tétrahydrofurane/MeOH.

Le filtrat est concentré, repris dans du pentane et filtré. Le solide obtenu est rincé à nouveau au pentane puis une fois avec un mélange de pentane et d'un peu d'éther éthylique. Le 3-amino-N-(4-ethyl-phenyl)-N-isobutyl-4-[(tetrahydro-pyran-4-ylmethyl)-amino]-benzene-sulfonamide (11.77 g; 81%) est obtenu sous la forme d'un solide beige avec une RMN¹H conforme.
MS : [M+H] = 446

### 5. Synthèse du composé 2 selon l'invention

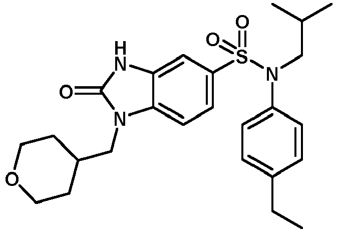

Le 3-amino-N-(4-éthyl-phényl)-N-isobutyl-4-[(tétrahydro-pyran-4-ylméthyl)-amino]-benzenesulfon-amide (1.00 g; 2.24 mmol) est solubilisé dans du tétrahydrofurane (10ml) avant d'ajouter le N,N'-carbonyl-diimidazole (1.09 g; 6.72 mmol). Le milieu réactionnel est agité pendant deux heures à température ambiante, hydrolysé et extrait à l'acétate d'éthyle. Les phases organiques sont rassemblées, lavées avec une solution saturée (NaHCO₃) et de l'eau puis, séchées (MgSO₄), filtrées et concentrées.

Le produit brut est chromatographié sur gel de silice (éluant dichlorométhane/méthanol de 0 à 8% de méthanol). L'acide 2-oxo-1-(tétrahydro-pyran-4-ylméthyl)-2,3-dihydro-1H-benzoimidazole-5-sulfonique (4-éthyl-phényl)-isobutyl-amide (785 mg; 74%) est obtenu sous la forme d'un solide cristallin blanc cassé après recristallisation dans un mélange acétone/eau
¹H NMR (DMSO-d₆) δ: 0.83 (d, J = 6.5 Hz, 6H), 1.17 (t, J = 7.6 Hz, 3H), 1.28 (qd, J = 12.2, 4.4 Hz, 2H), 1.39 (p, J = 6.7 Hz, 1H), 1.47 (d, J = 12.2 Hz, 2H), 2.00 (d, J = 12.5 Hz, 1H), 2.59 (q, J = 7.6 Hz, 2H), 3.21 (d, J = 11.3 Hz, 2H), 3.26 (d, J = 6.6 Hz, 2H), 3.72 (d, J = 7.2 Hz, 2H), 3.82 (dd, J = 12.2, 3.8 Hz, 2H), 6.96 (d, J = 8.0 Hz, 2H), 7.00 (s, 1H), 7.17 (d, J = 7.9 Hz, 2H), 7.20 (d, J = 8.3 Hz, 1H), 7.33 (d, J = 8.3 Hz, 1H), 11.16 (s, 1H)
MS : [M+H] = 472

### Exemple 2 : Synthèse de l'acide 1-(4-méthyl-tétrahydro-pyran-4-ylméthyl)-2-oxo-2,3-dihydro-1H-benzoimidazole-5-sulfonique(4-éthyl-ohényl)-isobutyl-amide

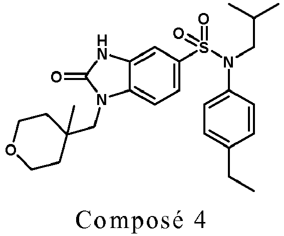

### 1. Synthèse de l'intermédiaire 2.1

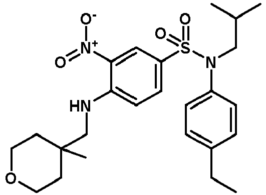

### N-(4-éthyl-phényl)-N-isobutyl-4-[(4-méthyl-tétrahydro-pyran-4-ylméthyl)-amino]-3-nitro-benzenesulfonamide

Avec un mode opératoire analogue à celui décrit pour l'intermédiaire 1.3, le N-(4-éthyl-phényl)-N-isobutyl-4-[(4-methyl-tetrahydro-pyran-4-ylmethyl)-amino]-3-nitro-benzenesulfonamid (100 mg; 27%) est obtenu sous la forme d'une huile jaune avec une RMN¹H conforme.
MS : [M+H] =490

### 2. Synthèse de l'intermédiaire 2.2

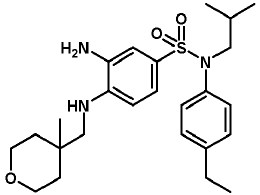

### 3-amino-N-(4-éthyl-phényl)-N-isobutyl-4-[(4-méthyl-tétrahydro-pyran-4-ylméthyl)-amino]-benzenesulfonamide

Avec un mode opératoire analogue à celui décrit pour l'intermédiaire 1.4, le 3-amino-N-(4-éthyl-phényl)-N-isobutyl-4-[(4-méthyl-tétrahydro-pyran-4-ylméthyl)-amino]-benzenesulfonamide (80 mg; 85%) est obtenu sous la forme d'une huile brune avec une RMN¹H conforme.
MS : [M+H] = 460

### 3. Synthèse du composé 4 (composé 269) selon l'invention

Avec un mode opératoire analogue à celui décrit dans l'exemple 1, l'acide 1-(4-méthyl-tétrahydro-pyran-4-ylmethyl)-2-oxo-2,3-dihydro-1H-benzoimidazole-5-sulfonique (4-éthyl-phényl)-isobutyl-amide (40 mg; 43%) est obtenu sous la forme d'un solide beige
¹H NMR (DMSO-d6) δ: 0.84 (d, J = 6.7 Hz, 6H), 1.03 (s, 3H), 1.18 (t, J = 7.6 Hz, 3H), 1.27 (d, J = 13.7 Hz, 2H), 1.40 (p, J = 6.8 Hz, 1H), 1.59 (s, 1H), 1.59 (dd, J = 23.5, 4.4 Hz, 1H), 2.53 - 2.66 (m, 3H), 3.27 (d, J = 7.3 Hz, 2H), 3.51 (t, J = 9.8 Hz, 2H), 3.64 - 3.74 (m, 4H), 6.93 - 7.04 (m, 3H), 7.14 - 7.24 (m, 3H), 7.35 (d, J = 8.4 Hz, 1H), 11.21 (s, 1H).
MS : [M+H] = 486

### Exemple 3 : Synthèse de l'acide 1-(4-fluoro-tétrahydro-pyran-4-ylméthyl)-2-oxo-2,3-dihydro-1H-benzoimidazole-5-sulfonique (4-éthyl-phényl)-isobutyl-amide

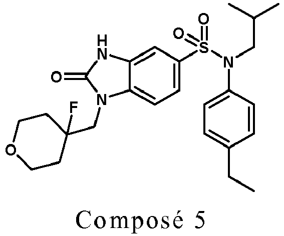

### 1. Synthèse de l'intermédiaire 3.1

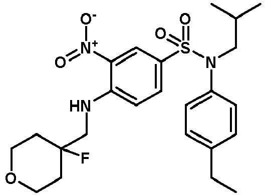

### N-(4-éthyl-phényl)-4-[(4-fluoro-tétrahydro-pyran-4-ylméthyl)-amino]-N-isobutyl-3-nitro-benzenesulfonamide

Un mélange de 4-chloro-N-(4-éthyl-phényl)-N-(1-éthyl-propyl)-3-nitro-benzenesulfonamide (200 mg; 0.50 mmol), de N,N-diméthylformamide (2.00 ml), de carbonate de potassium (77 mg; 0.55 mmol) et de (4-fluorooxan-4-yl)-methanamine (70.46 mg; 0.53 mmol) est agité pendant 2 heures à une température de 40°C, dilué à l'acétate d'éthyle. La phase organique est lavée avec une solution saturée de NH₄Cl, et de l'eau, séchée (MgSO₄), filtrée et concentrée à sec. Le N-(4-éthyl-phényl)-4-[(4-fluoro-tetrahydro-pyran-4-ylmethyl)-amino]-N-isobutyl-3-nitro-benzenesulfonamide (205 mg; 82%) est obtenu sous la forme d'une huile claire avec une RMN¹H conforme.
MS : [M+H] =494

### 2. Synthèse de l'intermédiaire 3.2

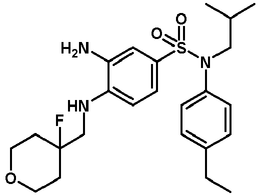

### 3-amino-N-(4-éthyl-phényl)-4-[(4-fluoro-tétrahydro-pyran-4-ylméthyl)-amino]-N-isobutyl-benzenesulfonamide

Avec un mode opératoire analogue à celui décrit pour l'intermédiaire 1.4, le 3-amino-N-(4-éthyl-phényl)-4-[(4-fluoro-tétrahydro-pyran-4-ylméthyl)-amino]-N-isobutyl-benzenesulfonamide (190 mg; 99%) est obtenu sous la forme d'une huile incolore avec une RMN¹H conforme.
MS : [M+H] = 465

### 3. Synthèse du composé 5 selon l'invention

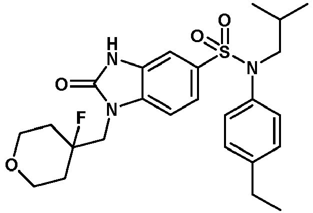

Avec un mode opératoire analogue à celui décrit dans l'exemple 1, l'acide 1-(4-fluoro-tétrahydro-pyran-4-ylméthyl)-2-oxo-2,3-dihydro-1H-benzoimidazole-5-sulfonique (4-éthyl-phényl)-isobutyl-amide (80 mg; 40%) est obtenu sous la forme d'un solide cristallin blanc.
¹H NMR (DMSO-d6) δ: 0.84 (d, J = 6.7 Hz, 6H), 1.18 (t, J = 7.6 Hz, 3H), 1.40 (p, J = 6.8 Hz, 2H), 1.69 (t, J = 12.4 Hz, 2H), 1.74 - 1.94 (m, 1H), 2.61 (q, J = 7.6 Hz, 2H), 3.27 (d, J = 7.3 Hz, 2H), 3.52 (t, J = 10.7 Hz, 2H), 3.76 (d, J = 11.7 Hz, 2H), 4.08 (d, J = 22.1 Hz, 2H), 6.97 - 7.00 (m, 2H), 7.02 (d, J = 1.7 Hz, 1H), 7.18 (d, J = 8.3 Hz, 2H), 7.26 (td, J = 8.2, 1.7 Hz, 2H), 11.25 (s, 1H).
MS : [M+H] = 490

### Exemple 4 : Synthèse de l'acide 1-[(1R,5S,6S)-1-(3-oxa-bicyclo[3.1.0]hex-6-yl)méthyl]-2-oxo-2,3-dihydro-1H-benzoimidazole-5-sulfonique(4-éthyl-phényl)-isobutyl-amide

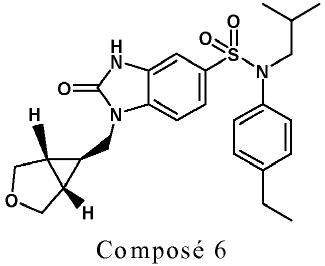

### 1. Synthèse de l'intermédiaire 4.1

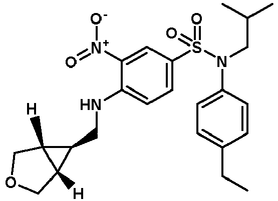

### N-(4-éthyl-phényl)-N-isobutyl-3-nitro-4-{[(1R,5S,6S)-1-(3-oxa-bicyclo[3.1.0]hex-6-yl)méthyl]-amino}-benzenesulfonamide

Avec un mode opératoire analogue à celui décrit pour l'intermédiaire 1.3, le N-(4-éthyl-phényl)-N-isobutyl-3-nitro-4-{[(1R,5S,6S)-1-(3-oxa-bicyclo[3.1.0]hex-6-yl)methyl]-amino}-benzenesulfonamide (238 mg; 100%) est obtenu sous la forme d'un solide jaune avec une RMN¹H conforme.
MS : [M+H] =474

### 2. Synthèse de l'intermédiaire 4.2

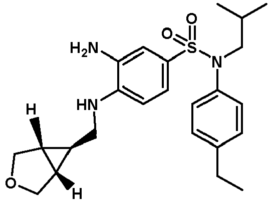

### 3-amino-N-(4-éthyl-phényl)-N-isobutyl-4-{[(1R,5S,6S)-1-(3-oxa-bicyclo[3.1.0]hex-6-yl)méthyl]-amino}-benzenesulfonamide

Avec un mode opératoire analogue à celui décrit pour l'intermédiaire 1.4, le 3-amino-N-(4-éthyl-phényl)-N-isobutyl-4-{[(1R,5S,6S)-1-(3-oxa-bicyclo[3.1.0]hex-6-yl)méthyl]-amino}-benzenesulfonamide (206 mg; 94%) est obtenu sous la forme d'une huile brune avec une RMN¹H conforme.
MS : [M+H] = 444

### 3. Synthèse du composé 6 selon l'invention

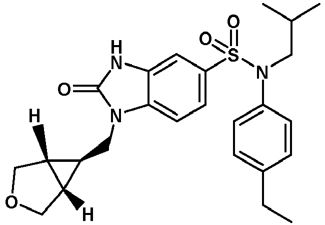

Avec un mode opératoire analogue à celui décrit dans l'exemple 1, l'acide 1-[(1R,5S,6S)-1-(3-Oxa-bicyclo[3.1.0]hex-6-yl)methyl]-2-oxo-2,3-dihydro-1H-benzoimidazole-5-sulfonique (4-ethyl-phenyl)-isobutyl-amide (56 mg; 25%) sous la forme d'un solide cristallin blanc cassé.
¹H NMR (DMSO-d6) δ: 0.84 (d, J = 6.7 Hz, 6H), 1.06 (dt, J = 7.1, 3.6 Hz, 1H), 1.18 (t, J = 7.6 Hz, 3H), 1.40 (dt, J = 13.6, 6.7 Hz, 1H), 1.75 (t, J = 2.6 Hz, 2H), 2.60 (q, J = 7.6 Hz, 2H), 3.25 - 3.30 (m, 2H), 3.53 (d, J = 8.3 Hz, 2H), 3.68 (d, J = 8.4 Hz, 2H), 3.79 (d, J = 7.1 Hz, 2H), 6.97 (d, J = 8.3 Hz, 2H), 7.02 (d, J = 1.8 Hz, 1H), 7.18 (d, J = 8.3 Hz, 2H), 7.23 (dd, J = 8.3, 1.8 Hz, 1H), 7.37 (d, J = 8.3 Hz, 1H), 11.14 (s, 1H).
MS : [M+H] = 470

### Exemple 5 : Synthèse de l'acide 1-oxetan-3-ylméthyl-2-oxo-2,3-dihydro-1H-benzoimidazole-5-sulfonique (4-éthyl-phényl)-isobutyl-amide

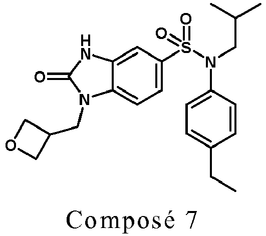

### 1. Synthèse de l'intermédiaire 5.1

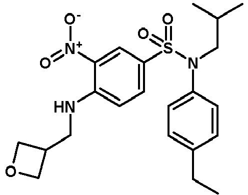

### N-(4-éthyl-phényl)-N-isobutyl-3-nitro-4-[(oxetan-3-ylmethyl)-amino]-benzenesulfonamide

Un mélange de 4-chloro-N-(4-ethyl-phenyl)-N-isobutyl-3-nitro-benzenesulfonamide (500 mg; 1.26 mmol), de 1-methyl-2-pyrrolidone (5ml), de carbonate de césium (1.03 g; 3.15 mmol) et d'hydro-chlorure de methyloxetane-3-ammonium- (187 mg; 1.51 mmol) est agité pendant 2 heures à température ambiante, dilué à l'acétate d'éthyle (20ml).

La phase organique est lavée avec une solution saturée de NH₄Cl (20ml), une solution saturée de NaHCO₃ (20ml) et de l'eau (20ml), séchée (MgSO₄), filtrée et concentrée à sec.

Le N-(4-éthyl-phényl)-N-isobutyl-3-nitro-4-[(oxetan-3-ylmethyl)-amino]-benzene-sulfonamide (520 mg; 92%) est obtenue sous la forme d'une huile jaune avec une RMN¹H conforme.
MS : [M+H] =450

### 2. Synthèse de l'intermédiaire 5.2

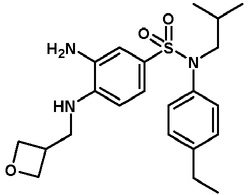

### 3-amino-N-(4-éthyl-phényl)-N-isobutyl-4-[(oxetan-3-ylmethyl)-amino]-benzenesulfonamide

Avec un mode opératoire analogue à celui décrit pour l'intermédiaire 1.4, le 3-amino-N-(4-éthyl-phényl)-N-isobutyl-4-[(oxetan-3-ylméthyl)-amino]-benzenesulfonamide (90 mg; 19%) est obtenu sous la forme d'une huile avec une RMN¹H conforme.
MS : [M+H] = 419

### 3. Synthèse du composé 7 selon l'invention

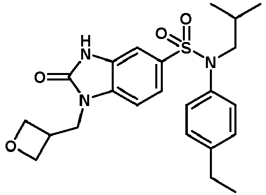

Avec un mode opératoire analogue à celui décrit pour l'exemple 1, l'acide 1-oxetan-3-ylméthyl-2-oxo-2,3-dihydro-1H-benzoimidazole-5-sulfonique (4-ethyl-phenyl)-isobutyl-amide (50 mg; 52%) sous la forme d'un solide beige
¹H NMR (DMSO-d6) δ: 0.84 (d, J = 6.7 Hz, 6H), 1.18 (t, J = 7.6 Hz, 3H), 1.40 (hept, J = 6.7 Hz, 1H), 2.60 (q, J = 7.6 Hz, 2H), 3.26 (d, J = 7.3 Hz, 2H), 4.15 (d, J = 7.2 Hz, 2H), 4.42 (t, J = 6.1 Hz, 2H), 4.63 (t, J = 7.0 Hz, 2H), 6.92 - 7.05 (m, 3H), 7.18 (d, J = 7.9 Hz, 2H), 7.23 (d, J = 8.3 Hz, 1H), 7.37 (d, J = 8.3 Hz, 1H).
MS : [M+H] = 444

### Exemple 6 : Synthèse de l'acide 2-oxo-1-(2-oxo-[1,3]-dioxolan-4-ylméthyl)-2,3-dihydro-1H-benzoimidazole-5-sulfonique (4-éthyl-phényl)-isobutyl-amide

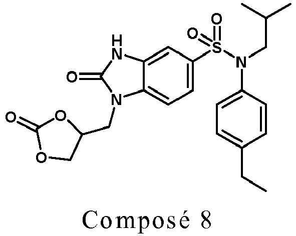

### 1. Synthèse de l'intermédiaire 6.1

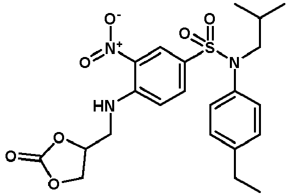

### N-(4-éthyl-phényl)-N-isobutyl-3-nitro-4-[(2-oxo-[1,3]dioxolan-4-ylméthyl)-amino]-benzenesulfonamide

Avec un mode opératoire analogue à celui décrit pour l'intermédiaire 1.3, le N-(4-éthyl-phényl)-N-isobutyl-3-nitro-4-[(2-oxo-[1,3]-dioxolan-4-ylmethyl)-amino]-benzenesulfonamide (300 mg; 100%) est obtenue sous la forme d'une huile brune avec une RMN¹H conforme.
MS : [M+H] =478

### 2. Synthèse de l'intermédiaire 6.2

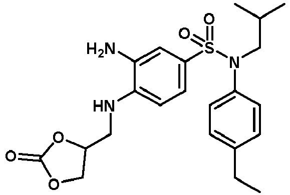

### 3-amino-N-(4-éthyl-phényl)-N-isobutyl-4-[(2-oxo-[1,3]-dioxolan-4-ylméthyl)-amino]-benzenesulfonamide

Avec un mode opératoire analogue à celui décrit pour l'intermédiaire 1.4, le 3-amino-N-(4-éthyl-phényl)-N-isobutyl-4-[(2-oxo-[1,3]-dioxolan-4-ylméthyl)-amino]-benzenesulfonamide (286 mg; 100%) est obtenu sous la forme d'une huile jaune pâle avec une RMN¹H conforme.
MS : [M+H] = 448

### 3. Synthèse du composé 8 selon l'invention

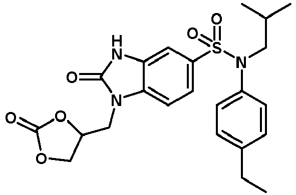

Avec un mode opératoire analogue à celui décrit pour l'exemple 1, l'acide 2-oxo-1-(2-oxo-[1,3]-dioxolan-4-ylméthyl)-2,3-dihydro-1H-benzoimidazole-5-sulfonique (4-éthyl-phényl)-isobutyl-amide (80 mg; 26%) sous la forme d'un solide cristallin blanc cassé.
¹H NMR (DMSO-d6) δ: 0.85 (dd, J = 6.6, 1.3 Hz, 6H), 1.18 (t, J = 7.6 Hz, 3H), 1.40 (dt, J = 13.5, 6.7 Hz, 1H), 2.60 (q, J = 7.6 Hz, 2H), 3.27 (d, J = 7.3 Hz, 2H), 4.19 (dd, J = 15.2, 3.5 Hz, 1H), 4.30 (dd, J = 15.2, 6.9 Hz, 1H), 4.37 (dd, J = 8.6, 6.2 Hz, 1H), 4.64 (t, J = 8.4 Hz, 1H), 5.10 - 5.19 (m, 1H), 6.93 - 6.99 (m, 2H), 7.02 (d, J = 1.7 Hz, 1H), 7.15 - 7.22 (m, 2H), 7.27 (dd, J = 8.4, 1.8 Hz, 1H), 7.40 (d, J = 8.4 Hz, 1H), 11.29 (s, 1H).
MS : [M+H] = 474

### Exemple 7 : Synthèse de l'acide 2-oxo-1-(2-oxo-oxazolidin-5-ylmethyl)-2,3-dihydro-1H-benzoimidazole-5-sulfonique (4-ethyl-phenyl)-isobutyl-amide

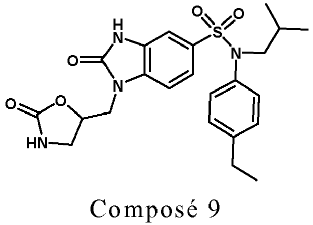

### 1. Synthèse de l'intermédiaire 7.1

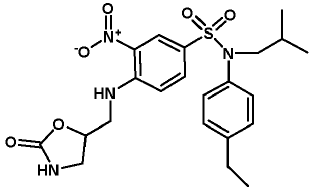

### N-(4-éthyl-phényl)-N-isobutyl-3-nitro-4-[(2-oxo-oxazolidin-5-ylmethyl)-amino]-benzenesulfonamide

Avec un mode opératoire analogue à celui décrit pour l'intermédiaire 1.3, le N-(4-éthyl-phényl)-N-isobutyl-3-nitro-4-[(2-oxo-oxazolidin-5-ylméthyl)-amino]-benzenesulfonamide (240 mg; 100%) est obtenue sous la forme d'une huile jaune avec une RMN¹H conforme. ²
MS : [M+H] =477

### 2. Synthèse de l'intermédiaire 7.2

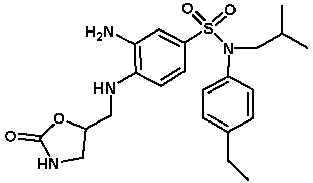

### 3-amino-N-(4-éthyl-phényl)-N-isobutyl-4-[(2-oxo-oxazolidin-5-ylméthyl)-amino]-benzenesulfonamide

Avec un mode opératoire analogue à celui décrit pour l'intermédiaire 1.4, le 3-amino-N-(4-éthyl-phényl)-N-isobutyl-4-[(2-oxo-oxazolidin-5-ylmethyl)-amino]-benzenesulfonamide (200 mg; 71%) est obtenu sous la forme d'une huile brune avec une RMN¹H conforme.
MS : [M+H] = 447

### 3. Synthèse du composé 9 selon l'invention

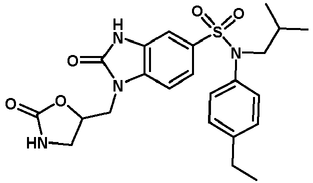

Avec un mode opératoire analogue à celui décrit dans l'exemple 1, l'acide 2-oxo-1-(2-oxo-oxazolidin-5-ylméthyl)-2,3-dihydro-1H-benzoimidazole-5-sulfonique (4-éthyl-phényl)-isobutyl-amide (37 mg; 18%) sous la forme d'un solide cristallin blanc.
¹H NMR (DMSO-d6) δ: 0.85 (dd, J = 6.7, 1.3 Hz, 6H), 1.18 (t, J = 7.6 Hz, 3H), 1.34 - 1.47 (m, 1H), 2.61 (q, J = 7.7 Hz, 2H), 3.27 (d, J = 7.3 Hz, 2H), 3.36 (d, J = 10.4 Hz, 2H), 3.61 (t, J = 8.9 Hz, 1H), 4.07 (dd, J = 14.9, 3.9 Hz, 1H), 4.16 (dd, J = 15.0, 6.9 Hz, 1H), 6.92 - 7.00 (m, 2H), 7.01 (d, J = 1.8 Hz, 1H), 7.19 (d, J = 8.3 Hz, 2H), 7.26 (dd, J = 8.3, 1.8 Hz, 1H), 7.37 (d, J = 8.3 Hz, 1H), 7.56 (s, 1H), 11.25 (s, 1H).
MS : [M+H] = 473

### Exemple 8 : Synthèse de l'acide 2-oxo-1-(2-oxo-tétrahydro-furan-3-ylméthyl)-2,3-dihydro-1H-benzoimidazole-5-sulfonique (4-éthyl-phényl)-isobutyl-amide

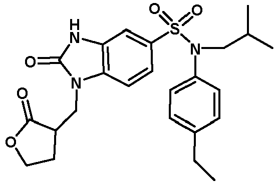

### 1. Synthèse de l'intermédiaire 8.1

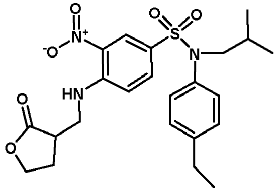

### N-(4-éthyl-phényl)-N-isobutyl-3-nitro-4-[(2-oxo-tétrahydro-furan-3-ylméthyl)-amino]-benzenesulfonamide

Avec un mode opératoire analogue à celui décrit pour l'intermédiaire 1.3, le N-(4-éthyl-phényl)-N-isobutyl-3-nitro-4-[(2-oxo-tétrahydro-furan-3-ylmethyl)-amino]-benzenesulfonamide (290 mg; 97%) est obtenue sous la forme d'une huile brune avec une RMN¹H conforme.
MS : [M+H] =476

### 2. Synthèse de l'intermédiaire 8.2

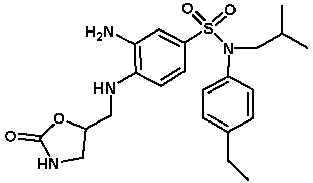

### 3-amino-N-(4-éthyl-phényl)-N-isobutyl-4-[(2-oxo-tétrahydro-furan-3-ylméthyl)-amino]-benzenesulfonamide

Avec un mode opératoire analogue à celui décrit pour l'intermédiaire 1.4, le 3-amino-N-(4-éthyl-phényl)-N-isobutyl-4-[(2-oxo-tétrahydro-furan-3-ylméthyl)-amino]-benzenesulfonamide (260 mg; 96%) est obtenu sous la forme d'une huile grise avec une RMN¹H conforme.
MS : [M+H] = 446

### 3. Synthèse de l'acide 2-oxo-1-(2-oxo-tetrahydro-furan-3-ylméthyl)-2,3-dihydro-1H-benzoimidazole-5-sulfonique(4-ethyl-phenyl)-isobutyl-amide

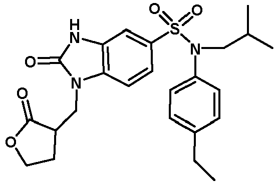

Avec un mode opératoire analogue à celui décrit dans l'exemple 1, l'acide 2-oxo-1-(2-oxo-tétrahydro-furan-3-ylmethyl)-2,3-dihydro-1H-benzoimidazole-5-sulfonique (4-éthyl-phényl)-isobutyl-amide (145 mg; 52%) sous la forme d'un solide blanc-cassé.
¹H NMR (DMSO-d6) δ: 0.84 (d, J = 6.6 Hz, 6H), 1.18 (t, J = 7.6 Hz, 3H), 1.38 (tt, J = 11.8, 5.8 Hz, 1H), 2.02 - 2.17 (m, 1H), 2.23 (dtt, J = 9.2, 6.7, 3.3 Hz, 1H), 2.60 (q, J = 7.6 Hz, 2H), 3.20 (ddt, J = 10.4, 8.6, 4.2 Hz, 1H), 3.27 (d, J = 7.3 Hz, 2H), 4.07 (dd, J = 14.5, 8.3 Hz, 1H), 4.12 - 4.20 (m, 2H), 4.33 (td, J = 8.5, 2.8 Hz, 1H), 6.93 - 7.00 (m, 2H), 7.01 (d, J = 1.7 Hz, 1H), 7.14 - 7.22 (m, 2H), 7.26 (dd, J = 8.3, 1.8 Hz, 1H), 7.37 (d, J = 8.3 Hz, 1H), 11.23 (s, 1H).
MS : [M+H] = 472

### Exemple 9 : Sythèse de l'acide 2-oxo-1-pyridin-4-ylméthyl-2,3-dihydro-1H-benzoimidazole-5-sulfonique(4-éthyl-phényl)-isobutyl-amide

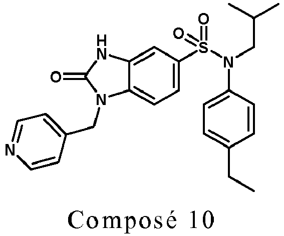

### 1. Synthèse de l'intermédiaire 9.1

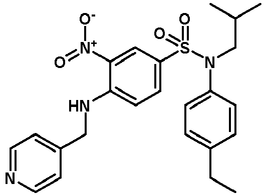

### N-(4-éthyl-phényl)-N-isobutyl-3-nitro-4-[(pyridin-4-ylméthyl)-amino]-benzene-sulfonamide

Un mélange de 4-chloro-N-(4-éthyl-phényl)-N-isobutyl-3-nitro-benzenesulfonamide (500 mg; 1.26 mmol), de tétrahydrofurane (10ml), de carbonate de césium (516 mg; 1.89 mmol) et de 4-picolylamine (409 mg; 3.78 mmol) est agité pendant 5 heures à une température de 60°C, dilué à l'acétate d'éthyle (20ml).

La phase organique est lavée avec une solution saturée de NH₄Cl (20ml), une solution saturée de NaHCO₃ (20ml) et de l'eau (20ml), séchée (MgSO₄), filtrée et concentrée à sec.

Le produit brut est chromatographié sur gel de silice (éluant heptane/acétate d'éthyle de 0 à 100% d'acétate d'éthyle). Le N-(4-éthyl-phényl)-N-isobutyl-3-nitro-4-[(pyridin-4-ylméthyl)-amino]-benzenesulfonamide (520 mg; 92%) est obtenue sous la forme d'un solide jaune avec une RMN¹H conforme.
MS : [M+H] =469

### 2. Synthèse de l'intermédiaire 9.2

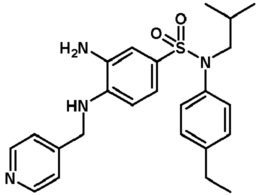

### 3-amino-N-(4-éthyl-phényl)-N-isobutyl-4-[(pyridin-4-ylméthyl)-amino]-benzene-sulfonamide

A une solution, dégazée sous argon, de N-(4-éthyl-phéenyl)-N-isobutyl-3-nitro-4-[(pyridin-4-ylméthyl)-amino]-benzene-sulfonamide (150mg; 0.32 mmol) dans du méthanol (5ml) est ajouté de l'oxyde (IV) de platine (7.27 mg; 0.03 mmol). Le milieu réactionnel est placé sous 1 atmosphère d'hydrogène et agité pendant 1 heure, filtré sur célite et concentré à sec.

Le 3-amino-N-(4-éthyl-phényl)-N-isobutyl-4-[(oxetan-3-ylméthyl)-amino]-benzenesulfonamide (140mg; 100%) est obtenu sous la forme d'une huile avec une RMN¹H conforme.
MS : [M-H] = 437

### 3. Synthèse du composé 10 (composé 275) selon l'invention

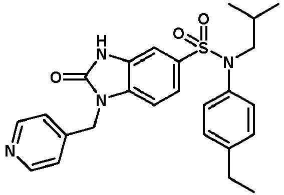

Avec un mode opératoire analogue à celui décrit pour l'exemple 1, l'acide 2-oxo-1-pyridin-4-ylmethyl-2,3-dihydro-1H-benzoimidazole-5-sulfonique (4-éthyl-phényl)-isobutyl-amide (70 mg; 44%) sous la forme d'un solide blanc
¹H NMR (DMSO-d6) δ: 0.83 (d, J = 6.6 Hz, 6H), 1.18 (t, J = 7.6 Hz, 3H), 2.55 - 2.64 (m, 3H), 3.26 (d, J = 7.3 Hz, 2H), 5.12 (s, 2H), 6.91 - 6.99 (m, 2H), 7.06 (d, J = 1.4 Hz, 1H), 7.14 - 7.19 (m, 2H), 7.20 - 7.23 (m, 2H), 7.25 - 7.28 (m, 2H), 8.51 - 8.56 (m, 2H), 11.36 (s, 1H).
MS : [M-H] = 463

### Exemple 10: Synthèse de l'acide 2-oxo-1-(tetrahydro-pyran-4-yl)-2,3-dihydro-1H-benzoimidazole-5-sulfonique (4-ethyl-phenyl)-isobutyl-amide

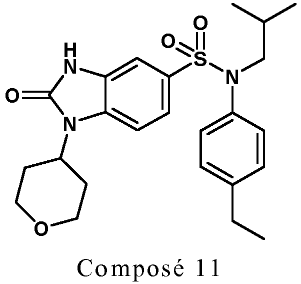

### 1. Synthèse de l'intermédiaire 10.1

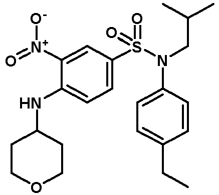

### N-(4-éthyl-phényl)-N-isobutyl-3-nitro-4-(tétrahydro-pyran-4-ylamino)-benzenesulfonamide

Avec un mode opératoire analogue à celui décrit pour l'intermédiaire 1.3, le N-(4-éthyl-phényl)-N-isobutyl-3-nitro-4-(tétrahydro-pyran-4-ylamino)-benzenesulfonamide (215 mg; 92%) est obtenue sous la forme d'une huile jaune avec une RMN¹H conforme. ²
MS : [M+H] =462

### 2. Synthèse de l'intermédiaire 10.2

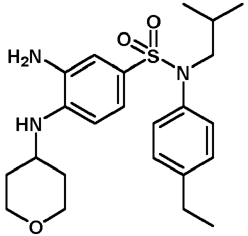

### 3-amino-N-(4-éthyl-phényl)-N-isobutyl-4-(tetrahydro-pyran-4-ylamino)-benzenesulfonamide

Avec un mode opératoire analogue à celui décrit pour l'intermédiaire 1.4, le 3-amino-N-(4-éthyl-phényl)-N-isobutyl-4-(tétrahydro-pyran-4-ylamino)-benzenesulfonamide (195 mg; 97%) est obtenu sous la forme d'une huile brune avec une RMN¹H conforme.
MS : [M+H] = 432

### 3. Synthèse du composé 11 selon l'invention

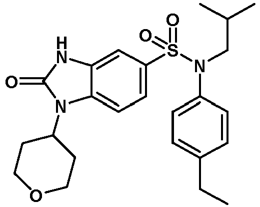

Avec un mode opératoire analogue à celui décrit dans l'exemple 1, l'acide 2-oxo-1-(tetrahydro-pyran-4-yl)-2,3-dihydro-1H-benzoimidazole-5-sulfonique (4-éthyl-phényl)-isobutyl-amide (37 mg; 18%) sous la forme d'un solide cristallin blanc cassé.
¹H NMR (DMSO-d6) δ: 0.84 (d, J = 6.6 Hz, 6H), 1.19 (t, J = 7.6 Hz, 3H), 1.39 (dq, J = 13.4, 6.7 Hz, 1H), 1.67 (d, J = 11.5 Hz, 2H), 2.30 - 2.45 (m, 2H), 2.61 (q, J = 7.6 Hz, 2H), 3.26 (d, J = 7.3 Hz, 2H), 3.48 (dd, J = 12.6, 10.6 Hz, 2H), 3.94 - 4.05 (m, 2H), 4.40 - 4.52 (m, 1H), 6.99 (d, J = 8.4 Hz, 2H), 7.02 (d, J = 1.8 Hz, 1H), 7.15 - 7.23 (m, 2H), 7.22 (dd, J = 8.4,1.8 Hz, 1H), 7.45 (d, J = 8.5 Hz, 1H), 11.16 (s, 1H).
MS : [M+H] = 458

### Exemple 11 : Synthèse du 4-{5-[(4-éthyl-phényl)-isobutyl-sulfamoyl]-2-oxo-2,3-dihydro-benzoimidazol-1-yl}-piperidine-1-carboxylate de tert-butyle

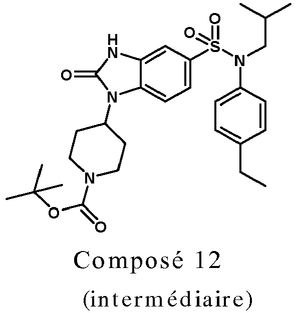

### 1. Synthèse de l'intermédiaire 11.1

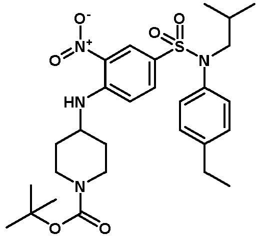

### 4- {4-[(4-éthyl-phényl)-isobutyl-sulfamoyl]-2-nitro-phénylamino}-piperidine-1-carboxylate de tert-butyle

Avec un mode opératoire analogue à celui décrit pour l'intermédiaire 1.3, le 4-{4-[(4-éthyl-phényl)-isobutyl-sulfamoyl]-2-nitro-phenylamino}-pipéridine-1-carboxylate de tert-butyle (353 mg; 100%) est obtenue sous la forme d'une huile brune avec une RMN¹H conforme.
MS : [M+H] =461

### 2. Synthèse de l'intermédiaire 11.2

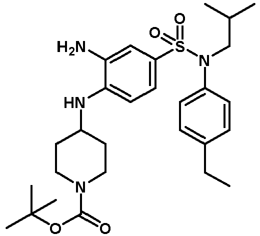

### 4-{2-amino-4-[(4-éthyl-phényl)-isobutyl-sulfamoyl]-phénylamino}-piperidine-1-carboxylate de tert-butyle

Avec un mode opératoire analogue à celui décrit pour l'intermédiaire 1.4, l'ester tert-butyl acide 4-{2-amino-4-[(4-éthyl-phényl)-isobutyl-sulfamoyl]-phénylamino}-pipéridine-1-carboxylique (340 mg; 100%) est obtenu sous la forme d'une huile brune avec une RMN¹H conforme.
MS : [M+H] = 531

### 3. Synthèse du composé 12

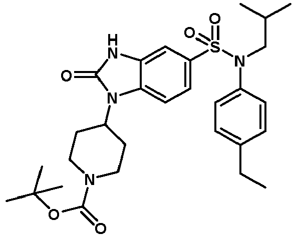

Avec un mode opératoire analogue à celui décrit dans l'exemple 1, le 4-{5-[(4-éthyl-phényl)-isobutyl-sulfamoyl]-2-oxo-2,3-dihydro-benzoimidazol-1-yl} -piperidine-1 -carboxylic acide tert-butyl ester (80 mg; 22%) sous la forme d'un solide cristallin beige.
¹H NMR (DMSO-d6) δ: 0.84 (d, J = 6.6 Hz, 6H), 1.19 (t, J = 7.6 Hz, 3H), 1.39 (dd, J = 13.6, 6.9 Hz, 1H), 1.44 (s, 9H), 1.72 (d, J = 12.4 Hz, 2H), 2.13 - 2.27 (m, 2H), 2.61 (q, J = 7.6 Hz, 2H), 2.88 (s, 2H), 3.25 (d, J = 7.4 Hz, 2H), 4.11 (s, 2H), 4.39 (t, J = 12.4 Hz, 1H), 6.98 (d, J = 8.4 Hz, 2H), 7.02 (d, J = 1.9 Hz, 1H), 7.17 - 7.22 (m, 3H), 7.40 (d, J = 8.5 Hz, 1H), 11.18 (s, 1H).
MS : [M+H] = 557

### Exemple 12 : Synthèse de l'acide 2-oxo-1-piperidin-4-yl-2,3-dihydro-1H-benzoimidazole-5-sulfonique (4-éthyl-phényl)-isobutyl-amide

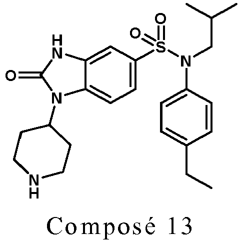

Un mélange d'ester tert-buty d'acide 4-{5-[(4-éthyl-phényl)-isobutyl-sulfamoyl]-2-oxo-2,3-dihydro-benzoimidazol-1-yl}-piperidine-1-carboxylique (85.0 mg; 0.15 mmol) dans du dichlorométhane (2ml) et d'acide trifluoroacétique (175.4 µl; 2.29 mmol) est agité pendant une nuit à température ambiante puis le milieu est concentré sous vide. L'acide 2-oxo-1-piperidin-4-yl-2,3-dihydro-1H-benzoimidazole-5-sulfonique (4-ethyl-phenyl)-isobutyl-amide (80 mg; 26%) est obtenu sous la forme d'un solide cristallin blanc cassé
¹H NMR (DMSO-d6) δ: 0.85 (d, J = 6.7 Hz, 6H), 1.14 - 1.23 (m, 3H), 1.41 (p, J = 6.8 Hz, 1H), 1.92 (d, J = 13.3 Hz, 2H), 2.61 (q, J = 7.6 Hz, 2H), 3.10 (d, J = 12.4 Hz, 2H), 3.26 (d, J = 7.3 Hz, 2H), 3.45 (d, J = 12.5 Hz, 2H), 4.57 (t, J = 12.1 Hz, 1H), 6.95 - 7.03 (m, 2H), 7.06 (d, J = 1.7 Hz, 1H), 7.16 - 7.23 (m, 2H), 7.26 (dd, J = 8.4, 1.8 Hz, 1H), 7.42 (d, J = 8.4 Hz, 1H), 8.59 (d, J = 11.0 Hz, 1H), 11.24 (s, 1H).
MS : [M+H] = 457

### Exemple 13: Synthèse de l'acide 1-(1-methanesulfonyl-piperidin-4-yl)-2-oxo-2,3-dihydro-1H-benzoimidazole-5-sulfonique (4-ethyl-phenyl)-isobutyl-amide

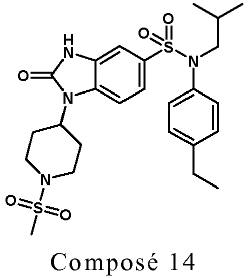

A une solution d'acide 2-oxo-1-pipéridin-4-yl-2,3-dihydro-1H-benzoimidazole-5-sulfonique (4-éthyl-phényl)-isobutyl-amide (60.00 mg; 0.13 mmol) dans du dichlorométhane (0.50 ml) sont rajoutés du chlorure de méthanesulfonyle (15.26 µl; 0.20 mmol) et de la triéthylamine (27.32 µl; 0.20 mmol). Le milieu réactionnel est hydrolysé, dilué au dichlorométhane et extrait. Les phases organiques sont lavées à l'eau, séchées (MgSO₄) et concentrées sous vide.

Le produit brut est purifié par HPLC préparative (colonne C18, éluant : acétonitrile dans eau/0.1% d'acide formique).

Après recristallisation dans un mélange acétone/eau, l'acide 1-(1-méthanesulfonyl-pipéridin-4-yl)-2-oxo-2,3-dihydro-1H-benzoimidazole-5-sulfonique (4-éthyl-phényl)-isobutyl-amide (10.00 mg; 14.11 %) est obtenu sous la forme d'un solide cristallin blanc cassé.
¹H 1H NMR (DMSO-d6) δ: 0.84 (d, J = 6.5 Hz, 6H), 1.19 (t, J = 7.6 Hz, 3H), 1.40 (dt, J = 13.7, 7.0 Hz, 1H), 1.84 (d, J = 12.2 Hz, 2H), 2.39 (dd, J = 12.5, 4.0 Hz, 2H), 2.61 (q, J = 7.6 Hz, 2H), 2.91 (d, J = 12.6 Hz, 2H), 2.95 (s, 3H), 3.26 (d, J = 7.3 Hz, 2H), 3.74 (d, J = 11.8 Hz, 2H), 4.29 - 4.46 (m, 1H), 6.96 - 7.04 (m, 3H), 7.21 (dd, J = 12.4, 8.5 Hz, 3H), 7.42 (d, J = 8.4 Hz, 1H), 11.20 (s, 1H).
MS : [M+H] = 535

Avec un mode opératoire analogue à celui de l'intermédiaire 1.1 correspondant à une amination réductrice entre 1 équivalent d'aldéhyde et 1.15 équivalent d'aniline dans du tétrahydrofurane en présence de 1.45 équivalent de triacétoxyborohydrure de sodium on obtient les anilines du tableau ci-après :

| | | |
|---|---|---|
| Intermédiaire 15.1 | 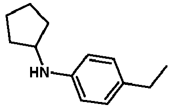 | Cyclopentylméthyl-(4-éthyl-phényl)-amine |
| | | (19.3 g; 56%) obtenu sous la forme d'une huile ambrée avec une RMN¹H conforme. |
| | | MS: [M+H] = 190 |
| Intermédiaire 16.1 | 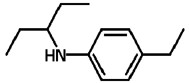 | (4-éthyl-phényl)-(1-éthyl-propyl)-amine |
| | | (700 mg; 98%) obtenu sous la forme d'une huile avec une RMN¹H conforme. |
| | | MS : [M+H] = 192 |
| Intermédiaire 17.1 | 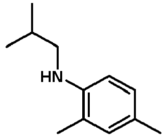 | (2,4-diméthyl-phenyl)-isobutyl-amine |
| | | (1.15 g; 83%) obtenu sous la forme d'une huile avec une RMN¹H conforme |
| | | MS: [M+H] = 179 |
| Intermédiaire 19.1 | 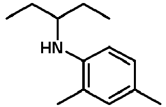 | 2,4-diméthyl-phenyl)-(1-éthyl-propyl)-aminé |
| | | (2.50 g; 7 obtenu sous la forme d'une huile brune avec une RMN¹H conforme |
| | | MS: [M+H] = 192. |

### Exemple 14: acide 2-oxo-1-(tetrahydro-pyran-4-ylmethyl)-2,3-dihydro-1H-benzoimidazole-5-sulfonique (4-ethyl-phenyl)-cyclopentyl-amide

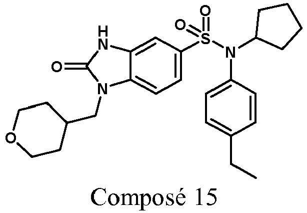

### 1. Synthèse de l'intermédiaire 14.2

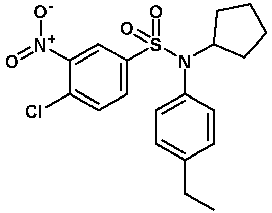

### 4-chloro-N-(4-éthyl-phényl)-N-cyclopentyl-3-nitro-benzenesulfonamide

Avec un mode opératoire analogue à celui décrit pour l'intermédiaire 1.2 appliqué à l'intermédiaire 14.1, le 4-chloro-N-cyclopentyl-N-(4-éthyl-phényl)-3-nitro-benzenesulfonamide (440 m g; 28%) est obtenu sous la forme d'un solide blanc cassé avec une RMN¹H conforme.
MS : [M+H] =409

### 2. Synthèse de l'intermédiaire 14.3

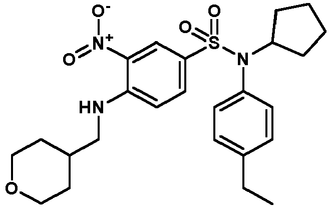

### N-(4-éthyl-phényl)-N-cyclopentyl-3-nitro-4-[(tétrahydro-pyran-4-ylmethyl)-amino]-benzenesulfonamide

Avec un mode opératoire analogue à celui décrit pour l'intermédiaire 1.3, le N-(4-éthyl-phényl)-N-icyclopentyl-3-nitro-4-[(tétrahydro-pyran-4-ylméthyl)-amino]-benzenesulfonamide (480 mg; 91%) est obtenu sous la forme d'un solide orangé avec une RMN¹H conforme.
MS : [M+H] =488

### 3. Synthèse de l'intermédiaire 14.4

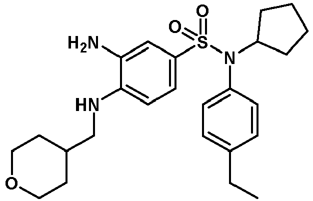

### 3-amino-N-(4-éthyl-phényl)-N-cyclopentyl-4-[(tétrahydro-pyran-4-ylmethyl)-amino]-benzenesulfonamide

Avec un mode opératoire analogue à celui décrit pour l'intermédiaire 1.4, le 3-amino-N-(4-éthyl-phényl)-N- cyclopentyl -4-[(tétrahydro-pyran-4-ylméthyl)-amino]-benzenesulfonamide (420 mg; 97%) est obtenu sous la forme d'un solide beige avec une RMN¹H conforme.
MS : [M+H] =458

### 4. Synthèse du composé 15 (composé 280) selon l'invention

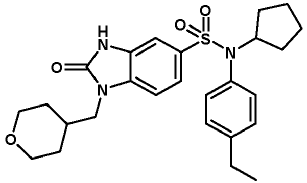

Avec un mode opératoire analogue à celui décrit dans l'exemple 1, l'acide 2-oxo-1-(tétrahydro-pyran-4-ylméthyl)-2,3-dihydro-1H-benzoimidazole-5-sulfonique (4-éthyl-phényl)-cyclopentyl-amide (262 mg; 59%) est obtenu sous la forme d'un solide cristallin blanc après recristallisation dans un mélange acétone/eau.
¹H NMR (DMSO-d6) δ: 1.19 (t, J = 7.6 Hz, 3H), 1.32 (tdd, J = 12.9, 7.2, 4.5 Hz, 3H), 1.41 (dd, J = 7.6, 4.7 Hz, 1H), 1.49 (d, J = 11.8 Hz, 1H), 1.73 (dd, J = 11.5, 6.5 Hz, 2H), 2.03 (ddd, J = 11.3, 7.6, 4.0 Hz, 1H), 2.62 (q, J = 7.6 Hz, 2H), 3.24 (td, J = 11.6, 2.1 Hz, 2H), 3.74 (d, J = 7.2 Hz, 2H), 3.80 - 3.87 (m, 2H), 4.43 (tt, J = 9.3, 7.3 Hz, 1H), 6.88 - 6.91 (m, 2H), 7.19 - 7.21 (m, 2H), 7.21 (d, J = 1.9 Hz, 1H), 7.35 - 7.43 (m, 2H), 11.19 (s, 1H)
MS : [M+H] = 484

### Exemple 15: Synthèse de l'acide 2-oxo-1-(tétrahydro-pyran-4-ylméthyl)-2,3-dihydro-1H-benzoimidazole-5-sulfonique (4-éthyl-phényl)-(l-éthyl-propyl)-amide

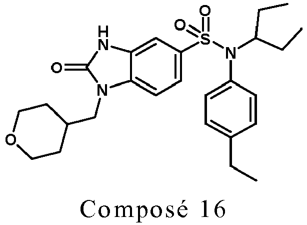

### 1. Synthèse de l'intermédiaire 15.2

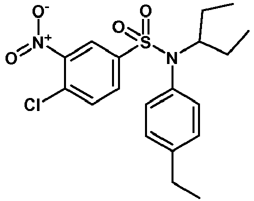

### 4-chloro-N-(4-éthyl-phényl)-N-(1-éthyl-propyl)-3-nitro-benzenesulfonamide

Avec un mode opératoire analogue à celui décrit pour l'intermédiaire 1.2 appliqué à l'intermédiaire 15.1, le 4-chloro-N-(4-éthyl-phényl)-N-(1 -éthyl-propyl)-3-nitro-benzenesulfonamide (900 mg; 88%) est obtenu sous la forme d'une huile orangée avec une RMN¹H conforme.
MS : [M+H] = 411

### 2. Synthèse de l'intermédiaire 15.3

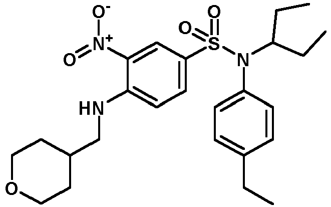

### N-(4-éthyl-phényl)-N-(1-ethyl-propyl)-3-nitro-4-[(tetrahydro-pyran-4-ylmethyl)-amino]-benzenesulfonamide

Avec un mode opératoire analogue à celui décrit pour l'intermédiaire 1.3, le N-(4-éthyl-phényl)-N-(1-éthyl-propyl)-3-nitro-4-[(tétrahydro-pyran-4-ylméthyl)-amino]-benzenesulfonamide (1.07g; 100%) est obtenu sous la forme d'une huile ambrée avec une RMN¹H conforme.
MS : [M+H] = 490

### 3. Synthèse de l'intermédiaire 15.4

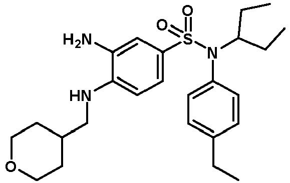

### 3-amino-N-(4-éthyl-phényl)-N-(1-éthyl-propyl)-4-[(tétrahydro-pyran-4-ylméthyl)-amino] -benzenesulfonamide

Par analogie au mode opératoire décrit pour l'intermédiaire 1.3, le 3-amino-N-(4-éthyl-phényl)-N-(1-éthyl-propyl)-4-[(tétrahydro-pyran-4-ylméthyl)-amino]-benzenesulfonamide (910 mg; 83%) est obtenu sous la forme d'une huile ambrée avec une RMN¹H conforme.
MS : [M+H] = 460

### 4. Synthèse du composé 16 selon l'invention

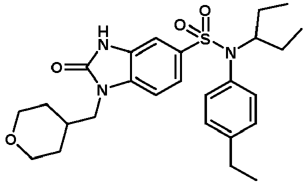

Avec un mode opératoire analogue à celui décrit dans l'exemple 1, l'acide 2-oxo-1-(tétrahydro-pyran-4-yl-méthyl)-2,3-dihydro-1H-benzoimidazole-5-sulfonique (4-éthyl-phényl)-(1-ethyl-propyl)-amide (120 mg; 12%) est obtenu sous la forme d'un solide cristallin blanc après recristallisation dans un mélange acétone/eau.
¹H NMR (DMSO-d6) δ: 0.87 (t, J = 7.3 Hz, 6H), 1.08 - 1.16 (m, 2H), 1.19 (t, J = 7.6 Hz, 3H), 1.29 (dddd, J = 14.4, 12.7, 7.1, 4.9 Hz, 4H), 1.43 - 1.51 (m, 2H), 2.02 (ddt, J = 11.5, 7.8, 3.8 Hz, 1H), 2.62 (q, J = 7.6 Hz, 2H), 3.23 (td, J = 11.6, 2.1 Hz, 2H), 3.74 (d, J = 7.2 Hz, 2H), 3.79 - 3.86 (m, 2H), 3.88 (ddd, J = 8.1, 5.7, 2.5 Hz, 1H), 6.88 - 6.92 (m, 2H), 7.16 (d, J = 1.2 Hz, 1H), 7.19 - 7.24 (m, 2H), 7.35 (d, J= 1.1 Hz, 2H), 11.19 (s, 1H)
MS : [M+H] = 486

### Exemple 16: Synthèse de l'acide 2-oxo-1-(tetrahydro-pyran-4-ylmethyl)-2,3-dihydro-1H-benzoimidazole-5-sulfonique (2,4-dimethyl-phenyl)-isobutyl-amide

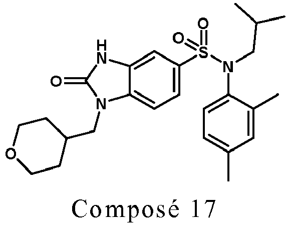

### 1. Synthèse de l'intermédiaire 16.2

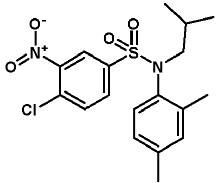

### 4-chloro-N-(2,4-diméthyl-phényl)-N-isobutyl-3-nitro-benzenesulfonamide

Avec un mode opératoire analogue à celui décrit pour l'intermédiaire 1.2 appliqué à l'intyermédiaiare 16.1, le 4-chloro-N-(2,4-diméthyl-phényl)-N-isobutyl-3-nitro-benzenesulfonamide (665 mg; 88%) est obtenu sous la forme d'une huile brune avec une RMN¹H conforme.
MS : [M-H] = 396

### 2. Synthèse de l'intermédiaire 16.3

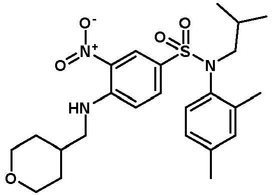

### N-(2,4-diméthyl-phényl)-N-isobutyl-3-nitro-4-[(tétrahydro-pyran-4-ylméthyl)-amino]-benzenesulfonamide

Avec un mode opératoire analogue à celui décrit pour l'intermédiaire 1.3, le N-(2,4-diméthyl-phényl)-N-isobutyl-3-nitro-4-[(tétrahydro-pyran-4-ylméthyl)-amino]-benzenesulfonamide (740 mg; 93%) est obtenu sous la forme d'une huile brune avec une RMN¹H conforme.
MS : [M+H] = 476

### 3. Synthèse de l'intermédiaire 16.4

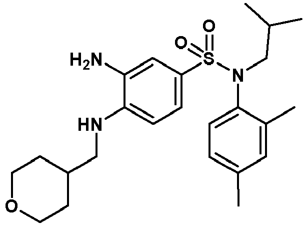

### 3-amino-N-(2,4-diméthyl-phényl)-N-isobutyl-4-[(tétrahydro-pyran-4-ylméthyl)-amino]-benzenesulfonamide

Par analogie au mode opératoire décrit pour l'intermédiaire 1.4, le 3-amino-N-(2,4-dimethyl-phenyl)-N-isobutyl-4-[(tetrahydro-pyran-4-ylmethyl)-amino]-benzenesulfonamide (693 g; 100%) est obtenu sous la forme d'une huile ambrée avec une RMN¹H conforme.
MS : [M+H] = 446

### 4. Synthèse du composé 17 selon l'invention

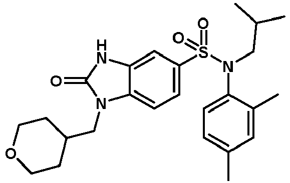

Avec un mode opératoire analogue à celui décrit dans l'exemple 1, l'acide 2-oxo-1-(tétrahydro-pyran-4-ylméthyl)-2,3-dihydro-1H-benzoimidazole-5-sulfonique (2,4-diméthyl-phényl)-isobutyl-amide (273 mg; 37%) est obtenu sous la forme d'un solide cristallin blanc après recristallisation dans un mélange acétone/eau.
¹H NMR (DMSO-d6) δ: 0.75 (d, J = 6.6 Hz, 3H), 0.94 (d, J = 6.5 Hz, 3H), 1.30 (qd, J = 12.1, 4.4 Hz, 2H), 1.40 (dtd, J = 9.0, 6.8, 4.8 Hz, 1H), 1.48 (dd, J = 13.6, 3.5 Hz, 2H), 2.03 (dtq, J = 11.2, 7.4, 3.9 Hz, 1H), 2.25 (d, J = 3.5 Hz, 6H), 3.04 (dd, J = 13.0, 4.6 Hz, 1H), 3.19 - 3.29 (m, 2H), 3.36 (dd, J = 13.0, 9.0 Hz, 1H), 3.75 (d, J = 7.2 Hz, 2H), 3.83 (dq, J = 11.6, 1.9 Hz, 2H), 6.52 (d, J = 8.1 Hz, 1H), 6.90 (dd, J = 8.1, 2.1 Hz, 1H), 7.09 (dd, J = 11.3, 1.9 Hz, 2H), 7.29 (dd, J = 8.3, 1.8 Hz, 1H), 7.38 (d, J = 8.3 Hz, 1H), 11.21 (s, 1H)
MS : [M+H] = 472

### Exemple 17: Synthèse de l'acide 2-oxo-1-(tetrahydro-pyran-4-ylméthyl)-2,3-dihydro-1H-benzoimidazole-5-sulfonique cyclopentyl-(2,4-diméthyl-phényl)-amide

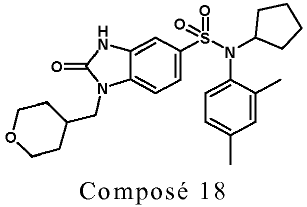

### 1. Synthèse de l'intermédiaire 17.1

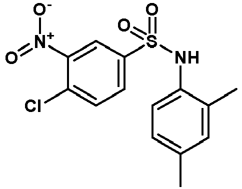

### 4-chloro-N-(2,4-diméthyl-phényl)-3-nitro-benzenesulfonamide

Avec un mode opératoire analogue à celui décrit pour l'intermédiaire 1.2, le 4-chloro-N-(2,4-diméthyl-phényl)-3-nitro-benzenesulfonamide (180 mg; 28%) est obtenu sous la forme d'une huile brune avec une RMN¹H conforme.
MS : [M-H] = 339

### 2. Synthèse de l'intermédiaire 17.2

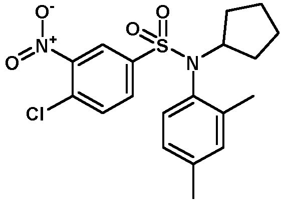

### 4-chloro-N-cyclopentyl-N-(2,4-diméthyl-phényl)-3-nitro-benzenesulfonamide

Un mélange de 4-chloro-N-(2,4-diméthyl-phényl)-3-nitro-benzenesulfonamide (180mg; 0.53 mmol), de carbonate de césium (258mg; 0.79 mmol), d'iodocyclopentane (90 µl; 0.79 mmol) dans de la 1-méthyl-2-pyrrolidone (5ml) est agité une nuit à une température de 80°C, hydrolysé, dilué et extrait à l'acétate. Les phases organiques sont rassemblées, lavées avec de l"eau, séchées (MgSO₄), filtrées et concentrées sous vide.

Le 4-chloro-N-cyclopentyl-N-(2,4-dimethyl-phényl)-3-nitro-benzenesulfonamide (216 mg; 100%) est obtenu sous la forme d'une huile brune avec une RMN¹H conforme.

### 3. Synthèse de l'intermédiaire 17.3

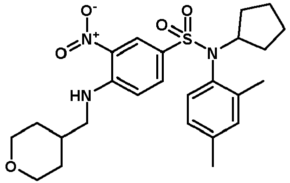

### N-cyclopentyl-N-(2,4-diméthyl-phényl)-3-nitro-4-[(tétrahydro-pyran-4-ylméthyl)-amino] -benzenesulfonamide

Avec un mode opératoire analogue à celui décrit pour l'intermédiaire 1.3, le N-cyclopentyl-N-(2,4-diméthyl-phenyl)-3-nitro-4-[(tétrahydro-pyran-4-ylméthyl)-amino]-benzenesulfonamide (105 mg; 35%) est obtenu sous la forme d'une huile jaune avec une RMN¹H conforme.
MS : [M+H] = 488

### 4. Synthèse de l'intermédiaire 17.4

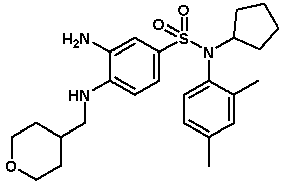

### 3-amino-N-cyclopentyl-N-(2,4-diméthyl-phényl)-4-[(tétrahydro-pyran-4-ylméthyl)-amino]-benzenesulfonamide

Par analogie au mode opératoire décrit pour l'intermédiaire 1.4, le 3-amino-N-cyclopentyl-N-(2,4-diméthyl-phényl)-4-[(tétrahydro-pyran-4-ylméthyl)-amino]-benzenesulfonamide (94 g; 100%) est obtenu sous la forme d'une huile verdâtre avec une RMN¹H conforme.
MS : [M+H] = 458

### 5. Synthèse du composé 18 selon l'invention

Avec un mode opératoire analogue à celui décrit dans l'exemple 1, l'acide 2-oxo-1-(tétrahydro-pyran-4-ylméthyl)-2,3-dihydro-1H-benzoimidazole-5-sulfonique cyclopentyl-(2,4-diméthyl-phenyl)-amide (10 mg; 8%) est obtenu sous la forme d'une huile brune.
¹H NMR (CDCl₃-d) δ: 1.24 (d, J = 11.7 Hz, 4H), 1.43 (dd, J = 13.9, 7.6 Hz, 8H), 1.95 - 2.05 (m, 1H), 2.31 (s, 6H), 3.32 - 3.41 (m, 2H), 3.79 (d, J = 7.2 Hz, 2H), 3.99 (dd, J = 11.6, 4.1 Hz, 2H), 4.48 (ddd, J = 10.1, 7.1, 3.0 Hz, 1H), 6.57 (d, J = 8.0 Hz, 1H), 6.86 (d, J = 8.2 Hz, 1H), 7.03 (d, J = 8.4 Hz, 1H), 7.11 (d, J = 2.1 Hz, 1H), 7.44 (d, J = 1.7 Hz, 1H), 7.54 (dd, J = 8.3, 1.7 Hz, 1H), 8.60 (s, 1H)
MS : [M+H] = 484

### Exemple 18: Synthèse de l'acide 2-oxo-1-(tetrahydro-pyran-4-ylmethyl)-2,3-dihydro-1H-benzoimidazole-5-sulfonique (2,4-dimethyl-phenyl)-(1-ethyl-propyl)-amide

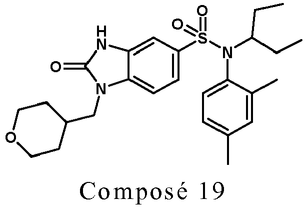

### 1. Synthèse de l'intermédiaire 18.2

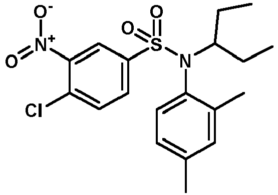

### 4-chloro-N-(2,4-diméthyl-phényl)-N-(1-éthyl-propyl)-3-nitro-benzenesulfonamide

Un mélange de 4-chloro-N-(2,4-diméthyl-phényl)-3-nitro-benzenesulfonamide (480mg; 1.41 mmol), de carbonate de césium (1 38 mg; 4.23 mmol) et de 3-bromopentane (520 µl; 4.23 mmol) dans de la 1-methyl-2-pyrrolidone (5ml) est agité une nuit à une température de 80°C, hydrolysé et extrait à l'acétate d'éthyle. Les phases organiques sont rassemblées, lavées à l"eau, séchées (MgSO₄), filtrées et concentrées. Le 4-chloro-N-(2,4-diméthyl-phényl)-N-(1-ethyl-propyl)-3-nitro-benzenesulfonamide (580mg; 100 %) est obtenu sous la forme d'une huile brune avec une RMN¹H conforme.
MS : [M+H] = 411

### 2. Synthèse de l'intermédiaire 18.3

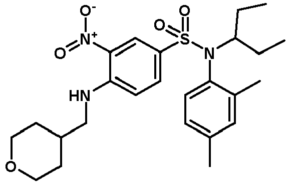

### N-(2,4-diméthyl-phényl)-N-(1-éthyl-propyl)-3-nitro-4-[(tétrahydro-pyran-4-ylméthyl)-amino]-benzenesulfonamide

Avec un mode opératoire analogue à celui décrit pour l'intermédiaire 1.3, le N-(2,4-diméthyl-phényl)-N-(l-ethyl-propyl)-3-nitro-4-[(tetrahydro-pyran-4-ylmethyl)-amino]-benzenesulfonamide (450 mg; 41%) est obtenu sous la forme d'une huile brune avec une RMN¹H conforme.
MS : [M+H] = 490

### 3. Synthèse de l'intermédiaire 18.4

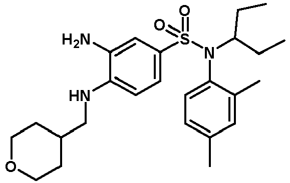

### 3-amino-N-(2,4-diméthyl-phényl)-N-(1-éthyl-propyl)-4-[(tétrahydro-pyran-4-ylméthyl)-amino] -benzenesulfonamide

Par analogie au mode opératoire décrit pour l'intermédiaire 1.4, le 3-amino-N-(2,4-diméthyl-phényl)-N-(1-éthyl-propyl)-4-[(tétrahydro-pyran-4-ylméthyl)-amino]-benzenesulfonamide (420 mg; 100%) est obtenu sous la forme d'une huile ambrée avec une RMN¹H conforme.
MS : [M+H] = 460

### 4. Synthèse du composé 19 selon l'invention

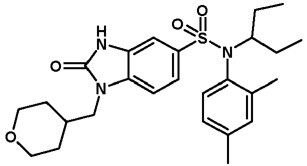

Avec un mode opératoire analogue à celui décrit dans l'exemple 1, l'acide 2-oxo-1-(tétrahydro-pyran-4-ylméthyl)-2,3-dihydro-1H-benzoimidazole-5-sulfonique (2,4-diméthyl-phényl)-(1-éthyl-propyl)-amide (92 mg; 19%) est obtenu sous la forme d'un solide cristallin blanc cassé après recristallisation dans un mélange acétone/eau
¹H NMR (DMSO-d6) δ: 0.67 (t, J = 7.3 Hz, 3H), 0.95 (t, J = 7.3 Hz, 3H), 1.08 - 1.21 (m, 2H), 1.30 (ttd, J = 14.6, 7.1, 3.9 Hz, 4H), 1.46 (d, J = 13.3 Hz, 3H), 2.03 (ddd, J = 11.4, 7.3, 3.8 Hz, 1H), 2.09 (s, 0H), 2.24 (s, 3H), 2.28 (s, 3H), 3.23 (td, J = 11.6, 2.2 Hz, 2H), 3.75 (d, J = 7.3 Hz, 2H), 3.79 - 3.87 (m, 2H), 6.65 (d, J = 8.1 Hz, 1H), 6.94 (dd, J = 8.1, 2.2 Hz, 1H), 7.12 - 7.19 (m, 2H), 7.38 (d, J = 1.6 Hz, 2H), 11.21 (s, 1H).
MS : [M+H] = 486

### Exemple 19 : Synthèse de l'acide 1-(1,1-dioxo-hexahydro-1λ⁶-thiopyran-4-ylmethyl)-2-oxo-2,3-dihydro-1H-benzoimidazole-5-sulfonique (4-ethyl-phenyl)-isobutyl-amide

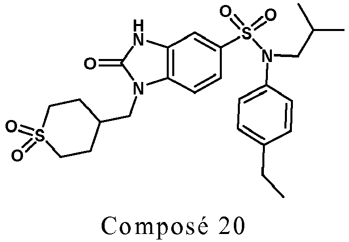

### 1. Synthèse de l'intermédiaire 19.1

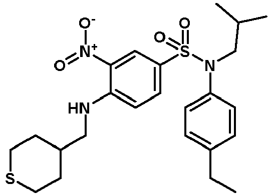

### N-(4-éthyl-phényl)-N-isobutyl-3-nitro-4-[(tétrahydro-thiopyran-4-ylméthyl)-amino] -benzenesulfonamide

Avec un mode opératoire analogue à celui décrit pour l'intermédiaire 1.3, le N-(4-éthyl-phényl)-N-isobutyl-3-nitro-4-[(tétrahydro-thiopyran-4-ylméthyl)-amino]-benzenesulfonamide (600 mg; 97%) est obtenu sous la forme d'une huile brune avec une RMN¹H conforme.
MS : [M+H] = 492

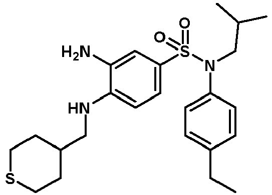

### 3-amino-N-(4-éthyl-phényl)-N-isobutyl-4-[(tétrahydro-thiopyran-4-ylmethyl)-amino] -benzenesulfonamide

Par analogie au mode opératoire décrit pour l'intermédiaire 1.4, le 3-amino-N-(4-éthyl-phényl)-N-isobutyl-4-[(tétrahydro-thiopyran-4-ylméthyl)-amino]-benzenesulfonamide (543 mg; 96%) est obtenu sous la forme d'une huile ambrée avec une RMN¹H conforme.
MS : [M+H] = 462

### 2. Synthèse du composé 20 selon l'invention

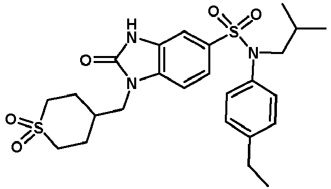

Avec un mode opératoire analogue à celui décrit dans l'exemple 1, l'acide 1-(1,1-dioxo-hexahydro-λ⁶-thiopyran-4-ylméthyl)-2-oxo-2,3-dihydro-1H-benzoimidazole-5-sulfonique (4-éthyl-phényl)-isobutyl-amide (140 mg; 40%) est obtenu sous la forme d'un solide cristallin blanc cassé après recristallisation dans un mélange acétone/eau
¹H NMR (DMSO-d6) δ: 0.84 (d, J = 6.6 Hz, 6H), 1.14 - 1.23 (m, 3H), 1.40 (dt, J = 13.5, 6.8 Hz, 1H), 1.73 (q, J = 12.4 Hz, 2H), 1.95 (d, J = 13.8 Hz, 2H), 2.07-2.18 m, 1H), 2.61 (q, J = 7.6 Hz, 2H), 3.00 - 3.17 (m, 4H), 3.27 (d, J = 7.3 Hz, 2H), 3.80 (d, J = 7.2 Hz, 2H), 6.97 (d, J = 8.3 Hz, 2H), 7.02 (d, J = 1.8 Hz, 1H), 7.14 - 7.22 (m, 2H), 7.24 (dd, J = 8.4, 1.8 Hz, 1H), 7.39 (d, J = 8.3 Hz, 1H), 11.17 (s, 1H).
MS : [M+H] = 520

### Partie II : Synthèse des sulfonamides bicycliques à partir du schéma réactionnel 2

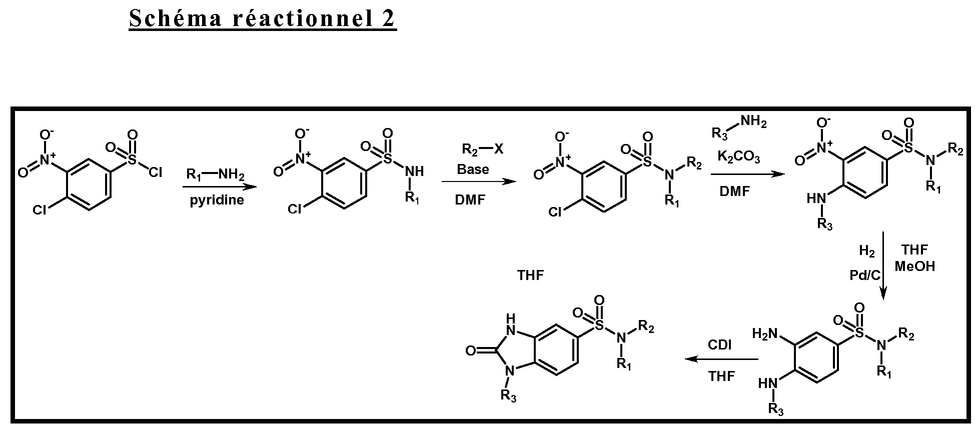

### Exemple 21: Synthèse de l'acide 1-oxetan-3-ylmethyl-2-oxo-2,3-dihydro-1H-benzoimidazole-5-sulfonique (4,6-dimethyl-pyridin-3-yl)-isobutyl-amide

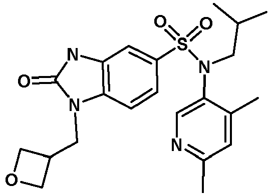

### 1. Synthèse de l'intermédiaire 21.1

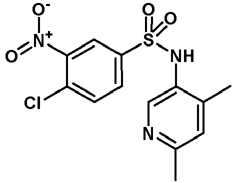

### 4-chloro-N-(4,6-dimethyl-pyridin-3-yl)-3-nitro-benzenesulfonamide

Le chlorure de 4-chloro-3-nitrobenzenesulfonyle (500mg; 1.91 mmol) est additionné sur la 4,6-dimethyl-pyridin-3-ylamine (487mg; 3.83 mmol), la triéthylamine (53µl; 0.38 mmol) et la pyridine (5.2ml). Le milieu réactionnel est agité pendant 10 minutes à température ambiante, hydrolysé avec une solution saturée de NH₄Cl et extrait à l'acétate d'éthyle. La phase organique est concentrée puis coévaporée avec du toluène.

Le produit brut est purifié par HPLC préparative (colonne C18, éluant : acétonitrile dans eau/0.1% d'acide formique).

Le 4-chloro-N-(4,6-diméthyl-pyridin-3-yl)-3-nitro-benzenesulfonamide (303mg; 46%) est obtenu sous la forme d'un solide crème avec une RMN¹H conforme.
MS : [M-H] = 340

### 2. Synthèse de l'intermédiaire 21.2

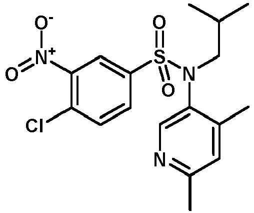

### 4-chloro-N-(4,6-diméthyl-pyridin-3-yl)-N-isobutyl-3-nitro-benzenesulfonamide

L'hydrure de sodium 60% (32 mg; 0.80 mmo) est ajouté sur le 4-chloro-N-(4,6-dimethyl-pyridin-3-yl)-3-nitro-benzenesulfonamide (248mg; 0.73 mmol) et le 1-iodo-2-methyl-propane (125µl; 1.09 mmol) en solution dans le N,N-diméthylformamide (6.6ml). Le milieu réactionnel est agité pendant 8 heures à une température de 80°C, hydrolysé et extrait à l'acétate d'éthyle. Les phases organiques sont rassemblées, lavées à la saumure et séchées (MgSO₄). Les solvants sont évaporés.

Le produit brut est purifié par chromatographie sur gel de silice (Heptane/Acétate d'éthyle de 20 à 60%). Le 4-chloro-N-(4,6-diméthyl-pyridin-3-yl)-N-isobutyl-3-nitro-benzenesulfonamide (140mg; 48%) est obtenu sous la forme d'un solide blanc avec une RMN¹H conforme.
MS : [M+H] = 398

### 3. Synthèse de l'intermédiaire 21.3

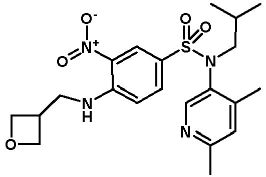

### N-(4,6-diméthyl-pyridin-3-yl)-N-isobutyl-3-nitro-4-[(oxetan-3-ylmethyl)-amino]-benzenesulfonamide

A une solution de 4-chloro-N-(4,6-dimethyl-pyridin-3-yl)-N-isobutyl-3-nitro-benzenesulfonamide (139mg; 0.35 mmol) dans du N,N-diméthylformamide (1.4ml) sont ajoutés du carbonate de potassium (72mg; 0.52 mmol) puis de l'oxetan-3-yl-methylamine (31.96 mg; 0.37 mmol). Le milieu réactionnel est agité pendant 16 heures à température ambiante, hydrolysé et extrait à l'acétate d'éthyle. La phases organique est lavée à la saumure, séchée sur sulfate de magnésium anhydre, filtrée et concentrée. Le produit brut est purifiée par chromatographie sur gel de silice (Heptane/Acétate d'éthyle de 20 à 60%puis à 50% d'acétate d'éthyle). Le N-(4,6-dimethyl-pyridin-3-yl)-N-isobutyl-3-nitro-4-[(oxetan-3-ylmethyl)-amino]-benzenesulfonamide (123mg; 78%) est obtenu sous la forme d'un solide jaune avec une RMN¹H conforme.
MS : [M+H] = 449

### 4. Synthèse de l'intermédiaire 21.4

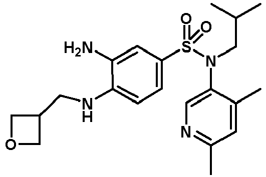

### 3-amino-N-(4,6-diméthyl-pyridin-3-yl)-N-isobutyl-4-[(oxetan-3-ylmethyl)-amino]-benzenesulfonamide

Le N-(4,6-diméthyl-pyridin-3-yl)-N-isobutyl-3-nitro-4-[(oxetan-3-ylméthyl)-amino]-benzenesulfonamide (120mg; 0.27 mmol) est mis en solution dans du tétrahydrofurane (1.2 ml). Le milieu est dégazé puis Palladium 10% sur charbon activé, stabilisé avec 50% d'eau (57mg; 0.03 mmol) est ajouté. Le milieu réactionnel est mis sous atmosphère de dihydrogène et agité pendant 4 heures à température ambiante. Le milieu réactionnel est filtré sur célite. Le filtrat est concentré sous vide.

Le 3-amino-N-(4,6-dimethyl-pyridin-3-yl)-N-isobutyl-4-[(oxetan-3-ylmethyl)-amino]-benzenesulfonamide (112mg; 100%) est obtenu sous la forme d'un film gris avec une RMN¹H conforme.
MS : [M+H] = 419

### 5. Synthèse de l'acide 1-oxétan-3-ylméthyl-2-oxo-2,3-dihydro-1H-benzoimidazole-5-sulfonique (4,6-diméthyl-pyridin-3-yl)-isobutyl-amide

A une solution de 3-amino-N-(4,6-diméthyl-pyridin-3-yl)-N-isobutyl-4-[(oxetan-3-ylméthyl)-amino]-benzenesulfonamide (110 mg; 0.26 mmol) dans du tétrahydrofurane (1.1 ml) est ajouté du N,N'-carbonyldiimidazole (128 mg; 0.79 mmol). Le milieu réactionnel est agité pendant 17 heures à une température de 60°C, hydrolysé à l'eau et extrait à l'acétate d'éthyle.

La phase organique est décantée, lavée à l'eau, séchée (MgSO₄), filtrée et concentrée.

Le produit brut est purifié par HPLC préparative (colonne C18, éluant : acétonitrile dans eau/0.1% d'acide formique). L'acide 1-oxetan-3-ylmethyl-2-oxo-2,3-dihydro-1H-benzoimidazole-5-sulfonique (4,6-dimethyl-pyridin-3-yl)-isobutyl-amide (73 mg; 61%) est obtenu sous la forme d'un solide blanc.
¹H NMR (DMSO-d6) δ: 0.77 (d, J = 6.7 Hz, 3H), 0.93 (d, J = 6.5 Hz, 3H), 1.40 (t, J = 7.1 Hz, 1H), 2.22 (s, 3H), 2.41 (s, 3H), 3.18 (dd, J = 13.1, 5.0 Hz, 1H), 3.31 - 3.46 (m, 1H), 4.16 (d, J = 7.1 Hz, 2H), 4.41 (t, J = 6.1 Hz, 2H), 4.63 (dd, J = 7.8, 6.1 Hz, 2H), 7.09 (d, J = 1.7 Hz, 1H), 7.20 (s, 1H), 7.30 (dd, J = 8.3,1.7 Hz, 1H), 7.41 (d, J = 8.3 Hz, 1H), 7.69 (s, 1H), 11.27 (s, 1H)
MS : [M-H] = 445

### Exemple 22 : Synthèse de l'acide2-oxo-1-pyridazin-4-ylmethyl-2,3-dihvdro-1H-benzoimidazole-5-sulfonique isopropyl-(4-methoxy-2-methyl-phenyl)-amide

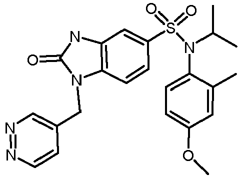

### 1. Synthèse de l'intermédiaire 22.1

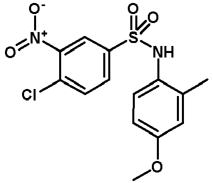

### 4-chloro-N-(4-methoxy-2-méthyl-phényl)-3-nitro-benzenesulfonamide

Le chlorure de 4-chloro-3-nitrobenzenesulfonyl (300 mg; 1.15 mmol) est ajouté sur un mélange de 4-méthoxy-2-méthylaniline (157.5 mg; 1.15 mmol) et de pyridine (1.50 ml; 18.6 mmol). Le milieu réactionnel est agité pendant 3 heures à une température de 45°C, hydrolysé à l'eau et dilué à l'acétate d'éthyle. La phase organique est extraite, lavée à l'eau, séchée (MgSO₄), filtrée et concentrée. Le produit brut est purifié par HPLC préparative (colonne C18, éluant : acétonitrile dans eau/0.1% d'acide formique).

Le 4-chloro-N-(4-méthoxy-2-méthyl-phényl)-3-nitro-benzenesulfonamide (264mg; 64%) est obtenu sous la forme d'un solide pourpre avec une RMN¹H conforme.
MS : [M-H] = 355

### 2. Synthèse de l'intermédiaire 22.2

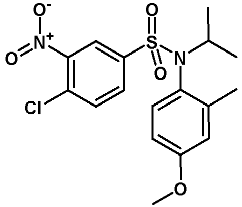

### 4-chloro-N-isopropyl-N-(4-méthoxy-2-méthyl-phényl)-3-nitro-benzenesulfonamide

A une température de 0°C, l'hydrure de sodium 60% (26mg; 0.65 mmol) est ajouté sur le 4-chloro-N-(4-méthoxy-2-méthyl-phényl)-3-nitro-benzenesulfonamide (210; 0.59 mmol) et le 2-iodopropane (90 µl; 0.88 mmol) en solution dans le N,N-diméthylformamide (5.5ml). Le milieu réactionnel est agité pendant 18 heures à une température de 30°C, hydrolysé et extrait à l'acétate d'éthyle. Les phases organiques sont rassemblées, lavées avec une solution saturée de chlorure de sodium et séchées (MgSO₄). Les solvants sont évaporés.

Le produit brut est purifié par chromatographie sur gel de silice (Heptane/Acétate d'éthyle de 5 à 20% d'acétate d'éthyle). Le 4-chloro-N-isopropyl-N-(4-méthoxy-2-méthyl-phényl)-3-nitro-benzenesulfonamide (183mg; 78%) est obtenu sous la forme d'une pâte incolore qui cristallise en un solide blanc avec une RMN¹H conforme.
MS : [M+H] = 399

### 3. Synthèse de l'intermédiaire 22.3

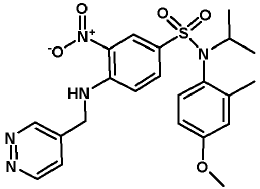

### N-Isopropyl-N-(4-méthoxy-2-méthyl-phényl)-3-nitro-4-[(pyridazin-4-ylméthyl)-amino] -benzenesulfonamide

A une solution de 4-chloro-N-isopropyl-N-(4-methoxy-2-methyl-phenyl)-3-nitro-benzenesulfonamide (167mg; 0.42 mmol) dans du N,N-diméthylformamide (1.7 ml) sont ajoutés de la N,N-diisopropyl-ethylamine (300µl; 1.75 mmol) puis du dihydrochlorure de pyridazin-4-ylmethanammonium (80 mg; 0.44 mmol).

Le milieu réactionnel est agité pendant 30 heures à température ambiante, hydrolysé avec de l'eau puis de l'acétate d'éthyle et de la saumure sont rajoutés. La phase organique est extraite, lavée à l'eau et à la saumure, séchée (MgSO₄), filtrée et concentrée.

Le produit brut est chromatographié sur gel de silice, éluant heptane acétate d'éthyle de 30 à 100% d'acétate d'éthyle. Le N-isopropyl-N-(4-methoxy-2-methyl-phenyl)-3-nitro-4-[(pyridazin-4-ylmethyl)-amino]-benzenesulfonamide (100mg, 51%) est obtenu sous la forme d'un film jaune avec une RMN¹H conforme.
MS : [M+H] = 472

### 4. Synthèse de l'intermédiaire 22.4

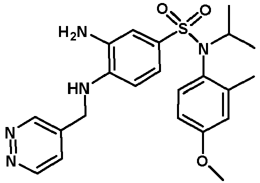

### 3-amino-N-(4,6-dimethyl-pyridin-3-yl)-N-isobutyl-4-[(oxetan-3-ylmethyl)-amino]-benzenesulfonamide

Avec un mode opératoire analogue à celui décrit pour l'intermédiaire 21.4, le 3-amino-N-isopropyl-N-(4-méthoxy-2-méthyl-phényl)-4-[(pyridazin-4-ylméthyl)-amino]-benzenesulfonamide (94mg; 100%) est obtenu sous la forme d'une huile foncée avec une RMN¹H conforme.
MS : [M+H] = 442

### 5. Synthèse de l'acide 2-oxo-1-pyridazin-4-ylméthyl-2,3-dihydro-1H-benzoimidazole-5-sulfonique isopropyl-(4-méthoxy-2-méthyl-phenyl)-amide

Avec un mode opératoire analogue à celui décrit dans l'exemple 21, l'acide 2-oxo-1-pyridazin-4-ylméthyl-2,3-dihydro-1H-benzoimidazole-5-sulfonique isopropyl-(4-méthoxy-2-méthyl-phényl)-amide (23 mg; 23%) est obtenu sous la forme d'un solide blanc.
¹H NMR (DMSO-d6) δ: 0.85 (d, J = 6.7 Hz, 3H), 0.97 (d, J = 6.6 Hz, 3H), 2.24 (s, 3H), 3.76 (s, 3H), 4.32 - 4.54 (m, 1H), 5.21 (s, 2H), 6.47 - 6.80 (m, 2H), 6.91 (d, J = 2.9 Hz, 1H), 7.16 - 7.57 (m, 4H), 9.04 - 9.35 (m, 2H), 11.44 (s, 1H)
MS : [M-H] = 468

## Revendications

1. Composé de formule (I), ses sels d'addition pharmaceutiquement acceptables, ses hydrates et/ou ses solvates : 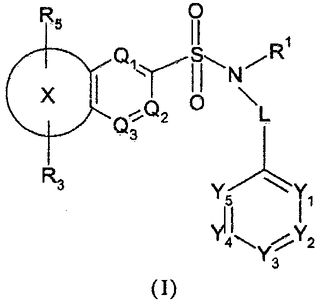 Formule (I) dans laquelle :
• L représente une liaison simple ou un groupe méthylène CH₂,
• X représente le radical cyclique suivant : 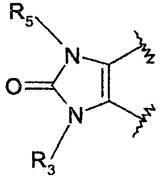
• un ou deux des éléments Y¹, Y², Y³, Y⁴ et Y⁵ représente(nt) un atome d'azote et les autres éléments correspondent à un groupement -CR², ou chacun des éléments Y¹, Y², Y³, Y⁴ et Y⁵ correspond à un groupement -CR²,
• un ou deux des éléments Q¹, Q² et Q³ représente(nt) un atome d'azote et le ou les autres éléments corresponde(nt) à un groupement -CR^{2a}, ou chacun des éléments Q¹, Q² et Q³ correspond à un groupement -CR^{2a},
• R¹ représente un radical alkyle, linéaire ou ramifié, en C₃-C₅, un radical cycloalkyle en C₃-C₅, un radical alkényle, linéaire ou ramifié, en C₂-C₅, un radical -CH₂-cycloalkyle en C₃-C₅, un radical hétérocycloalkyle en C₄-C₅, un radical -CH₂-hétérocycloalkyle en C₄-C₆,
• R² représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement cyano -CN, un radical -C(=O)R'² avec R'² désignant un radical alcoxy en C₁-C₃, un radical -CF₃ ; lesdits radicaux alkyle, alkényle et alcoxy pouvant être substitués par un ou plusieurs atomes d'halogène ;
• R^{2a} représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement -CN, un groupement hydroxy -OH, un groupement - CH(R^{3a})OH, un groupement carboxylique -COOH, un groupement carbamoyle -CONR^{2c}R^{2d}, un groupement amido -NR^{2c}COR^{2d}, un groupement -SO₂R^{2c}, un groupement -SOR^{2c}, un groupement-S(=O)(=NH-R^{2c}), lesdits radicaux alkyle, alkényle et alcoxy pouvant être substitués par un ou plusieurs atomes d'halogène,
• R^{2c} et R^{2d}, identiques ou différents, représentent un atome d'hydrogène ou un radical alkyle, linéaire ou ramifié, en C₁-C₅ ;
• R^{3a} représente un atome d'hydrogène ou un radical alkyle, linéaire ou ramifié, en C₁-C₅ ;
• R³ représente un atome d'hydrogène ou un groupement (CHR⁶)ₙ-(Z)ₒ-(CHR'⁶)ₚ-R⁷,
• n, o et p, identiques ou différents, représentent zéro ou un entier naturel allant de 1 à 3,
• Z représente un groupement divalent choisi parmi un groupement méthylène -CH₂-, un groupement amino -NH- et un atome d'oxygène - O-,
• R⁶ et R'⁶, identiques ou différents, représentent un atome d'hydrogène, un groupement méthyle -CH₃, un groupement -OH, un groupement hydroxyméthyle, une fonction carboxylique -COOH,
• R⁷ représente :
- un atome d'hydrogène ou un atome d'halogène,
- un groupement COOR'⁷ avec R'⁷ désignant alkyl(C₁)hétérocycle(C₆),
- un radical hétérocycloalkyle non cationique éventuellement substitué par un ou plusieurs atomes d'halogène, un ou plusieurs groupements alkyle en C₁-C₃, linéaire ou ramifié, un ou plusieurs groupements -OH, une ou plusieurs fonctions carbonyles, un ou plusieurs groupements hydroxyalkyle en C₁-C₄, linéaire ou ramifié, un ou plusieurs groupements amino, un ou plusieurs groupements -C(=O)R^{7a}, un ou plusieurs groupements S(=O)₂R^{7a} ; R^{7a} représentant un radical alkyle, linéaire ou ramifié, en C₁-C₃, un radical alcoxy en C₁-C₃, linéaire ou ramifié, ou un radical amino N(R^{8a})(R^{8b}),
- un radical cycloalkyle non cationique en C₃-C₆ éventuellement substitué par un ou plusieurs radicaux méthyle, un ou plusieurs atomes d'halogène, un groupement cyano -CN ou un ou plusieurs groupements -COR¹³ ; R¹³ désignant un radical alcoxy en C₁-C₃, linéaire ou ramifié, ou un groupement hydroxy,
- un radical aromatique ou hétéroaromatique, non cationique, éventuellement substitué par un ou plusieurs atomes d'halogène, un ou plusieurs groupements alkyle en C₁-C₃, linéaire ou ramifié, éventuellement substitué par un ou plusieurs atomes d'halogène, un ou plusieurs groupements alcoxy en C₁-C₃, un ou plusieurs groupements amino -NR¹¹R¹², un ou plusieurs groupements -COR¹¹, un ou plusieurs groupements -COOR¹¹, un ou plusieurs groupements amido-CONR¹¹R¹², un ou plusieurs groupements -SOR¹¹, un ou plusieurs groupements -SO₂R¹¹, un ou plusieurs groupements-NHCOR¹¹, un ou plusieurs groupements -NHCOOR¹¹, un ou plusieurs groupements -SO₂NR¹¹R¹² ou un ou plusieurs groupements -CN; R¹¹ et R¹², identiques ou différents, représentant un atome d'hydrogène, un radical hydroxy -OH, un radical alkyle, linéaire ou ramifié, en C₁-C₃, éventuellement substitué par un ou plusieurs atomes d'halogène,
• R⁵ représente un atome d'hydrogène ou un atome d'halogène, un radical alkyle en C₁-C₃, linéaire ou ramifié éventuellement substitué par un ou plusieurs atomes d'halogène; un radical amino - NH₂, un radical CH₂R^{'7a} avec R'^{7a} désignant un radical méthoxy, un groupement hydroxy -OH, un groupement -CH₂COOH, un groupement -CH(R^{5b})OH, un groupement carboxylique -COOH, un groupement - CN, une fonction thioxo,
• R^{5b} représente un atome d'hydrogène ; un radical alkyle, linéaire ou ramifié, en C₁-C₃ éventuellement substitué par une ou plusieurs fonctions carboxyliques ; un radical cyclopropyle,
• R^{8a} et R^{8b}, identiques ou différents, désignent un atome d'hydrogène, un radical alkyle, linéaire ou ramifié, en C₁-C₃ ou un radical cyclopropyle.

2. Composé de formule (I) selon la revendication 1, **caractérisé en ce que** R⁷ représente un radical hétérocyclique choisi parmi les hétérocycles suivants : 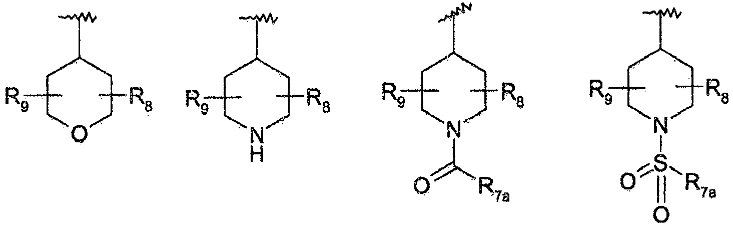 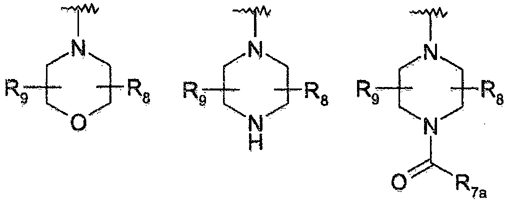 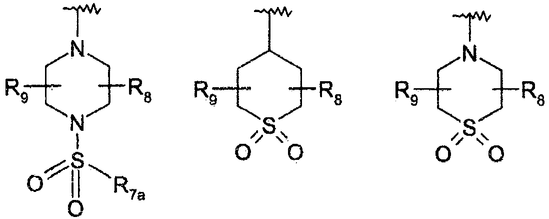 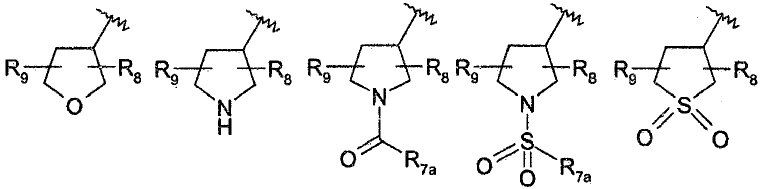 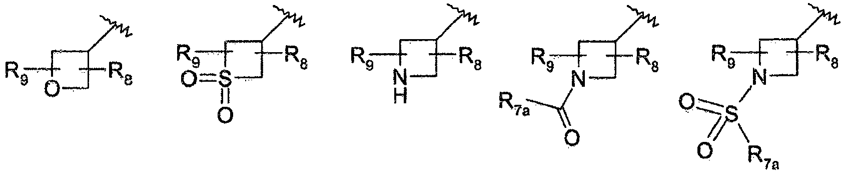 dans lesquels :
- R₇ₐ représente un radical alkyle, linéaire ou ramifié, en C₁-C₃, un radical alcoxy, linéaire ou ramifié, en C₁-C₃ ou un radical amino en N(R^{8a})(R^{8b}),
- R^{8a} et R^{8b}, identiques ou différents, désignent un atome d'hydrogène, un radical alkyle, linéaire ou ramifié, en C₁-C₃ ou un radical cyclopropyle,
- R₈ et R₉, identiques ou différents, représentent un atome d'hydrogène, un radical alkyle, linéaire ou ramifié, en C₁-C₃, un groupement hydroxy -OH, une fonction carbonyle =O, un radical hydroxyalkyle en C₁ (-CH₂OH), un groupe amino NH₂,
- R₈ et R₉ peuvent former ensemble avec les atomes de carbone auxquels ils sont attachés un cycle carbocyclique comportant de 5 à 7 chaînons,

3. Composé de formule (I) selon la revendication 1, **caractérisé en ce que** R⁷ représente un radical aromatique ou hétéroaromatique choisi parmi : 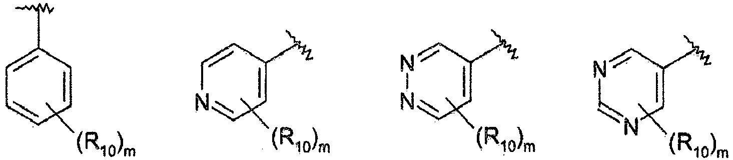 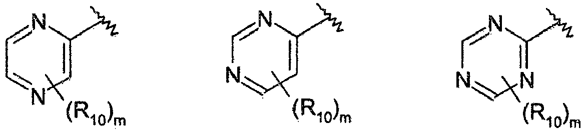 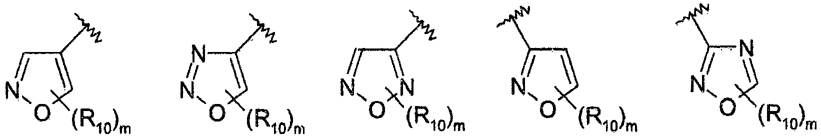 dans lesquels :
- R₁₀ représente un atome d'hydrogène ou un atome d'halogène, un groupement alkyle en C₁-C₃, linéaire ou ramifié, éventuellement substitué par un ou plusieurs atomes d'halogène ; un groupement alcoxy en C₁₋C₃, un groupement amino -NR¹¹R¹², un groupement -COR¹¹, un groupement -COOR¹¹, un groupement amido-CONR¹¹R¹², un groupement -SOR¹¹, un groupement -SO₂R¹¹, un groupement -NHCOR¹¹, un groupement -NHCOOR¹¹, un groupement-SO₂NR¹¹R¹² ou un groupement -CN ; R¹¹ et R¹², identiques ou différents, représentant un atome d'hydrogène ou un radical alkyle, linéaire ou ramifié, en C₁-C₃ éventuellement substitué par un ou plusieurs atomes d'halogène,
m désigne zéro ou un entier naturel allant de 1 à 3,

4. Composé de formule (I) selon la revendication 1, **caractérisé en ce qu'**il est choisi parmi le ou les composés de formule (IV) ainsi que leurs sels d'addition pharmaceutiquement acceptables, leurs hydrates et/ou leurs solvates : 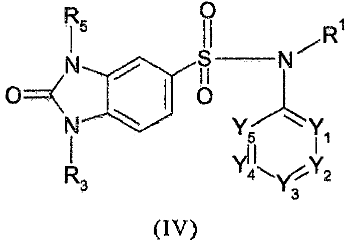 Formule (IV) dans laquelle R¹, R³, R⁵ et Y¹ à Y⁵ ont les mêmes significations que dans la formule (I) telle que définie selon la revendication 1.

5. Composé de formule (I) selon l'une quelconque des revendications précédentes, **caractérisé en ce que** R¹ représente un radical alkyle, linéaire ou ramifié, en C₃-C₅ ou un radical cycloalkyle en C₃-C₅,

6. Composé de formule (I) selon l'une quelconque des revendications précédentes, **caractérisé en ce que** R⁵ représente un atome d'hydrogène.

7. Composé de formule (I) selon l'une quelconque des revendications précédentes, **caractérisé en ce que** R³ représente un groupement (CHR⁶)ₙ-(Z)ₒ-(CHR'⁶)ₚ-R⁷ avec les indices n, p, o, R⁶, R'⁶ et R⁷ ayant la signification indiquée selon l'une quelconque des revendications 1 à 3.

8. Composé de formule (I) selon l'une quelconque des revendications précédentes, **caractérisé en ce qu'**il est choisi parmi les composés suivants :
| | |
|---|---|
| 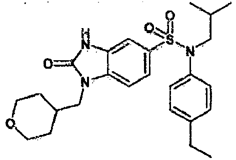 | Acide 2-oxo-1-(tétrahydro-pyan-4-ylméthyl)-2,3-dihydro-1H-benzoimidazole-5-sulfonique (4-éthyl-phényl)-isobutyl-amide |
| | Composé 2 |
| 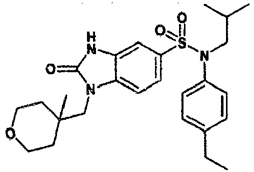 | Acide 1-(4-méthyl-tétrahydro-pyran-4-ylméthyl)-2-oxo-2,3-dihydro-1H-benzoimidazole-5-sulfonique (4-éthyl-phényl)- isobutyl-amide |
| | Composé 4 |
| 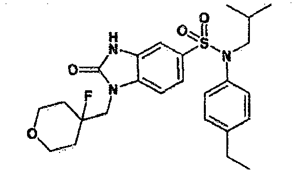 | Acide 1-(4-fluoro-tétrahydro-pyran-4- ylméthyl)- 2-oxo- 2,3 -dihydro-1H-benzoimidazole-5-sulfonique (4-éthyl-phényl)-isobutyl-amide |
| | Composé 5 |
| 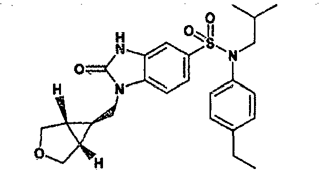 | Acide 1-[(1R,5S,6S)-1-(3-oxa-bicyclo[3.1.0]hex-6-yl)methyl]-2-oxo-2,3-dihydro-1H-benzoimidazole-5-sulfonique (4-éthyl-phényl)-isobutyl-amide |
| | Composé 6 |
| 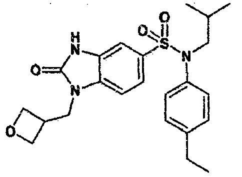 | Acide 1-oxetan-3-ylméthyl-2-oxo-2,3-dihydro-1H-benzoimidazole-5-sulfonique (4-éthyl-phényl)-isobutyl-amide |
| | Composé 7 |
| 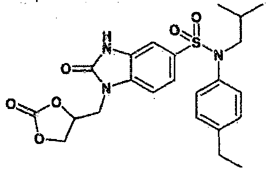 | Acide 2-oxo-1-(2-oxo-[1,3]-dioxolan-4-ylmethyl)-2,3-dihydro-1H-benzoimidazole-5-sulfonique (4-éthyl-phényl)-isobutyl-amide |
| | Composé 8 |
| 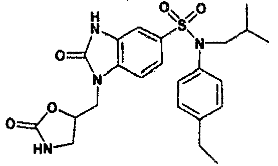 | Acide 2-oxo-1-(2-oxo-oxazolidin-5-ylmethyl)-2,3-dihydro-1H-benzoimidazole-5-sulfonique (4-éthyl-phényl)-isobutyl-amide |
| | Composé 9 |
| 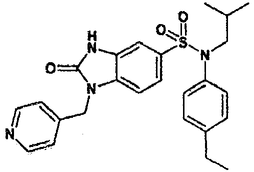 | Acide 2-oxo-1-pyridin-4-ylméthyl-2,3-dihydro-1H-benzoimidazole-5-sulfonique (4-éthyl-phényl)-isobutyl-amide |
| | Composé 10 |
| 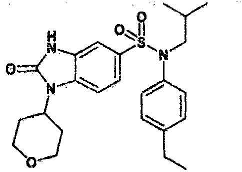 | Acide 2-oxo-1-(tétrahydro-pyran-4-yl)-2,3-dihydro-1H-benzoimidazole-5-sulfonique (4-éthyl-phényl)-isobutyl-amide |
| | Composé 11 |
| 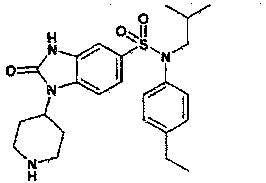 | Acide 2-oxo-1-piperidin-4-yl-2,3-dihydro-1H-benzoimidazole-5-sulfonique (4-éthyl-phényl)-isobutyl-amide |
| | Composé 13 |
| 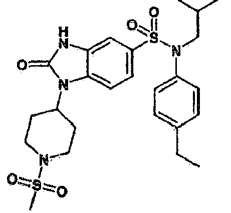 | Acide 1-(1-méthanesulfonyl-pipéridin-4-yl)-2-oxo-2,3-dihydro-1H-benzoimidazole-5-sulfonique (4-éthyl-phényl)-isobutyl-amide |
| | Composé 14 |
| 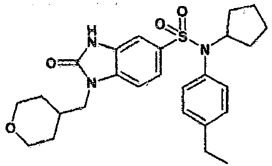 | Acide 2-oxo-1-(tétrahydro-pyran-4-ylmethyl)-2,3-dihydro-1H-benzoimidazole-5-sulfonique (4-éthyl-phényl)-cyclopentyl-amide |
| | Composé 15 |
| 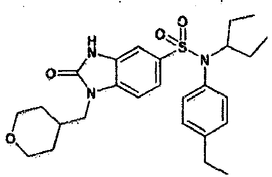 | Acide 2-oxo-1-(tétrahydro-pyran-4-ylméthyl)-2,3-dihydro-1H-benzoimidazole-5-sulfonique (4-éthyl-phényl)-(1-éthyl-propyl)-amide |
| | Composé 16 |
| 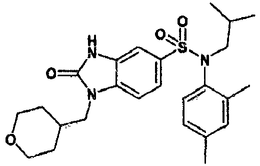 | Acide 2-oxo-1-(tetrahydro-pyran-4-ylmethyl)-2,3-dihydro-1H-benzoimidazole-5-sulfonique (2,4-diméthyl-phényl)-isobutyl-amide |
| | Composé 17 |
| 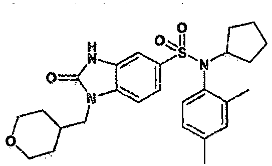 | Acide 2-oxo-1-(tétrahydro-pyran-4-ylméthyl)-2,3-dihydro-1H-benzoimidazole-5-sulfonique cyclopentyl-(2,4-diméthyl-phényl)-amide |
| | Composé 18 |
| 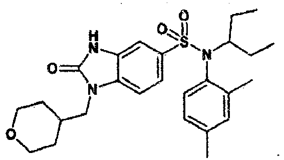 | Acide 2-oxo-1-(tétrahydro-pyran-4-ylméthyl)-2,3-dihydro-1H-benzoimidazole-5-sulfonique (2,4-diméthyl-phényl)-(1-éthyl-propyl)-amide |
| | Composé 19 |
| 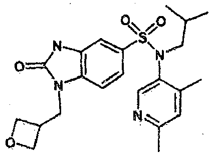 | Acide 1-oxetan-3-ylmethyl-2-oxo-2,3-dihydro-1H-benzoimidazole-5-sulfonique (4,6-dimethyl-pyridin-3-yl)- isobutyl-amide |
| | Composé 22 |
| 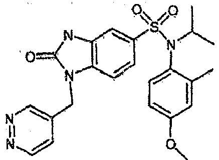 | Acide2-oxo-1-pyridazin-4-ylmethyl-2,3-dihydro-1H-benzoimidazole-5-sulfonique isopropyl-(4-methoxy-2-methyl-phényl)-amide |
| | Composé 23 |

9. Composé selon l'une quelconque des revendications précédentes pour son utilisation en tant que médicament.

10. Composé selon l'une quelconque des revendications 1 à 8 pour son utilisation dans le traitement de l'acné.

11. Composé selon l'une quelconque des revendications 1 à 8 pour son utilisation dans le traitement du psoriasis.

12. Composition pharmaceutique comprenant un ou plusieurs composés tels que définis selon l'une quelconque des revendications 1 à 8.

13. Composition pharmaceutique telle que définie selon la revendication 12 pour son utilisation dans le traitement de l'acné, la dermatite atopique et/ou le psoriasis.

## Patentansprüche

1. Verbindung der Formel (I), pharmazeutisch unbedenkliche Additionssalze davon, Hydrate davon und/oder Solvate davon: 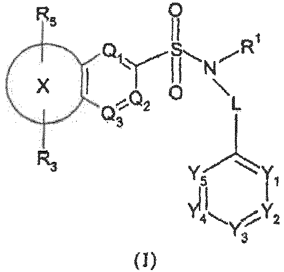 wobei in der Formel (I):
• L für eine Einfachbindung oder eine Methylengruppe CH₂ steht,
• X für den folgenden cyclischen Rest steht: 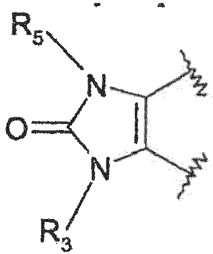
• ein oder zwei der Elemente Y¹, Y², Y³, Y⁴ und Y⁵ für ein Stickstoffatom steht bzw. stehen und die anderen Elemente einer Gruppe -CR² entsprechen oder jedes der Elemente Y¹, Y², Y³, Y⁴ und Y⁵ einer Gruppe -CR² entspricht,
• ein oder zwei der Elemente Q¹, Q² und Q³ für ein Stickstoffatom steht bzw. stehen und das bzw. die anderen Elemente einer Gruppe -CR^{2a} entspricht bzw. entsprechen oder jedes der Elemente Q¹, Q² und Q³ einer Gruppe -CR^{2a} entspricht,
• R¹ für einen linearen oder verzweigten C3-C₅-Alkylrest, einen C₃-C₅-Cycloalkylrest, einen linearen oder verzweigten C₂-C₅-Alkenylrest, einen -CH₂-C₃-C₅-Cycloalkylrest, einen C₄-C₅-Heterocycloalkylrest oder einen -CH₂-C₄-C₆-Heterocycloalkylrest steht,
• R² für ein Wasserstoffatom oder ein Halogenatom, einen linearen oder verzweigten C₁-C₅-Alkylrest, einen linearen oder verzweigten C₂-C₄-Alkenylrest, einen C₁-C₄-Alkoxyrest, eine Cyanogruppe-CN, einen Rest -C(=O)R'², wobei R'² einen C₁-C₃-Alkoxyrest bedeutet, oder einen -CF₃-Rest steht; wobei die Alkyl-, Alkenyl- und Alkoxyreste durch ein oder mehrere Halogenatome substituiert sein können;
• R^{2a} für ein Wasserstoffatom oder ein Halogenatom, einen linearen oder verzweigten C₁-C₅-Alkylrest, einen linearen oder verzweigten C₂-C₄-Alkenylrest, einen C₃-C₄-Alkoxyrest, eine -CN-Gruppe, eine Hydroxygruppe -OH, eine Gruppe -CH(R^{3a})OH, eine Carboxylgruppe -COOH, eine Carbamoylgruppe -CONR^{2c}R^{2d}, eine Amidogruppe -NR^{2c}COR^{2d}, eine Gruppe -SO₂R^{2c}, eine Gruppe -SOR^{2c} oder eine Gruppe -S(=O) (=NH-R^{2c}) steht, wobei die Alkyl-, Alkenyl- und Alkoxyreste durch ein oder mehrere Halogenatome substituiert sein können,
• R^{2c} und R^{2d} gleich oder verschieden sind und für ein Wasserstoffatom oder einen linearen oder verzweigten C₁-C₅-Alkylrest stehen;
• R^{3a} für ein Wasserstoffatom oder einen linearen oder verzweigten C₁-C₅-Alkylrest steht;
• R³ für ein Wasserstoffatom oder eine Gruppe (CHR⁶)ₙ-(Z)ₒ-(CHR'⁶)ₚ-R⁷ steht,
• n, o und p gleich oder verschieden sind und für null oder eine natürliche ganze Zahl im Bereich von 1 bis 3 stehen,
• Z für eine zweiwertige Gruppe steht, die aus einer Methylengruppe -CH₂-, einer Aminogruppe -NH- und einem Sauerstoffatom -O- ausgewählt ist,
• R⁶ und R'⁶ gleich oder verschieden sind und für ein Wasserstoffatom, eine Methylgruppe -CH₃, eine -OH-Gruppe, eine Hydroxymethylgruppe oder eine Carboxylfunktion -COOH stehen,
• R⁷ für:
- ein Wasserstoffatom oder ein Halogenatom,
- eine Gruppe COOR'⁷, wobei R'⁷ (C₁)Alkyl(C₆)-heterocyclus bedeutet,
- einen nichtkationischen Heterocycloalkylrest, der gegebenenfalls durch ein oder mehrere Halogenatome, eine oder mehrere lineare oder verzweigte C₁-C₃-Alkylgruppen, eine oder mehrere -OH-Gruppen, eine oder mehrere Carbonylfunktionen, eine oder mehrere lineare oder verzweigte C₁-C₄-Hydroxyalkylgruppen, ein oder mehrere Amino, eine oder mehrere Gruppen -C(=O)R^{7a} oder eine oder mehrere Gruppen S(=O)₂R^{7a} substituiert ist; wobei R^{7a} für einen linearen oder verzweigten C₃-C₃-Alkylrect, einen linearen oder verzweigten C₁-C₃-Alkoxyrest oder einen Aminorest N(R^{8a}) (R^{8b}) steht,
- einen nichtkationischen C₃-C₆-Cycloalkylrest, der gegebenenfalls durch einen oder mehrere Methylreste, ein oder mehrere Halogenatome, eine Cyanogruppe -CN oder eine oder mehrere Gruppen COR¹³ substituiert ist; wobei R¹³ für einen linearen oder verzweigten C₁-C₃-Alkoxyrest oder eine Hydroxygruppe steht,
- einen nichtkationischen aromatischen oder heteroaromatischen Rest, der gegebenenfalls durch ein oder mehrere Halogenatome, eine oder mehrere C₁-C₃-Alkylgruppen, die gegebenenfalls durch ein oder mehrere Halogenatome substituiert sind, eine oder mehrere C₁-C₃-Alkoxygruppen, eine oder mehrere Aminogruppen -NR¹¹R¹², eine oder mehrere Gruppen -COR¹¹, eine oder mehrere Gruppen -COOR¹¹, eine oder mehrere Amidogruppen -CONR¹¹R¹², eine oder mehrere Gruppen -SOR¹¹, eine oder mehrere Gruppen -SO₂R¹¹, eine oder mehrere Gruppen -NHCOR¹¹, eine oder mehrere Gruppen -NHCOOR¹¹, eine oder mehrere Gruppen -SO₂NR¹¹R¹² oder eine oder mehrere -CN-Gruppen substiuiert ist; wobei R¹¹ und R¹² gleich oder verschieden sind und für ein Wasserstoffatom, einen Hydroxyrest -OH oder einen linearen oder verzweigten C₁-C₃-Alkylrest, der gegebenenfalls durch ein oder mehrere Halogenatome substituiert ist, stehen, steht,
• R⁵ für ein Wasserstoffatom oder ein Halogenatom, einen linearen oder verzweigten C₁-C₃-Alkylrest, der gegebenenfalls durch ein oder mehrere Halogenatome substituiert ist, einen Aminorest -NH₂ oder einen Rest CH₂R'^{7a} steht, wobei R'^{7a} einen Methoxyrest, eine Hydroxygruppe -OH, eine Gruppe -CH₂COOH, eine Gruppe -CH(R^{5b})OH, eine Carboxylgruppe -COOH, eine -CN-Gruppe oder eine Thioxofunktion bedeutet,
• R^{5b} für ein Wasserstoffatom, einen linearen oder verzweigten C₁-C₃-Alkylrest, der gegebenenfalls durch eine oder mehrere Carboxylfunktionen substituiert ist, oder einen Cyclopropylrest steht,
• R^{8a} und R^{8b} gleich oder verschieden sind und ein Wasserstoffatom, einen linearen oder verzweigten C₁-C₃-Alkylrest oder einen Cyclopropylrest bedeuten.

2. Verbindung der Formel (I) nach Anspruch 1, **dadurch gekennzeichnet, dass** R⁷ für einen heterocyclischen Rest steht, der aus den folgenden Heterocyclen ausgewählt ist: 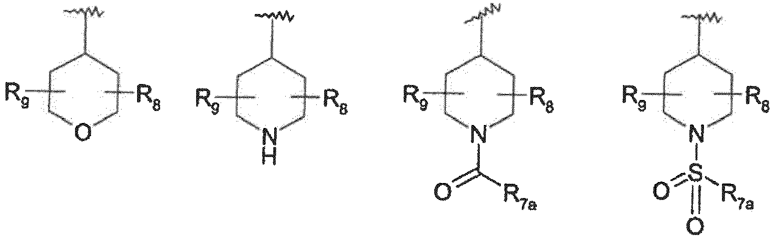 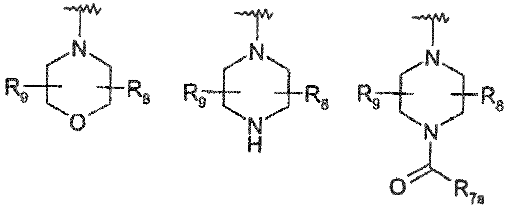 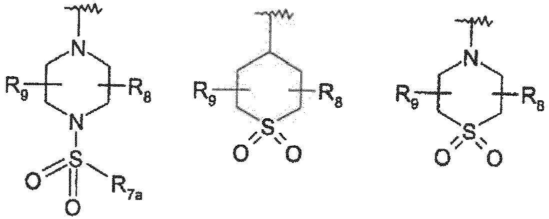 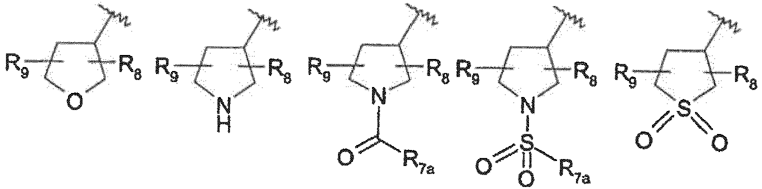 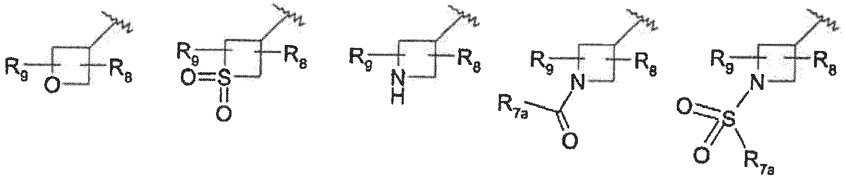 in denen:
- R₇ₐ für einen linearen oder verzweigten C₁-C₃-Alkylrest, einen linearen oder verzweigten C₁-C₃-Alkoxyrest oder einen Aminorest N(R^{8a})(R^{8b}) steht,
- R^{8a} und R^{8b} gleich oder verschieden sind und ein Wasserstoffatom, einen linearen oder verzweigten C₁-C₃-Alkylrest oder einen Cyclopropylrest bedeuten,
- R₈ und R₉ gleich oder verschieden sind und für ein Wasserstoffatom, einen linearen oder verzweigten C₁-C₃-Alkylrest, eine Hydroxygruppe -OH, eine Carbonylfunktion =O, einen C₁-Hydroxyalkylrest (-CH₂OH) oder eine Aminogruppe NH₂ stehen,
- R₈ und R₉ zusammen mit den Kohlenstoffatomen, an die sie gebunden sind, einen 5- bis 7-gliedrigen heterocyclischen Ring bilden können.

3. Verbindung der Formel (I) nach Anspruch 1, **dadurch gekennzeichnet, dass** R⁷ für einen aromatischen oder heteroaromatischen Rest steht, der aus den folgenden Resten ausgewählt ist: 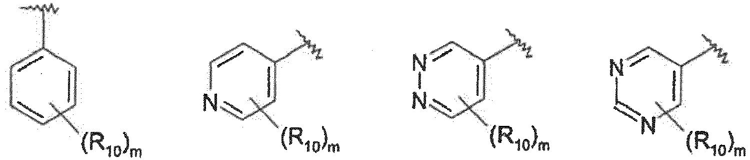 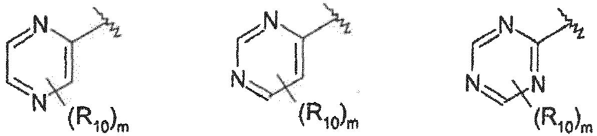 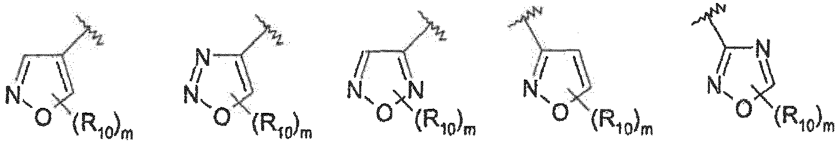 in denen:
- R₁₀ für ein Wasserstoffatom oder ein Halogenatom, eine lineare oder verzweigte C₁-C₃-Alkylgruppe, die gegebenenfalls durch ein oder mehrere Halogenatome substituiert ist, eine C₁-C₃-Alkoxygruppe, eine Aminogruppe -NR¹¹R¹², eine Gruppe -COR¹¹, eine Gruppe -COOR¹¹, eine Amidogruppe -CONR¹¹R¹², eine Gruppe -SOR¹¹, eine Gruppe -SO₂R¹¹, eine Gruppe -NHCOR¹¹, eine Gruppe -NHCOOR¹¹, eine Gruppe -SO₂NR¹¹R¹² oder eine -CN-Gruppe substiuiert ist; wobei R¹¹ und R¹² gleich oder verschieden sind und für ein Wasserstoffatom oder einen linearen oder verzweigten C₁-C₃-Alkylrest, der gegebenenfalls durch ein oder mehrere Halogenatome substituiert ist, stehen,
- m für null oder eine natürliche ganze Zahl im Bereich von 1 bis 3 steht.

4. Verbindung der Formel (I) nach Anspruch 1, **dadurch gekennzeichnet, dass** sie aus der Verbindung bzw. den Verbindungen der Formel (IV) sowie den pharmazeutisch unbedenklichen Additionssalzen davon, Hydraten davon und/oder Solvaten davon ausgewählt ist: 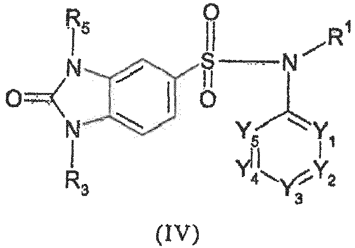 wobei in der Formel (IV) R¹, R³, R⁵ und Y¹ bis Y⁵ die gleichen Bedeutungen wie in der Formel (I) gemäß Anspruch 1 haben.

5. Verbindung der Formel (I) nach einem der vorhergehenden Ansprüche, **dadurch gekennzeichnet, dass** R¹ für einen linearen oder verzweigten C₃-C₅-Alkylrest oder einen C₃-C₅-Cycloalkylrest steht.

6. Verbindung der Formel (I) nach einem der vorhergehenden Ansprüche, **dadurch gekennzeichnet, dass** R⁵ für ein Wasserstoffatom steht.

7. Verbindung der Formel (I) nach einem der vorhergehenden Ansprüche, **dadurch gekennzeichnet, dass** R³ für eine Gruppe (CHR⁶)ₙ-(Z)ₒ-(CHR'⁶)ₚ-R⁷ steht, wobei die Indices n, p, o, R⁶, R'⁶ und R⁷ die angegebene Bedeutung gemäß einem der Ansprüche 1 bis 3 haben.

8. Verbindung der Formel (I) nach einem der vorhergehenden Ansprüche, **dadurch gekennzeichnet, dass** sie aus den folgenden Verbindungen ausgewählt ist:
| | |
|---|---|
| 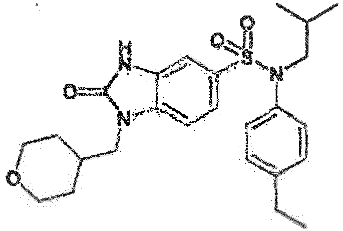 | 2-Oxo-1-(tetrahydropyran-4-ylmethyl)-2,3-dihydro-1H-benzimidazol-5-sulfonsäure(4-ethylphenyl)isobutylamid |
| | Verbindung 2 |
| 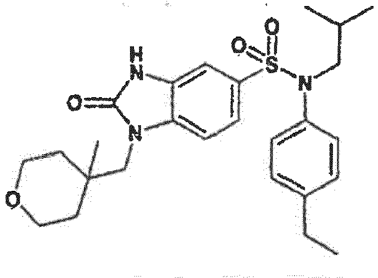 | 1-(4-Methyltetrahydro-pyran-4-ylmethyl)-2-oxo-2,3-dihydro-1H-benzimidazol-5-sulfonsäure(4-ethylphenyl)-isobutylamid |
| | Verbindung 4 |
| 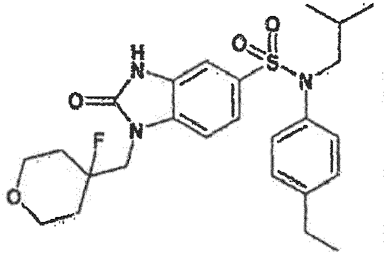 | 1-(4-Fluortetrahydro-pyran-4-ylmethyl)-2-oxo-2,3-dihydro-1H-benzimidazol-5-sulfonsäure(4-ethylphenyl)-isobutylamid |
| | Verbindung 5 |
| 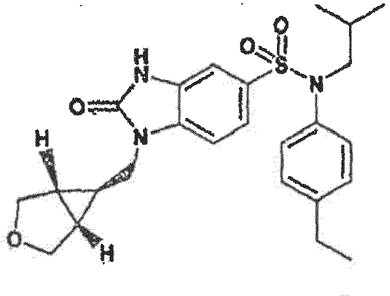 | 1-[(1R, 5S, 6S)-1-(3-Oxabicyclo[3.1.0]hex-6-yl)methyl]-2-oxo-2,3-dihydro-1H-benzimidazol-5-sulfonsäure(4-ethylphenyl)isobutylamid |
| | Verbindung 6 |
| 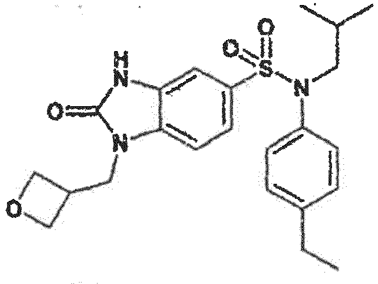 | 1-Oxetan-3-ylmethyl-2-oxo-2,3-dihydro-1H-benzimidazol-5-sulfonsäure(4-ethylphenyl)-isobutylamid |
| | Verbindung 7 |
| 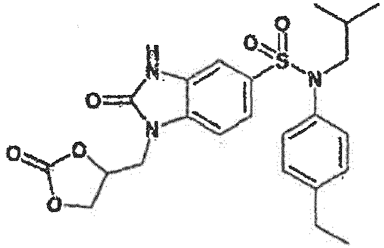 | 2-Oxo-1-(2-oxo[1,3]-dioxolan-4-ylmethyl)-2,3-dihydro-1H-benzimidazol-5-sulfonsäure(4-ethylphenyl)-isobutylamid |
| | Verbindung 8 |
| 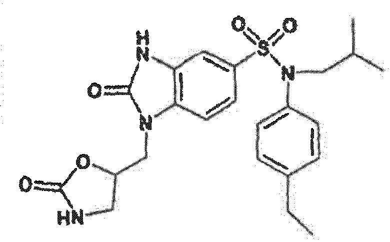 | 2-Oxo-1-(2-oxo-oxazolidin-5-ylmethyl)-2,3-dihydro-1H-benzimidazol-5-sulfonsäure(4-ethylphenyl)-isobutylamid |
| | Verbindung 9 |
| 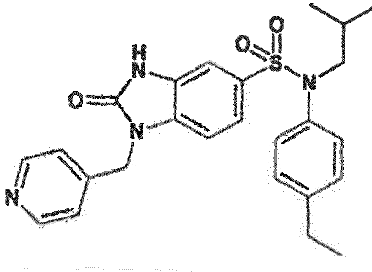 | 2-Oxo-1-pyridin-4-ylmethyl-2,3-dihydro-1H-benzimidazol-5-sulfonsäure(4-ethylphenyl)-isobutylamid |
| | Verbindung 10 |
| 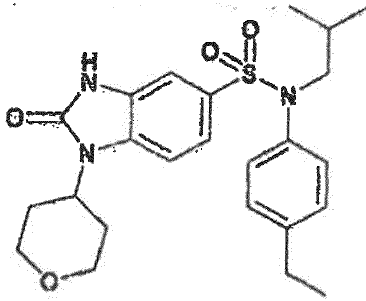 | 2-Oxo-1-(tetrahydropyran-4-yl)-2,3-dihydro-1H-benzimidazol-5-sulfonsäure(4-ethylphenyl)isobutylamid |
| | Verbindung 11 |
| 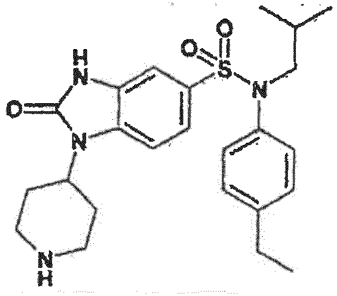 | 2-Oxo-1-piperidin-4-yl-2,3-dihydro-1H-benzimidazol-5-sulfonsäure(4-ethylphenyl)-isobutylamid |
| | Verbindung 13 |
| 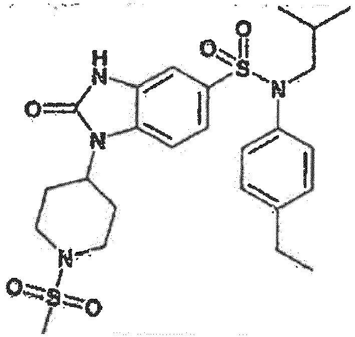 | 1-(1-Methansulfonyl-piperidin-4-yl)-2-oxo-2,3-dihydro-1H-benzimidazol-5-sulfonsäure(4-ethylphenyl)-isobutylamid |
| | Verbindung 14 |
| 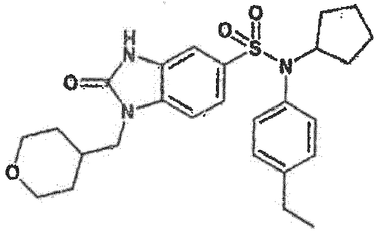 | 2-Oxo-1-(tetrahydropyran-4-ylmethyl)-2,3-dihydro-1H-benzimidazol-5-sulfonsäure(4-ethylphenyl)cyclopentylamid |
| | Verbindung 15 |
| 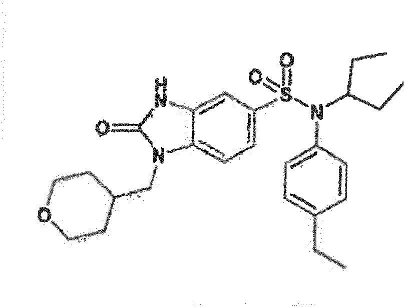 | 2-Oxo-1-(tetrahydropyran-4-ylmethyl)-2,3-dihydro-1H-benzimidazol-5-sulfonsäure(4-ethylphenyl)(1-ethylpropyl) amid |
| | Verbindung 16 |
| 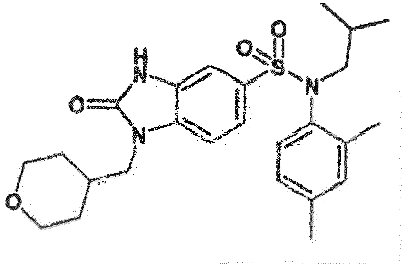 | 2-Oxo-1-(tetrahydropyran-4-ylmethyl)-2,3-dihydro-1H-benzimidazol-5-sulfonsäure(2,4-dimethylphenyl)isobutylamid |
| | Verbindung 17 |
| 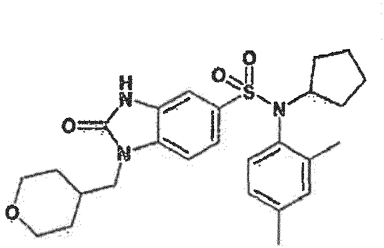 | 2-Oxo-1-(tetrahydropyran-4-ylmethyl)-2,3-dihydro-1H-benzimidazol-5-sulfonsäurecyclopentyl(2,4-dimethylphenyl) amid |
| | Verbindung 18 |
| 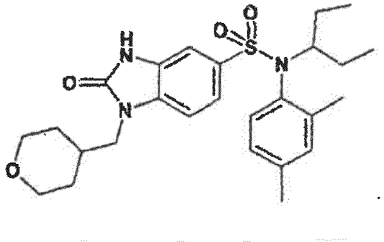 | 2-Oxo-1-(tetrahydropyran-4-ylmethyl)-2,3-dihydro-1H-benzimidazol-5-sulfonsäure(2,4-dimethylphenyl) (1-ethylpropyl) amid |
| | Verbindung 19 |
| 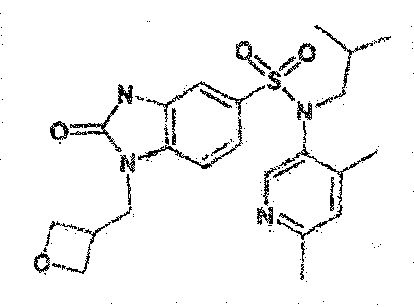 | 1-Oxetan-3-ylmethyl-2-oxo-2,3-dihydro-1H-benzimidazol-5-sulfonsäure(4,6-dimethylpyridin-3-yl)isobutylamid |
| | Verbindung 22 |
| 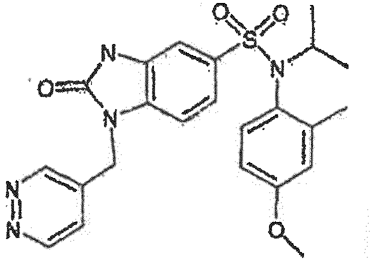 | 2-Oxo-1-pyridazin-4-ylmethyl-2,3-dihydro-1H-benzimidazol-5-sulfonsäureisopropyl(4-methoxy-2-methylphenyl)-amid |
| | Verbindung 23 |

9. Verbindung nach einem der vorhergehenden Ansprüche zur Verwendung als Medikament.

10. Verbindung nach einem der Ansprüche 1 bis 8 zur Verwendung bei der Behandlung von Akne.

11. Verbindung nach einem der Ansprüche 1 bis 8 zur Verwendung bei der Behandlung von Psoriasis.

12. Pharmazeutische Zusammensetzung, umfassend eine oder mehrere Verbindungen gemäß einem der Ansprüche 1 bis 8.

13. Pharmazeutische Zusammensetzung gemäß Anspruch 12 zur Verwendung bei der Behandlung von Akne, atopischer Dermatitis und/oder Psoriasis.

## Claims

1. Compound of formula (I), pharmaceutically acceptable addition salts thereof, hydrates thereof and/or solvates thereof: 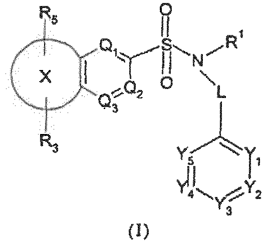 in which formula (I):
• L represents a single bond or a methylene group CH₂,
• X represents the following cyclic radical: 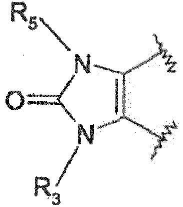
• one or two of the elements Y¹, Y², Y³, Y⁴ and Y⁵ represent(s) a nitrogen atom and the other elements correspond to a -CR² group, or each of the elements Y¹, Y², Y³, Y⁴ and Y⁵ corresponds to a -CR² group,
• one or two of the elements Q¹, Q² and Q³ represent(s) a nitrogen atom and the other element(s) correspond(s) to a -CR^{2a} group, or each of the elements Q¹, Q² and Q³ corresponds to a -CR^{2a} group,
• R¹ represents a linear or branched C₃-C₅ alkyl radical, a C₃-C₅ cycloalkyl radical, a linear or branched C₂-C₅ alkenyl radical, a -CH₂-(C₃-C₅)cycloalkyl radical, a C₄-C₅ heterocycloalkyl radical, or a -CH₂-(C₄-C₆)heterocycloalkyl radical,
• R² represents a hydrogen atom or a halogen atom, a linear or branched C₁-C₅ alkyl radical, a linear or branched C₂-C₄ alkenyl radical, a C₁-C₄ alkoxy radical, a cyano group -CN, a -C(=O)R^{'2} radical with R'² denoting a C₁-C₃ alkoxy radical, or a -CF₃ radical; said alkyl, alkenyl and alkoxy radicals possibly being substituted with one or more halogen atoms;
• R^{2a} represents a hydrogen atom or a halogen atom, a linear or branched C₁-C₅ alkyl radical, a linear or branched C₂-C₄ alkenyl radical, a C₁-C₄ alkoxy radical, a -CN group, a hydroxyl group -OH, a -CH(R^{3a})OH group, a carboxylic group -COOH, a carbamoyl group -CONR^{2c}R^{2d}, an amido group -NR^{2c}COR^{2d}, an SO₂R^{2c} group, an -SOR^{2c} group, or an - S(=O)(=NH-R^{2c}) group, said alkyl, alkenyl and alkoxy radicals possibly being substituted with one or more halogen atoms,
• R^{2c} and R^{2d}, which may be identical or different, represent a hydrogen atom or a linear or branched C₁-C₅ alkyl radical;
• R^{3a} represents a hydrogen atom or a linear or branched C₁-C₅ alkyl radical;
• R³ represents a hydrogen atom or a (CHR⁶)ₙ-(Z)ₒ-(CHR'⁶)ₚ-R⁷ group,
• n, o and p, which may be identical or different, represent zero or a natural integer ranging from 1 to 3,
• Z represents a divalent group chosen from a methylene group -CH₂-, an amino group -NH- and an oxygen atom -O-,
• R⁶ and R'⁶, which may be identical or different, represent a hydrogen atom, a methyl group -CH₃, an -OH group, a hydroxymethyl group, or a carboxylic function -COOH,
• R⁷ represents:
- a hydrogen atom or a halogen atom,
- a COOR'⁷ group with R'⁷ denoting (C₁) alkyl (C₆) heterocycle,
- a non-cationic heterocycloalkyl radical optionally substituted with one or more halogen atoms, one or more linear or branched C₁-C₃ alkyl groups, one or more -OH groups, one or more carbonyl functions, one or more linear or branched C₁-C₄ hydroxyalkyl groups, one or more amino groups, one or more -C(=O)R^{7a} groups, or one or more S (=O)₂R^{7a} groups; R^{7a} representing a linear or branched C₁-C₃ alkyl radical, a linear or branched C₁-C₃ alkoxy radical, or an amino radical N(R^{8a}) (R^{8b}),
- a non-cationic C₃-C₆ cycloalkyl radical optionally substituted with one or more methyl radicals, one or more halogen atoms, a cyano group -CN, or one or more -COR¹³ groups; R¹³ denoting a linear or branched C₁-C₃ alkoxy radical, or a hydroxyl group,
- a non-cationic aromatic or heteroaromatic radical optionally substituted with one or more halogen atoms, one or more linear or branched C₁-C₃ alkyl groups, optionally substituted with one or more halogen atoms, one or more C₁-C₃ alkoxy groups, one or more amino groups -NR¹¹R¹², one or more -COR¹¹ groups, one or more -COOR¹¹ groups, one or more amido groups -CONR¹¹R¹², one or more -SOR¹¹ groups, one or more -SO₂R¹¹ groups, one or more -NHCOR¹¹ groups, one or more -NHCOOR¹¹ groups, one or more -SO₂NR¹¹R¹² groups or one or more -CN groups; R¹¹ and R¹², which may be identical or different, representing a hydrogen atom, a hydroxyl radical -OH, or a linear or branched C₁-C₃ alkyl radical optionally substituted with one or more halogen atoms,
• R⁵ represents a hydrogen atom or a halogen atom, a linear or branched C₁-C₃ alkyl radical optionally substituted with one or more halogen atoms; an amino radical -NH₂, a CH₂R'^{7a} radical with R'^{7a} denoting a methoxy radical, a hydroxyl group -OH, a -CH₂COOH group, a -CH(R^{5b})OH group, a carboxylic group -COOH, a -CN group, or a thioxo function,
• R^{5b} represents a hydrogen atom, a linear or branched C₁-C₃ alkyl radical optionally substituted with one or more carboxylic functions; or a cyclopropyl radical,
• R^{8a} and R^{8b}, which may be identical or different, denote a hydrogen atom, a linear or branched C₁-C₃ alkyl radical or a cyclopropyl radical.

2. Compound of formula (I) according to Claim 1, **characterized in that** R⁷ represents a heterocyclic radical chosen from the following heterocycles: 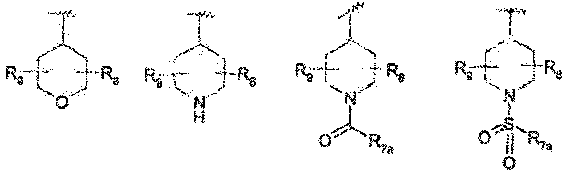 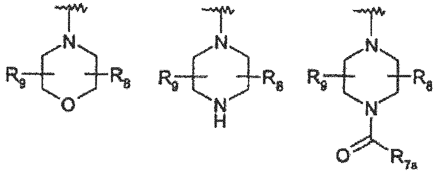 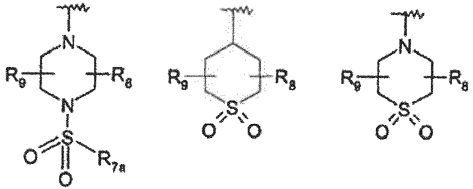 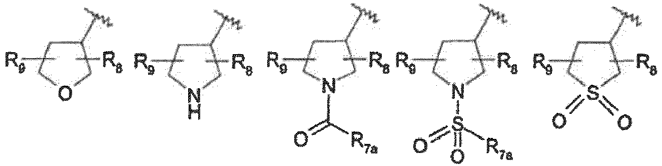 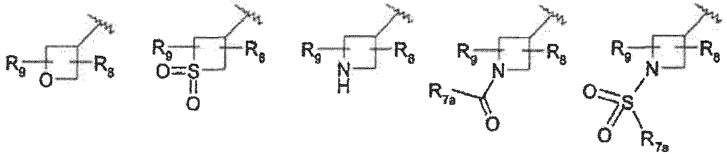 in which:
- R₇ₐ represents a linear or branched C₁-C₃ alkyl radical, a linear or branched C₁-C₃ alkoxy radical or an N(R^{8a}) (R^{8b}) amino radical,
- R^{8a} and R^{8b}, which may be identical or different, denote a hydrogen atom, a linear or branched C₁-C₃ alkyl radical or a cyclopropyl radical,
- R₈ and R₉, which may be identical or different, represent a hydrogen atom, a linear or branched C₁-C₃ alkyl radical, a hydroxyl group -OH, a carbonyl function =O, a C₁ hydroxyalkyl radical (-CH₂OH), or an amino group NH2,
- R₈ and R₉ can form, together with the carbon atoms to which they are attached, a carbocyclic ring comprising from 5 to 7 ring members.

3. Compound of formula (I) according to Claim 1, **characterized in that** R⁷ represents an aromatic or heteroaromatic radical chosen from: 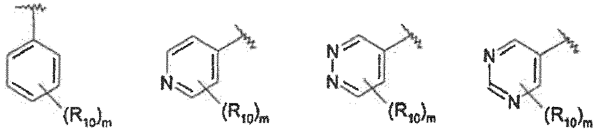 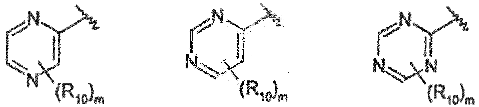 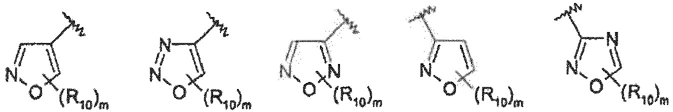 in which:
- R₁₀ represents a hydrogen atom or a halogen atom, a linear or branched C₁-C₃ alkyl group optionally substituted with one or more halogen atoms; a C₁-C₃ alkoxy group, an amino group -NR¹¹R¹², a -COR¹¹ group, a -COOR¹¹ group, an amido group -CONR¹¹R¹², an -SOR¹¹ group, an -SO₂R¹¹ group, an -NHCOR¹¹ group, an -NHCOOR¹¹ group, an -SO₂NR¹¹R¹² group or a -CN group; R¹¹ and R¹², which may be identical or different, representing a hydrogen atom or a linear or branched C₁-C₃ alkyl radical optionally substituted with one or more halogen atoms,
m denotes zero or a natural integer ranging from 1 to 3.

4. Compound of formula (I) according to Claim 1, **characterized in that** it is chosen from the compound(s) of formula (IV) and also pharmaceutically acceptable addition salts thereof, hydrates thereof and/or solvates thereof: 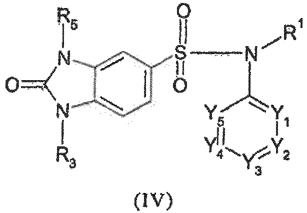 in which formula (IV), R¹, R³, R⁵ and Y¹ to Y⁵ have the same meanings as in formula (I) as defined according to Claim 1.

5. Compound of formula (I) according to any one of the preceding claims, **characterized in that** R¹ represents a linear or branched C₃-C₅ alkyl radical or a C₃-C₅ cycloalkyl radical.

6. Compound of formula (I) according to any of the preceding claims, **characterized in that** R⁵ represents a hydrogen atom.

7. Compound of formula (I) according to any one of the preceding claims, **characterized in that** R³ represents a (CHR⁶)ₙ- (Z)ₒ-(CHR'⁶)ₚ-R⁷ group with the indices n,p,o,R⁶,R'⁶ and R⁷ having the meaning indicated according to any one of the Claims 1 to 3.

8. Compound of formula (I) according to any one of the preceding claims, **characterized in that** it is chosen from the following compounds:
| | |
|---|---|
| 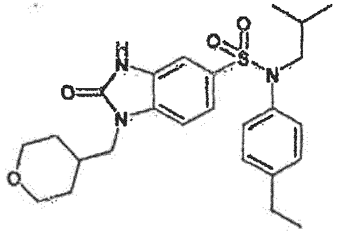 | 2-Oxo-1-(tetrahydropyran-4-ylmethyl)-2,3-dihydro-1H-benzimidazole-5-sulfonic acid (4-ethylphenyl)isobutylamide |
| | Compound 2 |
| 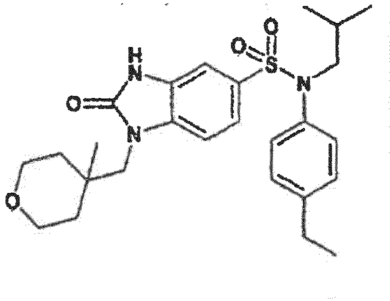 | 1-(4-Methyltetrahydropyran-4-ylmethyl)-2-oxo-2,3-dihydro-1H-benzimidazole-5-sulfonic acid (4-ethylphenyl)isobutylamide |
| | Compound 4 |
| 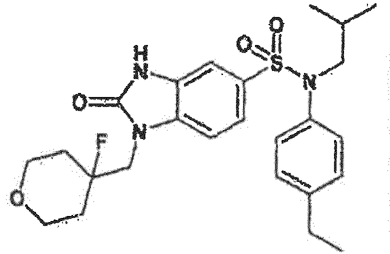 | 1-(4-Fluorotetrahydropyran-4-ylmethyl)-2-oxo-2,3-dihydro-1H-benzimidazole-5-sulfonic acid (4-ethyl phenyl) isobutylamide |
| | Compound 5 |
| 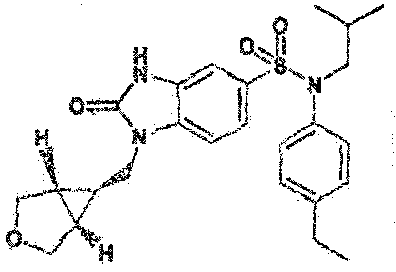 | 1-[(1R,5S,6S)-1-(3-Oxabicyclo[3.1.0]hex-6-yl)methyl]-2-oxo-2,3-dihydro-1H-benzimidazole-5-sulfonic acid (4-ethylphenyl)isobutylamide |
| | Compound 6 |
| 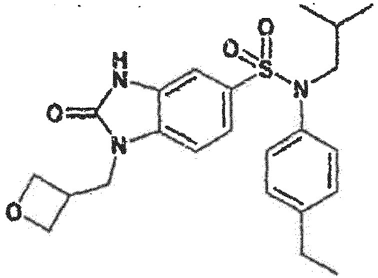 | 1-Oxetan-3-ylmethyl-2-oxo-2,3-dihydro-1H-benzimidazole-5-sulfonic acid (4-ethylphenyl)isobutylamide |
| | Compound 7 |
| 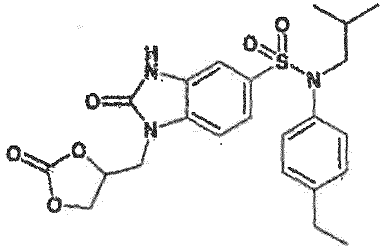 | 2-Oxo-1-(2-oxo[1,3]-dioxolan-4-ylmethyl)-2,3-dihydro-1H-benzimidazole-5-sulfonic acid (4-ethylphenyl)isobutylamide |
| | Compound 8 |
| 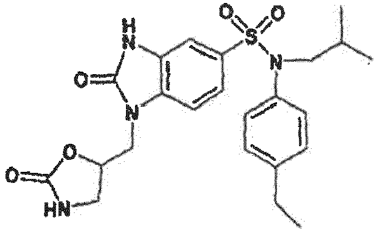 | 2-Oxo-1(2-oxo-oxazolidin-5-ylmethyl)-2,3-dihydro-1H-benzimidazole-5-sulfonic acid (4-ethylphenyl)isobutylamide |
| | Compound 9 |
| 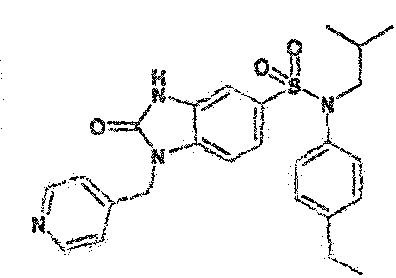 | 2-Oxo-1-pyridin-4-ylmethyl-2,3-dihydro-1H-benzimidazole-5-sulfonic acid (4-ethylphenyl)isobutylamide |
| | Compound 10 |
| 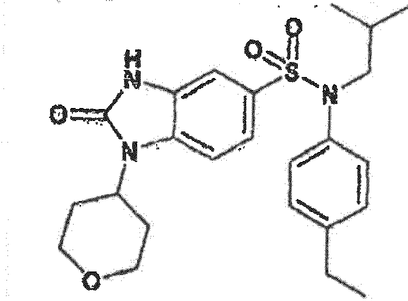 | 2-Oxo-1-(tetrahydropyran-4-yl)-2,3-dihydro-1H-benzimidazole-5-sulfonic acid (4-ethylphenyl)isobutylamide |
| | Compound 11 |
| 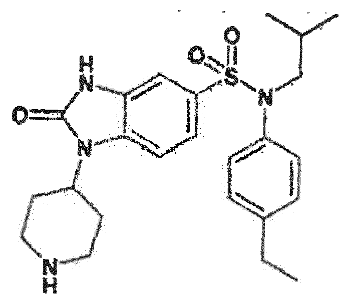 | 2-Oxo-1-piperidin-4-yl-2,3-dihydro-1H-benzimidazole-5-sulfonic acid (4-ethyl phenyl) isobutylamide |
| | Compound 13 |
| 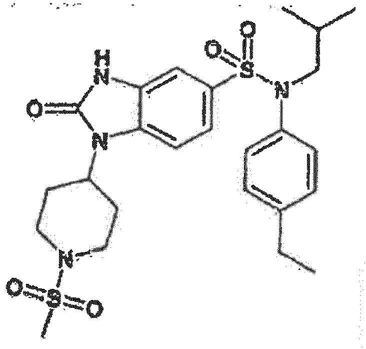 | 1-(1-Methanesulfonylpiperidin-4-yl)-2-oxo-2,3-dihydro-1H-benzimidazole-5-sulfonic acid (4-ethylphenyl)isobutylamide |
| | Compound 14 |
| 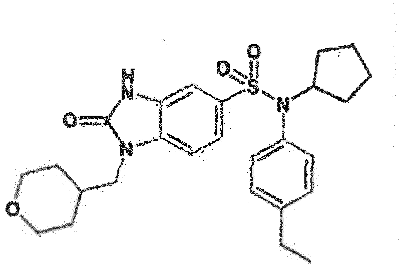 | 2-Oxo-1-(tetrahydropyran-4-ylmethyl)-2,3-dihydro-1H-benzimidazole-5-sulfonic acid (4-ethylphenyl)cyclopentylamide |
| | Compound 15 |
| 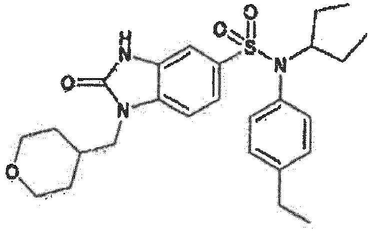 | 2-Oxo-1-(tetrahydropyran-4-ylmethyl)-2,3-dihydro-1H-benzimidazole-5-sulfonic acid (4-ethylphenyl)(1-ethylpropyl)amide |
| | Compound 16 |
| 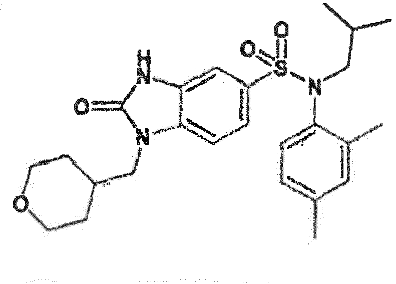 | 2-Oxo-1-(tetrahydropyran-4-ylmethyl)-2,3-dihydro-1H-benzimidazole-5-sulfonic acid (2,4-dimethylphenyl) isobutylamide |
| | Compound 17 |
| 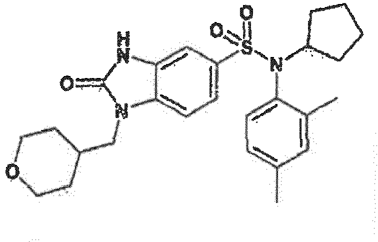 | 2-Oxo-1-(tetrahydropyran-4-ylmethyl)-2,3-dihydro-1H-benzimidazole-5-sulfonic acid cyclopentyl-(2,4-dimethylphenyl)amide |
| | Compound 18 |
| 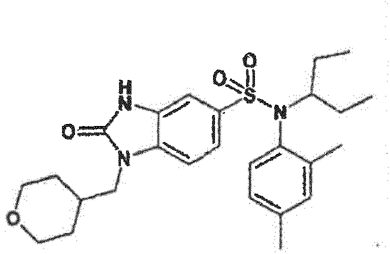 | 2-Oxo-1-(tetrahydropyran-4-ylmethyl)-2,3-dihydro-1H-benzimidazole-5-sulfonic acid (2,4-dimethylphenyl)(1-ethylpropyl)amide |
| | Compound 19 |
| 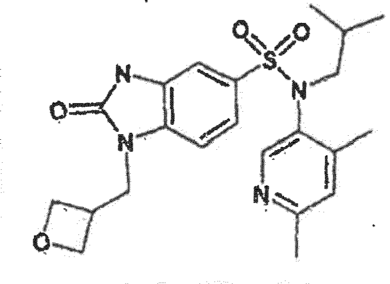 | 1-Oxetan-3-ylmethyl-2-oxo-2,3-dihydro-1H-benzimidazole-5-sulfonic acid (4,6-dimethylpyridin-3-yl)isobutylamide |
| | Compound 22 |
| 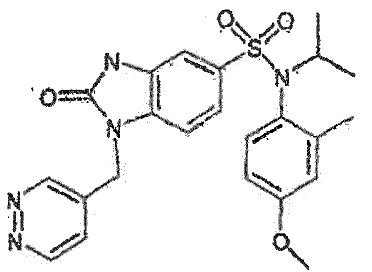 | 2-Oxo-1-pyridazin-4-ylmethyl-2,3-dihydro-1H-benzimidazole-5-sulfonic acid isopropyl(4-methoxy-2-methylphenyl)-amide |
| | Compound 23 |

9. Compound according to any one of the preceding claims, for use thereof as a medicament.

10. Compound according to any one of Claims 1 to 8, for use thereof in the treatment of acne.

11. Compound according to any one of Claims 1 to 8, for use thereof in the treatment of psoriasis.

12. Pharmaceutical composition comprising one or more compounds as defined according to any one of Claims 1 to 8.

13. Pharmaceutical composition as defined according to Claim 12, for use thereof in the treatment of acne, atopic dermatitis and/or psoriasis.
